# EUROPEAN PATENT SPECIFICATION

(11) **EP 2 948 547 B1**
(45) Date of publication and mention of the grant of the patent: **22.01.2020**
(21) Application number: 14702514.2
(22) Date of filing: 28.01.2014
(51) Int. Cl.: C12N 9/54

(54) **NOVEL GLUCOSE OXIDASES DERIVED FROM ASPERGILLUS NIGER**
NEUARTIGE, AUS ASPERGILLUS NIGER GEWONNENE GLUCOSEOXIDASEN
NOUVELLES GLUCOSES OXYDASES DÉRIVÉES D'ASPERGILLUS NIGER

(30) Priority: 28.01.2013 EP 13152935
(43) Date of publication of application: 02.12.2015
(73) Proprietor: Roche Diabetes Care GmbH, 68305 Mannheim (DE); F. Hoffmann-La Roche AG, 4070 Basel (CH)
(72) Inventor: BOCOLA, Marco, 52066 Aachen (DE); DUEFEL, Hartmut, 82444 Schlehdorf (DE); ARANGO GUTIERREZ, Erik Uwe, 88400 Biberach (DE); HEINDL, Dieter, 82396 Paehl (DE); MEIER, Thomas, 81373 München (DE); MUNDHADA, Hemanshu, DK-2800 Lyngby (DK); SCHWANEBERG, Ulrich, B-4728 Kelmis-Hergenrath (BE); TACKE, Michael, 80689 München (DE)
(74) Representative: Kilger, Ute
(86) International application number: PCT/EP2014/051602
(87) International publication number: WO 2014/114810

(56) References cited:
- EP-A1- 2 415 863
- Iuliana Carmen Momeu: "Improving Glucose Oxidase Properties by Directed Evolution", , 1 January 2007 (2007-01-01), XP055011653, Retrieved from the Internet: URL:http://www.jacobs-university.de/phd/fi les/1169800479.pdf [retrieved on 2011-11-09]
- FREDERICK K R ET AL: "GLUCOSE OXIDASE FROM ASPERGILLUS-NIGER.CLONING,GENE SEQUENCE,SECRETION FROM SACCHAROMYCES-CEREVISIAE AND KINETIC ANALYSIS OF A YEAST-DERIVED ENZYME", JOURNAL OF BIOLOGICAL CHEMISTRY, AMERICAN SOCIETY FOR BIOCHEMISTRY AND MOLECULAR BIOLOGY, US, vol. 265, no. 7, 5 March 1990 (1990-03-05) , pages 3793-3802, XP002271001, ISSN: 0021-9258
- BHATTI H N ET AL: "Purification and thermodynamic characterization of glucose oxidase from a newly isolated strain of Aspergillus niger", CANADIAN JOURNAL OF MICROBIOLOGY, NRC RESEARCH PRESS, CA, vol. 52, no. 6, 1 June 2006 (2006-06-01), pages 519-524, XP009098037, ISSN: 0008-4166, DOI: 10.1139/W05-158
- ZHU Z ET AL: "Making glucose oxidase fit for biofuel cell applications by directed protein evolution", BIOSENSORS AND BIOELECTRONICS, ELSEVIER BV, NL, vol. 21, no. 11, 15 May 2006 (2006-05-15), pages 2046-2051, XP024961490, ISSN: 0956-5663 [retrieved on 2006-05-15]

## Description

### Field of the invention

The present invention relates to novel glucose oxidase variants. The herein provided glucose oxidases are specific for the substrate glucose, and thereby exhibit significantly reduced oxygen-consumption rates. In another aspect, the present invention provides for glucose oxidases that are specific for the substrate glucose, and thereby exhibit significantly reduced oxygen-consumption rates and/or increased enzymatic activity for electron mediators other than oxygen. Further, the present invention relates to said glucose oxidases for use in a kit and a sensor for the measurement of glucose.

In particular, the present invention relates to glucose oxidases derived from *Aspergillus niger,* having besides the two substitutions T30V and I94V according to SEQ ID NO: 1, additionally at least one amino acid substitution in any of the five positions selected from the group S53; A173; A332; F414; and V560.

### Scientific Background

Diabetes mellitus reflects a metabolic disease, which can be found extensively all over the world. Diabetes patients have an impaired or missing production of the hormone insulin, which controls the blood-glucose level and thus these patients bear a risk of hyperglycemia as well as hypoglycemia in case of inadequate insulin application *[*Definition and diagnosis of diabetes mellitus and intermediate hyperglycaemia. WHO, and IDF (2006); WHO Document Production service ISBN 9241594934*].*

To ensure a correct application of insulin highly specific, accurate and easy to handle glucose measurement systems are needed for both, self-measurement systems and high-throughput measurement systems on clinical scale.

To allow the adequate determination of glucose concentrations in the blood and to make the measurement highly specific, enzymatic reactions are involved. These days, the two types of enzymes that are used in diabetes analytics, are reflected by the glucose dehydrogenases (hereinafter GDH(s)) and glucose oxidases (hereinafter GOx(s)) *[*Hönes, J., Müller, P., and Surridge, N. (2008). The Technology Behind Glucose Meters: Test Strips. DIABETES TECHNOLOGY & THERAPEUTICS 10, 10-26*].*

The main advantage of the GDHs is its oxygen-independent oxidation of glucose, but the enzyme shows slight side-activities on certain other clinical relevant sugars, and thus GDH is unspecific *[*Olsthoorn, A.J., and Duine, J.A. (1998). On the mechanism and specificity of soluble, quinoprotein glucose dehydrogenase in the oxidation of aldose sugars. Biochemistry 37, 13854-13861*].* By contrast, the GOxs are highly specific for glucose, but its oxidation is strongly oxygen-dependent *[*Bankar, S.B., Bule, M. V., Singhal, R.S., and Ananthanarayan, L. (2009). Glucose oxidase -- an overview. Biotechnology Advances 27, 489-501*;* Bentley, R., and Neuberger, A. (1949). The mechanism of the action of notation. Biochem J 45, 584-590*].*

In more detail, GOxs as flavoproteins belong to the family of oxidoreductases (i.e. β-D-glucose: oxygen 1-oxidoreductase). Native or wild-type (hereinafter WT) GOxs catalyze the oxidation of β-D-glucose to D-glucono-δ-lactone and H₂O₂ by employing molecular oxygen as an electron acceptor *[see e.g.,* Pazur, J.H., and Kleppe, K. (1964). The Oxidation of Glucose and Related Compounds by Glucose Oxidase from Aspergillus Niger. Biochemistry 3, 578-583*].* Said reaction is depicted by the following formula:

D-glucose + O₂ → Gluconolactone + H₂O₂

The substrates of the GOxs can be divided into two groups: i) the electron acceptors of the oxidative half reaction and ii) the electron donors of the reductive half reaction, see e.g. *[*Leskovac, V., Trivic, S., Wohlfahrt, G., Kandrac, J., and Pericin, D. (2005). Glucose oxidase from Aspergillus niger: the mechanism of action with molecular oxygen, quinones, and one-electron acceptors. Int J Biochem Cell Biol 37, 731-750*].* The person skilled in the art is aware that apart from D-glucose various derivatives of D-glucose are potential substrates for the reductive half reaction of GOxs.

GOxs from different origins have been described so far. For instance, the GOx from marine algae *Chondrus crispus* is described in US 7,544,795 and US 6,924,366; the GOx from filamentous fungi *Cladosporium spec.* is described in WO 95/29996; WO 1998/020136; and the GOx from *Talaromyces flavus* is described in US 6,054,318.

The best-described GOx in literature is from *Aspergillus niger [*Hecht, H.J., Schomburg, D., Kalisz, H., and Schmid, R.D. (1993). The 3D structure of glucose oxidase from Aspergillus niger. Implications for the use of GOD as a biosensor enzyme. Biosensors & Bioelectronics 8, 197-203*;* Wohlfahrt, G., Witt, S., Hendle, J., Schomburg, D., Kalisz, H.M., and Hecht, H.J. (1999). 1.8 and 1.9 A resolution structures of the Penicillium amagasakiense and Aspergillus niger glucose oxidases as a basis for modelling substrate complexes. Acta Crystallographica Section D Biological Crystallography 55, 969-977*].*

WO89/126675 describes the production of GOxs from *Aspergillus niger* in recombinant systems and WO 2008/079227 A1 relates to a GOx obtained from *Aspergillus niger* formulated in a composition conferring improved storage stability.

It is a well-characterized protein forming a dimer of 160 kDa in size and crystal structures have been solved thereof *[*Hecht, H.J., et al., Crystal-Structure Of Glucose-Oxidase From Aspergillus-Niger Refined At 2.3 Angstrom Resolution. Journal Of Molecular Biology, 1993. 229(1): p. 153-172*].*

It is further known that particularly the wild-type GOx of *Aspergillus niger* (hereinafter GOx-WT) exhibits significant temperature stability and specificity for the substrate glucose. The GOx is a glycoprotein with a high-mannose type carbohydrate content of 10-16% *[*Hayashi, S., and Nakamura, S. (1981). Multiple forms of glucose oxidase with different carbohydrate compositions. Biochim Biophys Acta 657, 40-51*;* Pazur, J.H., Kleppe, K., and Cepure, A. (1965). A glycoprotein structure for glucose oxidase from Aspergillus niger. Arch Biochem Biophys 111, 351-357*].*

These days, GOxs are commonly used in biosensors for the detection of glucose in either industrial solutions or in bodily fluids of a subject, e.g. in blood and urine.

The most of currently available self-measurement devices are electrochemical sensors consisting in principle of
a) a biological component, i.e. the respective enzyme having glucose as substrate,
b) an indicator (the electronic component), and
c) a signal transducer.

In the measurement device, electrons from the glucose are transferred by the biological component (a) to an electrode (b) via mediators. The signal transducer (c) then converts the electrical signal into the real-time glucose concentration, which is proportional to the amount of transferred electrons.

Apart from the above-described electrochemical sensors, there are also photometric sensors available. The difference here is that the electrons from the glucose are transferred to redox-indicator dye (serving as indicator). The resulting color change of the reduced dye is measured photometrically.

Another main application might become the use of GOxs in the anodic compartment of implanted and miniaturized biofuel cells burning glucose from the blood stream and thereby powering miniature diagnostic devices or pumps.

Moreover, GOx applications in the food industry are numerous, since its capability of generating hydrogen peroxide, which has an anti-microbial effect, can be utilized to improve the storage stability of certain food products including for instance cheese, butter and fruit juice.

Applications of GOxs in cosmetic compositions may utilize the anti-microbial properties as well. Potential uses for hexose oxidases in pharmaceutical and cosmetic compositions were suggested e.g. in US 6,924,366; US 6,251,626 and WO 2007/045251 A2.

Furthermore, a use of GOx is suggested in the production of transgenic plants and other organisms with reduced susceptibility or increased resistance to pests or diseases (see e.g. WO 1995/021924).

However, the use of GOxs in glucose-biosensors is of significant interest in accordance with the present invention. In this regard, WO 2009/104836 A1 describes a glucose sensor comprising a genetically engineered GOx variant improved for its attachment to metal surfaces.

Zhu *et al.* described in 2006 and 2007 mutants of a GOx derived from *Aspergillus niger,* being mutated in T30V and/or I94V. Thus, also the corresponding double-mutant T30V; I94V have been described *[*Zhu, Z., Momeu, C., Zakhartsev, M., and Schwaneberg, U. (2006). Making glucose oxidase fit for biofuel cell applications by directed protein evolution. Biosensors and Bioelectronics 21, 2046-2051*;* Zhu, Z., Wang, M., Gautam, A., Nazor, J., Momeu, C., R, P., and U, S. (2007). Directed evolution of glucose oxidase from Aspergillus niger for ferrocenemethanol-mediated electron transfer. Biotechnology Journal 2, 241-248*]* (hereinafter Zhu *et al.* (2006/2007).

Specifically, the above-mentioned double-mutant having the substitutions T30V and I94V is slightly increased in enzyme activity (k_{cat} from 69.5/s to 137.7/s), exhibits an increased thermostability in the range of 58°C to 62°C, and an improved pH stability in the range of 8 to 11 as compared to GOx-WT. However, said double-mutant derived from *Aspergillus niger* exhibits equal oxygen-consumption rates as compared to the GOx-WT.

EP 2 415 863 A1 describes nucleic acid molecules and polypeptides thereof having GOx activity but being mutated in at least three of the following amino acid positions 2, 13, 30, 94, and 152. Particularly, the M12 variant of EP 2 415 863 A1, having the substitutions N2Y, K13E, T30V, I94V, and K152R shows besides an increased expression level in *Saccharomyces cerevisiae,* a twice-increased activity for oxygen as electron acceptor.

Horaguchi *et al. [*Horaguchi, Y., Saito, S., Ferri, S., Mori, K., Kojima, K., Tsugawa, W., and Sode, K. (2012). Turning Glucose Oxidase into Essentially Dehydrogenase. Meet. Abstr., Volume MA2012-02, Issue 18, Pages 2057*]* identified in 2012 one amino acid residue position being involved in the oxidative half reaction of the GOx variants described therein, being it the GOx of *Penicillium amagasakiense* and the GOx variant of *Aspergillus niger.* Said one position is S114 of the *Penicillium amagasakiense* GOx variant, and the corresponding T110 of the *Aspergillus niger* variant. Both positions were replaced by the amino acid Alanine leading to a decrease in activity for oxygen as electron acceptor. For instance, the GOx variant of *Aspergillus niger* exhibited a 6.6-fold reduced oxygen consumption and thus was having a residual oxygen activity of 30.4% besides a mediator activity of 363%.

### Underlying problem of the invention

The GDHs that are unspecific for glucose, and the oxygen-dependent GOxs represent the key enzymes of current glucose measurement systems.

With the context of the present disclosure the expressions "unspecific for glucose", "oxygen-dependent" and "oxygen-dependency" have the meaning as set forth in the Definitions section.

### Solution to the underlying problem by the invention

The solution to the underlying problem of the invention is the provision of specifically modified and thus optimized GOx variants derived from fungus *Aspergillus niger.*

Surprisingly and unexpectedly, the inventors have found novel GOx variants derived from *Aspergillus niger* that are specific for glucose, but independent from oxygen for glucose oxidation and thus more accurate for glucose measurements.

Moreover, the present invention, surprisingly und unexpectedly, provides for novel GOx variants that are specific for glucose, and thereby exhibit significantly reduced oxygen-consumption rates and/or significantly increased mediator activity for electron mediators other than oxygen.

Subject matter of the present invention are novel GOx variants having besides the two substitutions T30V and I94V according to SEQ ID NO: 1, additionally at least one amino acid substitution in any of the five positions selected from the group S53; A173; A332; F414; and V560.

In the context of the present disclosure the expressions "enzyme specificity for glucose" or "specific for glucose" denotes activity of the herein provided GOxs for the substrate glucose of > 99.5%, or > 99.9%, in particular 100% when determined by mediator assay as outlined in the Material and Methods under item ff). By implication, the residual activity of the herein provided GOxs for sugars other than glucose such as galactose, maltose, xylose, and maltotriose is < 6% when determined by mediator assay as outlined in the Material and Methods section under item ff).

In the context of the present disclosure the expression "significantly reduced oxygen-consumption rates" denotes for the herein provided GOx variants a residual oxygen content of > 95% over a time period of 3 min when oxygen-dependency is determined indirectly by the oxidation of the chromogenic substrate ABTS as pursuant to the ABTS assay outlined in the Materials and Methods section under item gg).

In the context of the present disclosure the expressions "significantly reduced oxygen activity" or "significantly reduced activity for oxygen as electron acceptor" denote a GOx activity characterized by residual oxygen activity ≤ 30%, or < 25%, or < 20%, particularly < 15%, or ≤ 10% when determined by the ABTS assay as outlined in the Materials and Methods section under item gg).

In the context of the present disclosure the expressions "significantly increased mediator activity" or "significantly increased activity for electron mediators other than oxygen" denote a GOx activity characterized by at least a 1.5-fold increased activity for electron mediators other than oxygen of that of the glucose oxidase according to SEQ ID NO: 1 when determined by mediator assay as outlined in the Materials and Methods section under item ff).

The thus optimized GOx variants of the invention are suitable to be implemented in improved blood-glucose measurement systems.

### Brief description of the invention

Surprisingly and unexpectedly, the inventors found the GOx double-mutant T30V; I94V (hereinafter abbreviated GOx-T30V; I94V) as described in Zhu *et al.* (2006; 2007), suitable as basis for further specific amino acid substitution(s) to obtain oxygen-independent GOx variants having a significantly reduced oxidase activity and concomitantly a significantly increased dehydrogenase activity while remaining specific for the substrate glucose. Further, the inventors found GOx variants in accordance with the invention that exhibit additionally or alone a significantly increased mediator activity for electron mediators other than oxygen. In this regard, the inventors also found GOx variants in accordance with the invention that accept certain electron mediators other than oxygen for electron transfer. Thus, the GOx variants in accordance with the invention are suitable for improved glucose measurements, in particular for improved blood-glucose measurements.

With the context of the present disclosure the expression "accept certain electron mediators other than oxygen for electron transfer" denotes the capability of herein provided GOx variants to interact with and thus accept mediators selected from the group comprising nitrosoanilines and derivatives thereof, azo-compounds, phenazines and derivatives thereof, phenothiazines and derivatives thereof, phenoxazines and derivatives thereof, ferrocenes and derivatives thereof, potassium ferricyanide, Ru- and Os-complexes, quinones and derivatives thereof, indophenols, viologens, tetrathiafulvalene and derivatives thereof, and phthalocyanines for mediated electron transfer.

The present invention relates to GOx variants derived from *Aspergillus niger* having besides the two substitutions T30V and I94V according to SEQ ID NO: 1, additionally at least one amino acid substitution in any of the five positions selected from the group S53; A173; A332; F414; and V560.

In another aspect, the present invention relates to GOx variants derived from *Aspergillus niger* having besides the two substitutions T30V and I94V according to SEQ ID NO: 1, additionally at least one amino acid substitution in any of the four positions selected from the group A173; A332; F414; and V560.

In a further aspect, the present invention relates to GOx variants having at least two, or three, or four, or five cooperative, and thus diverse amino acid substitutions in any of the five positions selected from the group S53; A173; A332; F414; and V560 of the GOx according to SEQ ID NO: 1, leading to a significant decrease in oxygen-consumption rates.

Further, the present invention relates to GOx variants having at least two, or three, or four, or five cooperative, and thus diverse amino acid substitutions in any of the five positions selected from the group S53; A173; A332; F414; and V560 of the GOx according to SEQ ID NO: 1, leading to a significant decrease in oxygen-consumption rates and/or a significant increase in mediator-activity for certain electron-mediators other than oxygen.

Thus, the inventors of the present invention have been able to provide for GOx variants, whose oxidase activity is significantly reduced in comparison to the GOx-WT and the GOx- T30V; I94V according to SEQ ID NO: 1 while at the same time the enzyme's dehydrogenase activity is significantly increased in comparison to the GOx-WT and the GOx-T30V; I94V.

In this regard, it is a further unexpected and surprising finding that specific amino acid substitutions in the positions F414 and V560 reduce significantly the oxygen consumption rates of the herein provided GOx variants, when compared to the GOx-WT and the GOx-T30V; I94V in the ABTS assay as outlined in the Materials and Methods section under item gg). Suitable amino acids for said substitutions are Ile, Leu, Met, Val for the position F414, and Leu, Pro, Thr for the position V560.As another unexpected and surprising finding, specific amino acid substitutions to both positions F414 and V560 in combination, besides the two substitutions T30V and I94V in the herein provided GOx variants, increase significantly the mediator-activity for electron mediators other than oxygen when compared to the GOx-T30V; I94V. Suitable amino acids for said substitutions are Ile, Leu, Met, Val for the position F414, and Leu, Pro, Thr for the position V560.

As a further finding the present invention reveals that each of the positions A173 and A332, besides the two substitutions T30V and I94V in the herein provided GOx variants, lead generally to an increase in GOx activity when compared to the GOx-WT and the GOx-T30V;I94V. Suitable amino acids for said substitutions are Ile, Thr, and Val for the position A173, and Ser, Val, Thr for the position A332.

Therefore, the present invention enables the person skilled in the art to obtain GOx variants, having significantly reduced oxygen-consumption rates.

In addition, the present invention enables the person skilled in the art to obtain GOx variants, having significantly reduced oxygen-consumption rates and/or significantly increased mediator-activity for certain electron mediators other than oxygen, either individually or for both features in combination via specific amino acid substitution(s).

### Detailed description of the invention

Diabetic patients are in need of accurate blood glucose measurements during their everyday life. As outlined in the scientific background section, existing enzymatic measurement systems are based on oxygen-dependent GOxs and GDHs that are unspecific for glucose.

In the GOx-WT from *Aspergillus niger,* the oxidase activity is about three to four times higher than its dehydrogenase activity. Accordingly, when dissolved oxygen is present in a blood-glucose assay system, the electrons generated during oxidation of the substrate glucose will be also transferred to the oxygen. Thus, the enzyme activity measured in the presence of an electron mediator may be affected by the dissolved oxygen concentration. However, the GOx-WT from *Aspergillus niger* is specific for glucose and exhibits sufficient temperature stability.

By contrast, the GDH(s) are resistant to dissolved oxygen in the blood samples but are not sufficiently specific for glucose and thus may also be affected by other sugar types present in the blood sample as e.g. maltose, galactose, xylose, and maltotriose.

It was an object of the instant invention to eliminate the effects of dissolved oxygen during blood-glucose measurements based on GOxs while preserving and further increasing its advantageous properties.

Thus, the inventors of the present invention have been able to provide for GOx variants, whose oxidase activity is significantly reduced in comparison to the GOx-WT and the GOx- T30V; I94V according to SEQ ID NO: 1 while at the same time the enzyme's dehydrogenase activity is significantly increased in comparison to the GOx-WT and the GOx-T30V; I94V.

As used herein "oxidase activity" is the enzymatic activity of the herein provided GOx variants to catalyze oxidation of glucose to generate gluconolactone with utilizing oxygen as an electron acceptor. The "oxidase activity" may be assayed by measuring the amount of generated H₂O₂ by any methods known in the art. For instance the "oxidase activity" may be assayed by reagents for H₂O₂ detection such as 4AA/TODB/POD (4-aminoantipyrine(N,N-Bis(4-sulfobutyl)-3-methylalanine disodium salt/horseradish peroxidase) or by Pt electrode. As used herein in the context of the relative or quantitative activity, the oxidase activity is specifically defined to be the mole amount of the substrate (glucose) oxidized per unit time measured by the amount of generated H₂O₂ at 25°C in 10mM PPB, pH 7.0, 1.5 mM TODB, 2 U/mL horseradish peroxidase (POD), and 1.5 mM 4-aminoantipyrine (4AA). The formation of quinoneimine dye may be measured spectrophotometrically at 546 nm.

As used herein, "dehydrogenase activity" is an enzymatic activity of the herein provided GOx variants to catalyze oxidation of glucose to generate gluconolactone by utilizing an electron mediator other that oxygen as an electron acceptor. The "dehydrogenase activity" may be assayed by measuring the amount of electrons transferred to the used mediator other than oxygen.

As used herein in the context of the relative or quantitative activity, the "dehydrogenase activity" is specifically defined to be the mole amount of the substrate (glucose) oxidized per unit time measured by the amount of electrons transferred to the mediator other than oxygen at 25 °C in 10 mM PPB (pH 7.0), 0.6 mM methoxy PMS (mPMS).

With the context of the present disclosure the expressions "electron mediator(s)" and "mediator(s) other than oxygen" denote a small organic or inorganic chemical capable of existing in both, an oxidized and a reduced form, and that reacts quickly to donate or receive electrons. Particularly, the expressions "electron mediator(s)" and "mediator(s) other than oxygen" denote small organic or inorganic chemicals, being an electron acceptor for glucose and thereby being converted from the oxidized into the reduced form. Following this, said mediator delivers the electrons in the reduced form to a working electrode for either electrochemical glucose measurement or to an indicator molecule for colorimetric measurement in a blood glucose assay system. Some electron acceptors act as an indicator molecule by itself and can be used directly for colorimetric measurement of glucose (see e.g. EP 8 313 27).

In a specific aspect of the invention, the electron mediator or mediator other than oxygen is selected from the group comprising nitrosoanilines and derivatives thereof, azo-compounds, phenazines and derivatives thereof, phenothiazines and derivatives thereof, phenoxazines and derivatives thereof, ferrocenes and derivatives thereof, potassium ferricyanide, Ru- and Os-complexes, quinones and derivatives thereof, indophenols, viologens, tetrathiafulvalene and derivatives thereof, and phthalocyanines.

In another more specific aspect of the invention the electron mediator or mediator other than oxygen is selected from the group comprising quinones, such as e.g. phenanthrendiones, 1,4 diamino anthraquinone and metallcomplexes of phenanthrendione, and nitrosoanilines, such as e.g. N,N-bis(2-hydroxyethyl)-4-nitrosoaniline or N,N-bis(2-hydroxyethyl)-2-methoxy-4-nitrosoaniline. Either of the latter two nitrosoanilines will hereinafter be referred to as "Nitrosoaniline Mediator".
Such electron mediators as mentioned above have been thoroughly described e.g. in US 5,393,615; US 5,498,542; US 5,520,786; WO 2009/129108; US 2009/0095642; WO 93/25898; JP 57-128678; EP 0 441 222 and in Prevoteau, A. et al., Electrochemistry Communications (2010), 12(2), 213-215*;* Berchmans, S. et al., Materials Chemistry and Physics (2003), 77(2), 390-396*.*

Especially suitable for the subject matter of the instant disclosure are electron mediators which are fast reduced by FADH2 but slow or not reduced by ascorbic acids; such mediators are e.g. derivatives of 2-[4-(dimethylamino)phenyl]-diazenecarboxamide.

Another use of the herein described methods to generate "tailor made" oxygen-independent GOx variants is to adapt the respective GOx variant to an immobilized mediator.

In this regard, the term "tailor made" means that the polypeptide sequence of the respective GOx variant of the invention enables the GOx to interact with and thus accept electrons from a certain immobilized mediator.

The expression "certain immobilized mediator" denotes an electron mediator or mediator other than oxygen selected from the group comprising nitrosoanilines and derivatives thereof, azo-compounds, phenazines and derivatives thereof, phenothiazines and derivatives thereof, phenoxazines and derivatives thereof, ferrocenes and derivatives thereof, potassium ferricyanide, Ru- and Os-complexes, quinones and derivatives thereof, indophenols, viologens, tetrathiafulvalene and derivatives thereof, and phthalocyanines.

In another very specific aspect of the present disclosure, mediators from the group of nitrosoanilines, in particular from the group of p-nitrosoanilines or derivatives thereof, and in particular the Nitrosoaniline Mediators or derivatives thereof is/are preferred as electron mediators in accordance with the invention. In general, mediators from the group of nitrosoanilines react *in situ* e.g. on a test strip with glucose and the GOx to form a species that acts as electron mediator. Nitrosoanilines with the context of glucose measurements and mediator activity are described in detail in Hönes, J., Müller, P., and Surridge, N. (2008). The Technology Behind Glucose Meters: Test Strips. Diabetes Technology & Therapeutics 10, 10-26*; and in* EP 0 354 441*.*

The above listing should be not limited to mediators other than oxygen in accordance with the invention as also other commercially available electron mediators known to the person skilled in the art are fully comprised by the present disclosure.

In another very specific aspect of the present disclosure the GOx variants according to the sequences SEQ ID NO: 2 to SEQ ID NO: 9 exhibit at least a mediator activity of 150% for the mediator N,N-bis(2-hydroxyethyl)-4-nitrosoaniline as compared to the respective mediator activity of the GOx-T30V; I94V according to SEQ ID NO: 1 when determined by the mediator assay as outlined in the Materials and Methods section under item ff).

In another very specific aspect of the present disclosure the GOx variants having besides the two substitutions T30V and I94V according to SEQ ID NO: 1, the additional amino acid substitutions
- A173V; A332S; F414Y; and V560A or
- A173V; A332S; and F414Y,
exhibit at least a mediator activity of 150% for the mediator N,N-bis(2-hydroxyethyl)-2-methoxy-4-nitrosoaniline as compared to the respective mediator activity of the GOx-T30V; I94V according to SEQ ID NO: 1 when determined by the mediator assay as outlined in the Materials and Methods section under item ff).

The above substitutions
- A173V; A332S; F414Y; and V560A or
- A173V; A332S; and F414Y
in addition to the two substitutions T30V and I94V according to SEQ ID NO: 1, reflect another characteristic of the herein provided GOx variants, namely the capability to accept certain electron mediators other than oxygen for electron transfer, in this case the N,N-bis(2-hydroxyethyl)-2-methoxy-4-nitrosoaniline mediator.

With this context, the expression "certain electron mediator(s) other than oxygen" denotes an electron mediator or electron mediators other than oxygen selected from the group comprising nitrosoanilines and derivatives thereof, azo-compounds, phenazines and derivatives thereof, phenothiazines and derivatives thereof, phenoxazines and derivatives thereof, ferrocenes and derivatives thereof, potassium ferricyanide, Ru- and Os-complexes, quinones and derivatives thereof, indophenols, viologens, tetrathiafulvalene and derivatives thereof, and phthalocyanines.

In the **first aspect** the present invention relates to the subject matter of
a glucose oxidase according to SEQ ID NO: 1, selected from the group consisting of
a) a glucose oxidase according to SEQ ID NO: 1, having besides the two amino acid substitutions T30V and I94V, at least one additional amino acid substitution in any of the five positions selected from the group S53; A173; A332; F414; V560 in said SEQ ID NO: 1; or
b) a glucose oxidase that exhibits at least 70%, particularly at least 80%, or > 90% amino acid sequence identity to the glucose oxidase according to a) provided that the glucose oxidase of b) is having besides the two amino acid substitutions T30V and I94V, at least one additional amino acid substitution in any of the five positions selected from the group S53; A173; A332; F414; V560 according to SEQ ID NO: 1,
   and provided that the glucose oxidase according to b) exhibits at least 70%, particularly at least 80%, or > 90% of the enzyme activity of the glucose oxidase according to a), and exhibits at least 70%, particularly at least 80%, or > 90% of the enzyme specificity for glucose of the glucose oxidase according to a),
   and provided that the glucose oxidase according to b) exhibits at least a 5-fold reduced activity for oxygen as electron acceptor of the glucose oxidase according to SEQ ID NO: 1 or exhibits at least a 1.5-fold increased activity for electron mediators other than oxygen of the glucose oxidase according to SEQ ID NO: 1, or both; or
c) an active fragment of a glucose oxidase according to a) or b), provided that in the active fragment according to c) the amino acid substitutions as outlined under a) or b) are preserved when compared to the glucose oxidase according to a) or b),
   and provided that the glucose oxidase according to c) exhibits at least 70%, particularly at least 80%, or > 90% of the enzyme activity of the glucose oxidase according to a), and exhibits at least 70%, particularly at least 80%, or > 90% of the enzyme specificity for glucose of the glucose oxidase according to a),
and provided that the glucose oxidase according to c) exhibits at least a 5-fold reduced activity for oxygen as electron acceptor of the glucose oxidase according to SEQ ID NO: 1 or exhibits at least a 1.5-fold increased activity for electron mediators other than oxygen of the glucose oxidase according to SEQ ID NO: 1, or both, and wherein the amino acids for additional substitution(s) are selected from the group
Phe for the position S53; and/or
Ile, Thr, Val for the position A173; and/or
Ser, Val, Thr for the position A332; and/or
Ile, Leu, Met, Val for the position F414; and/or
Leu, Pro, Thr, for the position V560.

In the context of the present specification the expressions "additional modification(s) in the amino acid sequence" and "additional amino acid substitution(s)" denote a substitution of at least one amino acid by any of Phe for the position S53 and/or, Ile, Thr, Val for the position A173; and/or, Ser, Val, Thr for the position A332; and/or, Ile, Leu, Met, Val for the position F414; and/or Leu, Pro, Thr, for the position V560, according to SEQ ID NO: 1. Further, the above expressions encompasses also cooperative amino acid substitutions by any of Phe for the position S53 and/or, Ile, Thr, Val for the position A173; and/or, Ser, Val, Thr for the position A332; and/or, Ile, Leu, Met, Val for the position F414; and/or Leu, Pro, Thr, for the position V560, or chemical equivalents thereof, provided that at least two, or three, or four, or five positions are substituted in any of positions selected from the group S53; A173; A332; F414; and V560 according to SEQ ID NO: 1.

The term "enzyme activity" in relation to the GOx variants of the invention specifies a protein that catalyzes the oxidation of beta-D-glucose into D-glucono-1,5-lactone (D-glucose + O₂ → Gluconolactone + H₂O₂), which then may hydrolyze to gluconic acid.

In a **second aspect** the present invention relates to the subject matter of the following embodiments that are specifying the first aspect of the invention:
In one aspect the subject matter of the present invention is a GOx variant according to the first aspect of the invention, wherein
- said activity for oxygen as electron acceptor is determined by ABTS assay, comprising the steps of
   a) 75 µE of sample enzyme solution are transferred to a 96-well flat-bottom microplate containing 100 µE of phosphate buffer (pH 7)
   b) 20 µE of reaction mixture is added to each well resulting in the following concentrations: 0.91 U/mL HRP (horseradish peroxidase); 2.3 mM ABTS (2,2'-azino-bis(3-ethylbenzothiazoline-6-sulphonic acid))
   c) the reaction starts by adding 25 µL glucose substrate solution and subsequent shaking of the plate at 1000 rpm for 30 sec
   d) the oxidation of ABTS is kinetically determined at 414 nm using a microplate reader
- said activity for electron mediators other than oxygen is determined by mediator assay, comprising the steps of
   a) 75µL sample of enzyme solution is transferred to a 96-well flat-bottom microplate
   b) 100 µL of mediator solution (19.05 mM N,N-bis(2-hydroxyethyl)-4-nitrosoaniline); 5% (w/w) Polyvinylpyrrolidone, pH 7 and 20 µL of 25 mM Phosphomolybdic acid are added
   c) the reaction starts by adding 25 µL glucose substrate solution and subsequent shaking of the plate at 1000 rpm for 1 min
   d) the kinetic of Phosphomolybdic acid reduction is monitored at 700 nm using a microplate-reader.

In another aspect that is related to the above aspect, the electron mediators other than oxygen are selected from the group comprising nitrosoanilines and derivatives thereof, azo-compounds, phenazines and derivatives thereof, phenothiazines and derivatives thereof, phenoxazines and derivatives thereof, ferrocenes and derivatives thereof, potassium ferricyanide, Ru- and Os-complexes, quinones and derivatives thereof, indophenols, viologens, tetrathiafulvalene and derivatives thereof, and phthalocyanines.

In another aspect that is related to the above aspect, the subject matter of the present invention is a GOx variant, having besides the two substitutions T30V and I94V according to SEQ ID NO: 1, additional two, or three, or four, or five amino acid substitutions in any of the six positions selected from the group S53; A173; A332; F414; V560 according to the SEQ ID NO: 1.

In another aspect that is related to the above aspect, the present invention provides for a GOx variant, wherein the amino acids for said additional substitution(s) are selected from the group Phe for the position S53 and/or, Ile, Thr, Val for the position A173; and/or, Ser, Val, Thr for the position A332; and/or, Ile, Leu, Met, Val for the position F414; and/or Leu, Pro, Thr, for the position V560 , according to SEQ ID NO: 1.

In another aspect that is related to any of the above aspects, the present invention provides for GOx variants, wherein besides the substitutions T30V and I94V according to the SEQ ID NO: 1, at least one additional amino acid substitution in the position(s) F414 and/or V560 is/are combined with at least one amino acid substitution in the position(s) A173 and/or A332.

In another aspect that is related to any of the above aspects, the present invention provides for a GOx variant according to SEQ ID NO: 4, having besides the substitutions T30V and I94V according to the SEQ ID NO: 1, the additional amino acid substitutions A173I; A332S; and F414L.

In another aspect that is related to any of the above aspects, the present invention provides for a GOx variant according to SEQ ID NO: 3, having besides the substitutions T30V and I94V according to the SEQ ID NO: 1, the additional amino acid substitutions A173V; A332S; F414I; and V560T.

In another aspect that is related to any of the above aspects, the present invention provides for a GOx variant according to SEQ ID NO: 6, having besides the substitutions T30V and I94V according to the SEQ ID NO: 1, the additional amino acid substitutions A173V; A332N; F414V; and V560L.

In another aspect that is related to any of the above aspects, the present invention provides for a GOx variant, having an at least 5-fold reduced activity for oxygen as electron acceptor when compared to wild-type GOx of *Aspergillus niger* and/or compared to the GOx according to SEQ ID NO: 1 by means of ABTS assay, comprising the steps of
a.) 75 µL of sample enzyme solution are transferred to a 96-well flat-bottom microplate containing 100 µL of phosphate buffer (pH 7)
b.) 20 µL of reaction mixture is added to each well resulting in the following concentrations: 0.91 U/mL HRP (horseradish peroxidase); 2.3 mM ABTS (2,2'-azino-bis(3-ethylbenzothiazoline-6-sulphonic acid))
c.) the reaction starts by adding 25 µL glucose substrate solution and subsequent shaking of the plate at 1000 rpm for 30 sec
d.) the oxidation of ABTS is kinetically determined at 414 nm using a microplate reader.

In another aspect that is related to any of the above aspects, the present invention provides for a GOx variant, whereby said GOx variant exhibits an at least 1.5-fold increased activity for mediators other than oxygen as compared to the GOx according to SEQ ID NO: 1, when determined by mediator assay, comprising the steps of
a.) 75µL sample of enzyme solution is transferred to a 96-well flat-bottom microplate
b.) 100 µL of mediator solution (19.05 mM N,N-bis(2-hydroxyethyl)-4-nitrosoaniline); 5% (w/w) Polyvinylpyrrolidone, pH 7 and 20 µL of 25 mM Phosphomolybdic acid are added
c.) the reaction starts by adding 25 µL glucose substrate solution and subsequent shaking of the plate at 1000 rpm for 1 min
d.) the kinetic of Phosphomolybdic acid reduction is monitored at 700 nm using a microplate-reader.

In another aspect that is related to any of the above aspects, the present invention provides for a GOx variant, exhibiting a glucose specificity of at least 99.9% and/or a galactose specificity < 4% and/or a maltose specificity < 0.3% and/or a xylose specificity < 6% and/or a maltotriose specificity < 0.1%, when determined by mediator assay, comprising the steps of
a.) 75µL sample of enzyme solution is transferred to a 96-well flat-bottom microplate
b.) 100 µL of mediator solution (19.05 mM N,N-bis(2-hydroxyethyl)-4-nitrosoaniline); 5% (w/w) Polyvinylpyrrolidone, pH 7 and 20 µL of 25 mM Phosphomolybdic acid are added
c.) the reaction starts by adding 25 µL of the respective sugar substrate solution and subsequent shaking of the plate at 1000 rpm for 1 min
d.) the kinetic of Phosphomolybdic acid reduction is monitored at 700 nm using a microplate-reader.

In the context of the present disclosure the expression "specific for glucose or specific for the substrate glucose" denotes activity of the herein provided GOxs for the substrate glucose of 100% when determined by mediator assay as outlined in the Material and Methods section below. By implication, the residual activity of the herein provided GOxs for sugars other than glucose such as galactose, maltose, xylose, and maltotriose is < 6% when determined by mediator assay as outlined in the Material and Methods section under item ff).

In another aspect that is related to any of the above aspects, the present invention provides for GOx variants, exhibiting an activity of > 400%, or > 500%, or > 600% for Nitrosoaniline Mediator for electron transfer when compared to the Nitrosoaniline Mediator activity of the GOx according to SEQ ID NO: 1 by means of mediator assay, comprising the steps of
a.) 75µL sample of enzyme solution is transferred to a 96-well flat-bottom microplate
b.) 100 µL of mediator solution (19.05 mM N,N-bis(2-hydroxyethyl)-4-nitrosoaniline); 5% (w/w) Polyvinylpyrrolidone, pH 7 and 20 µL of 25 mM Phosphomolybdic acid are added
c.) the reaction starts by adding 25 µL glucose substrate solution and subsequent shaking of the plate at 1000 rpm for 1 min
d.) the kinetic of Phosphomolybdic acid reduction is monitored at 700 nm using a microplate-reader.
and an oxygen activity of ≤ 30%, or < 25%, or < 20%, particularly < 15%, or ≤ 10% when compared to the oxygen activity of the GOx according to SEQ ID NO: 1 by means of ABTS assay, comprising the steps of
a.) 75 µL of sample enzyme solution are transferred to a 96-well flat-bottom microplate containing 100 µL of phosphate buffer (pH 7)
b.) 20 µL of reaction mixture is added to each well resulting in the following concentrations: 0.91 U/mL HRP (horseradish peroxidase); 2.3 mM ABTS (2,2'-azino-bis(3-ethylbenzothiazoline-6-sulphonic acid))
c.) the reaction starts by adding 25 µL glucose substrate solution and subsequent shaking of the plate at 1000 rpm for 30 sec
d.) the oxidation of ABTS is kinetically determined at 414 nm using a microplate reader.

In another aspect the subject matter of the present invention is related to an isolated polynucleotide encoding a GOx variant according to any of the above aspects or an active fragment thereof.

In another aspect, the present invention provides for an active (functional) equivalent/fragment of the GOx variants according to the invention.

The terms "active equivalent(s)/fragment(s) thereof" or synonymous "functional equivalent(s)/fragment(s) thereof" refer to any modified and thus optimized GOx variant in accordance with the present invention, whereby at least one amino acid is missing or substituted by another amino acid as in the corresponding sequence according to SEQ ID NO: 1, with the proviso that such equivalent(s)/fragment(s) still exhibit the essential properties as regards enzyme activity, enzyme specificity and the significantly reduced oxygen consumption rates and/or significantly increased activity for specific mediators other than oxygen, by having present at least the substitutions T30V and I94V according to SEQ ID NO: 1, and further having at least one additional amino acid substitution in any of the positions selected from the group consisting of S53; A173; A332; F414; and V560 according to SEQ ID NO: 1.

In a specific aspect of the present invention the functional equivalent(s)/fragment(s) of the GOx variants according to the invention encompass amino acid sequence(s) being at least 70% homologous, particularly at least 80% homologous or > 90% homologous to the sequences according to the SEQ ID NO: 2 to SEQ ID NO: 9.

In another aspect the subject matter of the present invention is related to a method of detecting, determining or measuring glucose in an *ex vivo* sample by a GOx variant according to the present invention or an active fragment thereof; said detection, determination or measurement is comprising contacting an *ex vivo* sample with said GOx or an active fragment thereof.

In another aspect that is related to the above aspect, said detection, determination or measurement of glucose is performed using a sensor or a test strip device.

The present invention also relates to a process for producing the GOx variants of the instant invention, to nucleic acids encoding said enzymes, to vectors, host cells, and a method of detecting, determining or measuring glucose in a sample using said enzymes, and to devices comprising said enzymes as well.

In a specific aspect, the present invention provides a device for assaying glucose in a sample comprising at least one of the GOx variants of the invention and an electron mediator other than oxygen.

In another specific aspect of the invention, the electron mediator or mediator other than oxygen is selected from the group comprising nitrosoanilines and derivatives thereof, azo-compounds, phenazines and derivatives thereof, phenothiazines and derivatives thereof, phenoxazines and derivatives thereof, ferrocenes and derivatives thereof, potassium ferricyanide, Ru- and Os-complexes, quinones and derivatives thereof.

Another specific aspect of the invention that is related to the subject matter of any of the above aspects is a GOx variant, having besides the two substitutions T30V and I94V according to the SEQ ID NO: 1, at least one additional amino acid substitution in any of the four positions selected from the group A173; A332; F414; V560 of the SEQ ID NO: 1.

One of the major applications of the herein provided GOx variants intended by the instant invention is their use in test strips to monitor the blood-glucose level in *ex vivo* samples of diabetic patients. Of course many kinds of samples may be investigated. In particular, bodily fluids like blood, serum, and plasma are the sources for such samples.

Thus, in another aspect, the present invention provides an enzyme electrode bearing at least one of the GOx variants of the invention, which is immobilized on said electrode.

In another aspect, the present invention provides an enzyme sensor for assaying glucose comprising the enzyme electrode of the invention as a working electrode and thus bearing at least one of the GOx variants of the invention.

In yet another aspect, the present invention provides a kit for assaying glucose in a sample comprising at least one GOx variant pursuant to the invention and an electron mediator other than oxygen.

A kit for the measurement of glucose may be constructed using at least one GOx variant of the present invention. In addition to the GOx variant of the invention, the kit contains buffer necessary for the measurement, an appropriate electron mediator other than oxygen, in particular a Nitrosoaniline Mediator and, if necessary, enzymes such as peroxidases, and standard solution of glucose for the preparation of a calibration curve and an instruction for use.

The concentration of the glucose in a sample may be determined by measuring the amount of electrons generated by the enzyme reaction. Various sensor systems have been known in the art, including carbon electrode, metal electrode, and platinum electrode. The GOx variant of the present invention is immobilized on the electrode. Examples of the means for immobilization include cross-linking, encapsulating into a macromolecular matrix, coating with a dialysis membrane, optical cross-linking polymer, electroconductive polymer, oxidation-reduction polymer, and any combination thereof.

The assay device may have a similar structure as any of conventional, commercially available amperometric biosensor test strips for monitoring the blood glucose level. One example of such a device has two electrodes (working electrode and reference or counter electrode) positioned on an insulating substrate, a reagent port and a sample receiver. The reagent port contains the GOx variant of the invention and a mediator other than oxygen. When a sample such as blood sample is added to the sample receiver, glucose contained in the sample will react with the GOx variant, thereby the electron transfer is indicative for the amount of glucose in the sample. Typical examples of electrochemical sensors suited for the determination of enzyme substrates are known, e.g. from WO 2004/113900 and US 5,997,817. As an alternative to electrochemical sensors, optical detection technologies might be used. Typically, such optical devices are based on color changes that occur in a reagent system comprising the enzyme, an electron mediator and an indicator. The color changes can be quantified using fluorescence, absorption or remission measurements. Typical examples of optical devices suited for the determination of enzyme substrates are known, e.g. from US 7,008,799; US 6,036,919, and US 5,334,508.

In another aspect, the present invention provides an expression vector containing the isolated polynucleotide encoding a GOx variant according to the invention or an active fragment thereof.

In another aspect, the present invention provides a host cell comprising the expression vector containing the isolated polynucleotide encoding a GOx variant according to the invention or an active fragment thereof, also herein referred to as transformant.

In another aspect, the present invention provides a process for producing a GOx variant according to the invention or an active fragment thereof, the process comprises culturing the above-described transformant.

In another aspect, the present invention provides a GOx variant according to the invention or an active fragment thereof obtainable by the above-described process.

In another specific aspect, the herein provided expression vector preferably comprises all or a part of one of the DNA sequences encoding for a GOx variant of the present invention.

Suitable expression vectors containing the desired coding and control sequences of the herein provided GOx variants may be constructed using standard recombinant DNA techniques known in the art, many of which are described in Sambrook et al., in "Molecular Cloning: A Laboratory Manual" (1989), Cold Spring Habor, NY Cold Spring Habor Laboratory Press.

Suitable host cells include, for example, *E.coli* HB101 (ATCC33694) available from Promega (2800 Woods Hollow Road, Madison, WI, USA), XL1-Blue MRF' available from Stratagene (11011 North Torrey Pine Road, La Jolla, CA, USA) and the like. Suitable *Pichia* host cells include, for example *P. pastoris* X33 or *P. pastoris* KM71H available from Invitrogen (5791 Van Allan Way, Carlsbad, CA 92008, USA)

The recombinant production of the GOx variants according to the present invention may be conducted in hosts known in the art. Suitable hosts may be selected from strains of filamentous fungi as *e.g. Aspergillus niger, Aspergillus sojae* and *Aspergillus oryyzae.* Suitable hosts may be selected from strains of yeast as e.g. *Pichia pastoris, Saccharomyces cerevisiae* and *Hansenula polymorpha.*

In another specific aspect the GOx variants or functional equivalents thereof according to the present invention are obtainable by expression of a polynucleotide encoding said GOx variants in yeast, in particular in *Saccharomyces cerevisiae.*

Expression vectors may be introduced into host cells by various methods known in the art. For example, transformation of host cells with expression vectors can be carried out by polyethylene glycol mediated protoplast transformation method (Sambrook *et al.* 1989, supra). However, other methods for introducing expression vectors into host cells, for example, electroporation, ballistic DNA injection, or protoplast fusion, can also be employed.

Once an expression vector containing a GOx variant pursuant to the invention has been introduced into an appropriate host cell, the host cell may be cultured under conditions permitting expression of the desired GOx variant pursuant to the invention. Host cells containing the desired expression vector (and thus bearing the DNA sequence encoding for the GOx variant pursuant to the invention), can be easily identified by e.g. antibiotic selection or complementation of auxotrophic mutants and selection from minimal medium [J. Sambrook, D. W. Russell: "Molecular Cloning: a laboratory manual", 3rd edition, Cold Spring Harbor, New York (2001*)].* The expression of the herein provided GOx variants can be identified by different methods like measuring production of GOx mRNA transcripts, detection of the gene product immunologically or detection of the enzymatic activity of the gene product know to the person skilled in the art. In particular, an enzymatic assay should be applied as outlined under the mediator assay in the Materials and Methods section under item ff). In addition, the herein provided GOx variants can be identified by the significantly reduced oxygen-consumption rates as in accordance with the invention when determined by the ABTS assay as outlined in the Materials and Methods section set out under item gg).

In another aspect of the invention the herein described polypeptides for the inventive GOx variants are obtainable by production in host cells expressing a DNA sequence encoding for one of the GOx variants of the present invention. The polypeptides of the present invention may also be obtained by *in vitro* translation of the mRNA encoded by a DNA sequence encoding for one of the GOx variants of the instant invention. For example, the DNA sequences may be inserted into a suitable expression vector, which in turn may be used in an *in vitro* transcription/translation system.

An expression vector comprising an isolated polynucleotide as defined and described above operably linked to a promoter sequence capable of promoting its expression in a cell-free peptide synthesis system represents another specific aspect of the present invention.

The polypeptides produced *e.g.* by procedures as describe above, may then be isolated and purified using various routine protein purification techniques. For example, chromatographic procedures such as ion exchange chromatography, gel filtration chromatography and affinity chromatography may be employed.

In another aspect, the present invention provides a pharmaceutical composition comprising at least one GOx variant according to the invention or an active fragment thereof.

In another aspect, the GOx variants according to the invention retain its inherent property to be specific for the substrate glucose, but due to the at least one additional amino acid substitution in the amino acid sequence in specific positions besides the substitutions T30V and I94V according to SEQ ID NO: 1, the maximal residual oxygen activity is reduced to ≤ 30% when determined by the ABTS assay as outlined in the Materials and Methods section under item gg). Concomitantly, the herein provided GOxs exhibit an enzyme activity of > 400%, or > 500%, or > 600% for electron mediators other than oxygen when determined by mediator assay as outlined in the Materials and Methods section below under item ff).

In view of the above, the present invention combines in the herein provided GOx variants
i) the glucose specificity properties of GOxs
ii) with the oxygen-independent enzyme activity properties of GDHs via a shift from oxidase activity towards dehydrogenase activity
iii) and optionally an increased activity for certain electron mediators other than oxygen
for achieving accurate glucose measurements, in particular accurate blood glucose measurements.

With the context of the GOx variants pursuant to the present invention, the expression "shift from oxidase activity towards dehydrogenase activity" denotes
- a decrease in oxygen activity starting from 1.0 of the GOx-T30V; I94V as reference towards ≤ 0.3, or < 0.25, or < 0.2, particularly < 0.15, or ≤ 0.1 residual oxygen activity of the GOx variants pursuant to the invention when determined by the ABTS assay as outlined in the Materials and Methods sections under item gg), and
- a concomitant increase in dehydrogenase activity starting from a mediator activity 1.0 of the GOx-T30V; I94V as reference towards at least 1.5 mediator activity of the GOx variants pursuant to the invention when determined by the mediator assay as outlined in the Materials and Methods section under item ff).

The above values represent the ratio (quotient) between the enzyme activity of the GOx variants of the invention in terms of oxygen activity and mediator activity, and the respective enzyme activity of the GOx-T30V; I94V as references. The ratio (quotient) is based on both GOx activities (oxygen activity and mediator activity in [U/mg]) when determined by ELISA as outlined in the Materials and Methods section under item ee).

Each of the nucleic acid molecules according to the sequences SEQ ID NO: 11 to SEQ ID NO: 17 according to the invention encode a modified polypeptide or fragment thereof, which is derived from the SEQ ID NO: 10 (GOx-T30V; I94V) encoding a polypeptide according to SEQ ID NO: 1.

The term "GOx-T30V; I94V" as used herein denotes a GOx based on the *Aspergillus niger* wild-type (WT) sequence according the SEQ ID NO: 2, further having the two substitutions T30V and I94V in its polypeptide sequence, i.e. the SEQ ID NO: 1.

The term "GOx double-mutant" as used herein equally denotes a GOx based on the *Aspergillus niger* WT sequence according the SEQ ID NO: 2, further having the two substitutions T30V and I94V in its polypeptide sequence, i.e. the SEQ ID NO: 1. Said GOx double-mutant is derived from Zhu *et al.* (2006; 2007).

Pursuant to the invention the nucleic acid molecule corresponding to SEQ ID NO. 1 (i.e. SEQ ID NO: 10) was used to further improve the kinetic properties of the GOx enzyme in accordance with the invention, characterized by a shift from oxidase activity towards dehydrogenase activity. This was achieved by the inventors via specific nucleotide sequence modifications resulting in amino acid substitutions in at least one of the positions S53; A173; A332; F414; and V560, whereas the two substitutions T30V and I94V have been already present according to SEQ ID NO: 1.

In another aspect, the present invention provides a GOx variant having besides the two substitutions T30V and I94V according to the SEQ ID NO: 1, additional two, or three, or four, or five acid substitutions in any of the five positions selected from the group S53; A173; A332; F414; V560 of the SEQ ID NO: 1, thereby exhibiting cooperative effects on the GOx enzyme activity.

The expression "cooperative effects on GOx enzyme activity" with the context of the present invention denotes at least two additional amino acid substitutions in any of the positions S53; A173; A332; F414; and V560 besides the two substitutions T30V and I94V according to SEQ ID NO: 1, having effects on GOx enzyme activity in terms of decreasing the GOx oxygen-consumption rate.

Further, "cooperative effects on GOx enzyme activity" with the context of the present invention denotes at least two additional amino acid substitutions in any of the positions S53; A173; A332; F414; and V560 besides the two substitutions T30V and I94V according to SEQ ID NO: 1, having effects on GOx enzyme activity in terms of decreasing the GOx oxygen-consumption rate and/or increasing the GOx mediator activity for electron mediators other than oxygen.

In addition, the expression "cooperative effects on GOx enzyme activity" with the context of the present invention denotes at least two additional amino acid substitutions in any of the positions S53; A173; A332; F414; and V560 besides the two substitutions T30V and I94V according to SEQ ID NO: 1, having effects on GOx enzyme activity in terms of accepting certain electron mediators other than oxygen for electron transfer. In a specific aspect, the present invention provides GOx variants having besides the two amino acid substitutions T30V and I94V according to SEQ ID NO: 1, additional amino acid substitutions in the positions F414 by Ile, Leu, Met, Val, and V560 by Leu, Pro, Thr, combined with amino acid substitutions in the positions A173 by Ile, Thr, Val; and A332 by Ser, Val, Thr..

In a specific aspect, the present invention provides GOx variants having besides the two amino acid substitutions T30V and I94V according to SEQ ID NO: 1, additional combined amino acid substitutions in the positions F414 by Ile, Leu, Met, Val and V560 by Leu, Pro, Thr..

A further specific aspect of the invention is related to the above aspect and provides for GOx variants, having besides the two amino acid substitutions T30V and I94V according to SEQ ID NO: 1, the additional combined amino acid substitutions F414M or F414V and V560P or V560L.

In a further specific aspect, the present invention provides for GOx variants, having besides the two amino acid substitutions T30V and I94V according to SEQ ID NO: 1, the additional amino acid substitutions F414M or F414V, combined with the amino acid substitution(s) V560P or V560L and/or A173I or A173V and/or A332S or A332V or A332T.

In a further specific aspect, the present invention provides GOx variants having besides the two amino acid substitutions T30V and I94V according to SEQ ID NO: 1, the additional amino acid substitutions F414M or F414V, combined with the amino acid substitution(s) V560P or V560L and/or A173I or A173V and/or A332S or A332V or A332T.

A further specific aspect of the invention is related to the above aspect and provides for GOx variants, having besides the two amino acid substitutions T30V and I94V according to SEQ ID NO: 1, at least two combined amino acid substitutions selected from any of the above-mentioned substitutions.

The GOx specificity for electron mediators other than oxygen can be determined by the mediator assay as outlined in the Materials and Methods section under item ff).

In another specific aspect, the present invention provides for GOx variants having a mediator activity of > 400 %, or > 450 %, or > 500 %, or > 550 %, or even > 600% for the electron mediator N,N-bis(2-hydroxyethyl)-4-nitrosoaniline when determined by mediator assay as outlined in the Materials and Methods section under item ff).

In another specific aspect, the present invention provides for GOx variants, having an at least 5-fold reduced activity for oxygen as electron acceptor, or at least a 6-fold reduced activity for oxygen as electron acceptor, or at least a 7-fold reduced activity for oxygen as electron acceptor when compared to GOx-WT or compared to the GOx-T30V; I94V according to SEQ ID NO: 1 by means of ABTS assay as outlined in the Materials and Methods section under item gg).

In another specific aspect, the present invention provides for GOx variants, having a remaining oxidase activity of ≤ 30% of that of the GOx WT oxidase activity or of that of the GOx-T30V; I94V oxidase activity according to SEQ ID NO: 1, or particularly having a remaining oxidase activity of < 20 %, or < 15% of that of the GOx-WT oxidase activity or of that of the GOx-T30V; I94V oxidase activity according to SEQ ID NO: 1, or even more particularly having a remaining oxidase activity of < 10% of that of the GOx-WT oxidase activity or of that of the GOx-T30V; I94V oxidase activity according to SEQ ID NO: 1, when determined by the ABTS assay as outlined in the Materials and Methods section under item gg)

In a furthermore specific aspect of the invention a functional equivalent/fragment of the nucleotide molecules as given in the SEQ ID NO: 11 to SEQ ID NO: 17 is a corresponding RNA molecule, which is encoded by said DNA sequence or a sequence being substantially complementary to the sequence of the SEQ ID NO: 11 to SEQ ID NO: 17.

In the context of the present invention the term "RNA molecule" is meant to refer to a linear polymer of ribonucleotide molecules, which is single-stranded and serves as a template for protein synthesis of the herein provided GOx variants according to the SEQ ID NO: 3 to SEQ ID NO: 9.

In a further specific aspect of the present invention the degree or percentage of homology is at least 70%, or at least 80%, or at least 90%, or at least 95%, or at least 99% or 100% to the SEQ ID NO: 3 to SEQ ID NO: 9; provided that they exhibit the same substitutions as outlined throughout the specification and further exhibit essentially the same properties as the GOx variants according to the present invention, those essential properties being enzyme activity, enzyme specificity for glucose and the significantly reduced oxygen-consumption rates and/or the significantly increased activity for specific mediators other than oxygen.

Sequence identity may be determined by the BLAST algorithm, the Basic Local Alignment Search Tool (BLAST) *[*Altschul, S.F. et al. 1990. J. Mol. Biol. 215:403*;* Altschul, S.F. et al. 1997. Nucleic Acid Res. 25:3389-3402*].* The herein mentioned percentages of amino acid sequence identity refer to the determination of sequence identity by said BLAST algorithm, wherein the region over which the homology is determined is the entire sequence of the GOx variants of the instant invention. Sequence identity may be determined by any other method known to the person skilled in the art for purpose of sequence alignment and comparison.

A person skilled in the art understands that the herein provided GOx variants may have or may have not further modifications different from the before-mentioned substitutions without changing the essential properties of said GOx variants, i.e. specificity for glucose, the significantly reduced oxygen-consumption rates and/or the significantly increased activity for specific mediators other than oxygen as in the sense of the instant invention.

Another specific aspect of the present invention is an isolated polynucleotide encoding a GOx variant or an active equivalent/fragment thereof according to the present invention. The isolated polynucleotide may be a DNA or RNA molecule or a corresponding gene thereof encoding for the following sequences as explicitly listed in the section - Sequence Listing, i.e. the SEQ ID NO: 11 to SEQ ID NO: 17.

In another specific aspect, the GOx variants provided by the instant invention exhibit temperature stability by having a residual enzyme activity of at least 70%, in particular at least 80% in the range of 30 °C to 47 °C, when determined by the mediator assay as outlined in the Materials and Methods section under item ff)

In another specific aspect, the GOx variants provided by the instant invention exhibit an temperature stability by having a residual enzyme activity of at least 10% in the range of 48 °C to 60 °C, when determined by the mediator assay as outlined in the Materials and Methods section under item ff).

The GOx variants of the present invention may be provided in various forms, for example, as a freeze-dried reagent or as a solution in an appropriate storage solution.

The herein provided GOx variants are predominantly intended for application in more accurate diabetes blood-glucose testing devices. Specifically, the oxygen-independent GOx variants of the invention utilize oxygen for electron transfer only by significantly reduced oxygen-consumption rates, this means the residual GOx activity for oxygen is ≤ 30%, or < 25%, or < 20%, particularly < 15%, or ≤ 10% when determined by the ABTS assay as outlined in the Materials and Methods section under item gg). Accordingly, the significant amount of glucose reacts in the mediator-related reaction for more adequate blood glucose measurements, whereas the reaction with the dissolved oxygen content in the blood sample is significantly reduced. This is beneficial to the diabetic patient when arterial, venous or capillary blood is the source for measurements or in case of measuring blood-glucose concentrations in different heights above sea level.

In another aspect, the present invention provides for GOx variants that exhibit significantly reduced oxygen-consumption rates and/or significantly increased mediator activity for electron mediators other than oxygen, which also results in more accurate blood-glucose measurements the diabetic patient benefits from.

### Definitions

With the context of the present specification the expressions "oxygen-dependent" and "oxygen-dependency" denote a GOx activity characterized by residual oxygen activity > 30% when determined by the ABTS assay as outlined in the Materials and Methods section under item gg).

By contrast, the expressions "oxygen-independent" and "oxygen-independency" denote a GOx activity characterized by residual oxygen activity ≤ 30%, or < 25%, or < 20%, particularly < 15%, or < 10% when determined by the ABTS assay as outlined in the Materials and Methods section under item gg).

With the context of the present invention the expression "unspecific for glucose" denotes a GOx activity for the substrate glucose of < 100%, or < 99.9%, or < 99.5% when determined by mediator assay as outlined in the Material and Methods section under item ff). By implication, the GOxs that are "unspecific for glucose" exhibit a GOx activity for sugars other than glucose such as galactose, maltose, xylose, and maltotriose of > 6% when determined by mediator assay as outlined in the Material and Methods section under item ff).

The terms "modified" or "modification" in relation to the GOx variants of the instant invention refer to a GOx protein containing besides the two amino acid substitutions T30V and I94V according to SEQ ID NO: 1, at least one additional amino acid substitution in the polypeptide sequence according to SEQ ID NO: 1, at any of the position(s) S53; A173; A332; F414; and V560. The term also denotes the respective polynucleotide or sequence-conservative variations thereof encoding such a "modified" GOx variant.

"Sequence-conservative variations" of a polynucleotide sequence are those in which a change of one or more nucleotides in a given codon position results in no alteration in the amino acid encoded at that position.

The term "GOx variant(s)" denotes glucose oxidases pursuant to the invention having the two amino acid substitutions T30V and I94V according to SEQ ID NO: 1, and at least one additional amino acid substitution in the polypeptide sequence according to SEQ ID NO: 1, at any of the position(s) S53; A173; A332; F414; and V560, characterized by exhibiting enzyme specificity for the substrate glucose, and significantly reduced oxygen-consumption rates and/or increased activity for specific mediators other than oxygen.

It should be understood that the numbering of the amino acid sequences throughout the present specification begins with "1" at the initial S (Ser, Serine) as in the mature GOx-T30V; I94V protein according to SEQ ID NO: 1.

The term "enzyme" in accordance with the invention means any substance composed wholly or largely of protein or polypeptides that catalyzes or promotes, more or less specifically, one or more chemical or biochemical reaction(s). The term "enzyme" can also refer to a catalytic polynucleotide (e.g. RNA or DNA).

The term "oxidation reaction" means in general terms a chemical or biochemical reaction involving the addition of oxygen to a substrate, to form an oxygenated or oxidized substrate or product. An oxidation reaction is typically accompanied by a reduction reaction (hence the term "redox" reaction encompasses both, oxidation and reduction). A compound is "oxidized" when it receives oxygen or loses electrons. A compound is "reduced" when it loses oxygen or gains electrons. GOxs typically catalyze the oxidation of a primary alcohol group to an aldehyde.

The term "glucose oxidase(s)" as used throughout the specification specifies a protein that catalyzes the oxidation of beta-D-glucose into D-glucono-1,5-lactone (D-glucose + O₂ → Gluconolactone + H₂O₂), which then may hydrolyzes to gluconic acid. Accordingly, the glucose oxidase is an enzyme. Further, the term "a polypeptide having the activity of a glucose oxidase" refers to a polypeptide having the afore-mentioned activity. Glucose oxidase(s) may be abbreviated by "GOx(s)" or "E.C. 1.1.3.4." The terms "GOx-WT or WT" as used throughout the specification denote the wild-type GOx of the fungus *Aspergillus niger.* The terms "GOx-T30V; I94V; or T30VI94V; or double-mutant; or parent-mutant" denote the GOx variant having the two substitutions T30V and I94V according to SEQ ID NO: 1 as described in Zhu *et al.* (2006; 2007).

The GOx variant(s) pursuant to the invention do all have in common the two substitutions T30V and I94V according to SEQ ID NO: 1, and at least one additional amino acid substitution at any of the position(s) S53; A173; A332; F414; and V560 by proteinogenic amino acids suitable for replacement in accordance with the invention, namely Phe for the position S53 and/or, Ile, Thr, Val for the position A173; and/or, Ser, Val, Thr for the position A332; and/or, Ile, Leu, Met, Val for the position F414; and/or Leu, Pro, Thr, for the position V560. Respective "EZ" numbers may abbreviate the GOx variant(s) pursuant to the invention throughout the specification text. "EZ" stands for enzyme.

The enzyme "glucose oxidase" is thus a member of the class of oxidation enzymes, which catalyzes an oxidation reaction, by adding, inserting, contributing or transferring oxygen from a source or donor to a substrate. Such enzymes are also called oxidoreductases or redox enzymes, and encompass oxygenases, hydrogenases or reductases, oxidases and peroxidases.

In this regard, the terms "oxygen donor", "oxidizing agent" and "oxidant" mean a substance, molecule or compound which donates oxygen to a substrate in an oxidation reaction. Typically, the oxygen donor is reduced (accepts electrons). Exemplary oxygen donors include for instance molecular oxygen or dioxygen (O₂), and peroxides include for instance alkyl peroxides such as t-butyl peroxide, and most preferably hydrogen peroxide (H₂O₂). "Peroxide(s)" is any compound(s) having two oxygen atoms bound to each other.

As mentioned the "activity" or "enzyme activity" of a glucose oxidase or of the GOx variants as used herein is directed to a measure of its ability to catalyze the oxidation reaction D-glucose+O₂ → Gluconolactone+H₂O₂, and may be expressed as the rate at which the product of the reaction is produced. For example glucose oxidase activity can be represented as the amount of product (Gluconolactone and/or H₂O₂) produced per unit of time, or per unit (e.g. concentration or weight) of glucose oxidase.

In general, the polypeptide(s) of the invention may also be referred to herein as being a "mutant", "mutein" or "variant" or "a modified GOx" or a "modification", meaning that it has been made, altered, derived, or is in some way different or changed from the GOx-WT of *Aspergillus niger* itself, and from the GOx-T30V; I94V according to SEQ ID NO: 1.

The GOx-WT protein of *Aspergillus niger* comprises the natural sequence of amino acids according to SEQ ID NO: 2. Accordingly, the "parent mutant" or "double-mutant" is a GOx polypeptide from which any other herein provided GOx polypeptide is derived or made from, using any methods, tools or techniques described herein. In accordance with the invention the "parent mutant" or "double-mutant" is the polypeptide according to SEQ ID NO: 1.

Consequently, a "parent polynucleotide" is one that encodes a parent polypeptide, i.e. the polynucleotide according to SEQ ID NO: 10.

The term "mutation" or "variation" or "modification" means any detectable change in genetic material, e.g. DNA or RNA, or any process, mechanism, or result of such a change. This includes gene mutations, in which the structure (e.g. DNA or RNA sequence) of a gene is altered, any gene or DNA or RNA arising from any mutation process, and any expression product (e.g. protein or polynucleotide) expressed by a modified gene or DNA sequence. Such changes also include changes in the promoter, ribosome-binding site etc.

With the context of the present disclosure the expressions "certain mediators other than oxygen", "certain electron mediators other than oxygen", "certain immobilized mediator" and "certain electron acceptors other than oxygen" denote the mediators/electron mediators selected from the group comprising nitrosoanilines and derivatives thereof, azo-compounds, phenazines and derivatives thereof, phenothiazines and derivatives thereof, phenoxazines and derivatives thereof, ferrocenes and derivatives thereof, potassium ferricyanide, Ru- and Os-complexes, quinones and derivatives thereof, indophenols, viologens, tetrathiafulvalene and derivatives thereof, and phthalocyanines.

Accordingly, the expressions "certain mediators other than oxygen", "certain electron mediators other than oxygen", "certain immobilized mediator" and "certain electron acceptors other than oxygen" in accordance with the invention also denote electron mediators, which are fast reduced by FADH2 but slow or not reduced by ascorbic acids such as derivatives of 2- [4-(dimethylamino)phenyl] -diazenecarboxamide.

### Abbreviations

AA - aminoantipyrine
HRP - horseradish peroxidase(s)
GOx - glucose oxidase (s)
GOx-WT or WT - wild-type GOx of the fungus *Aspergillus niger*
GDH - glucose dehydrogenase
ABTS - 2,2'-azino-bis(3-ethylbenzothiazoline-6-sulphonic acid)
PMO - Phosphomolybdic acid

### Figure Description

**Figure 1**

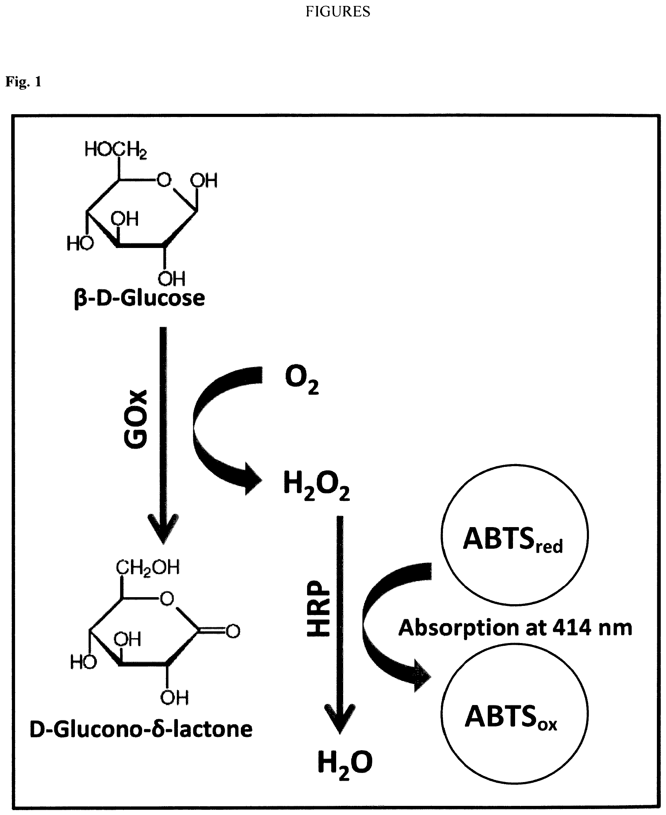

   Fig. 1 shows the principle of the activity determination on oxygen for the herein provided GOx variants: The H₂O₂ produced during the reductive half reaction of GOx and HRP is used to oxidize the chromogenic substrate ABTS. The color change of ABTS is monitored by absorption at 414 nm. This principle forms the basis of the ABTS assay as outlined in the Materials and Methods section under item gg).
**Figure 2**

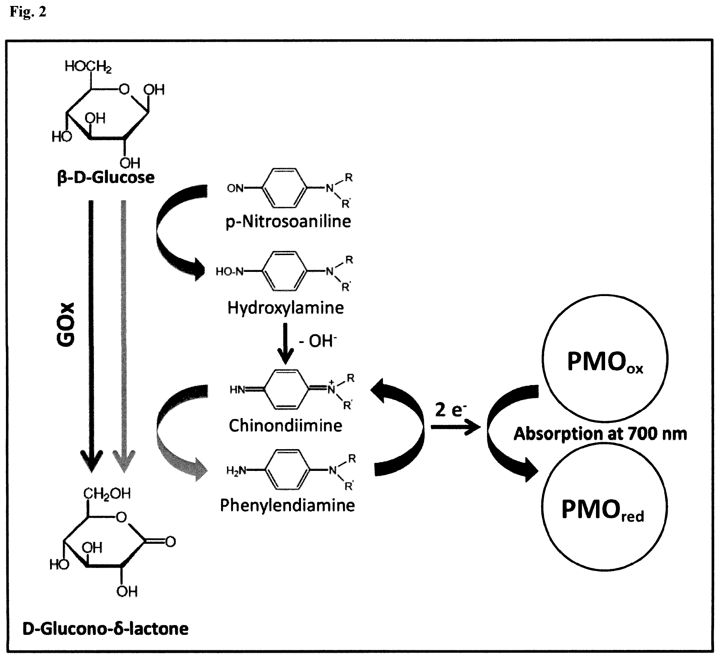

   Fig. 2 depicts the mediator assay or PMO-assay (Phosphomolybdic acid) for detection of mediated electron transfer. The mediator compound gets reduced in two steps during the reductive half reaction of GOx. The mediator transfers two electrons to the redox indicator PMO which gets subsequently reduced. The color change of PMO during the reduction is monitored by absorption at 700 nm.
**Figure 3**

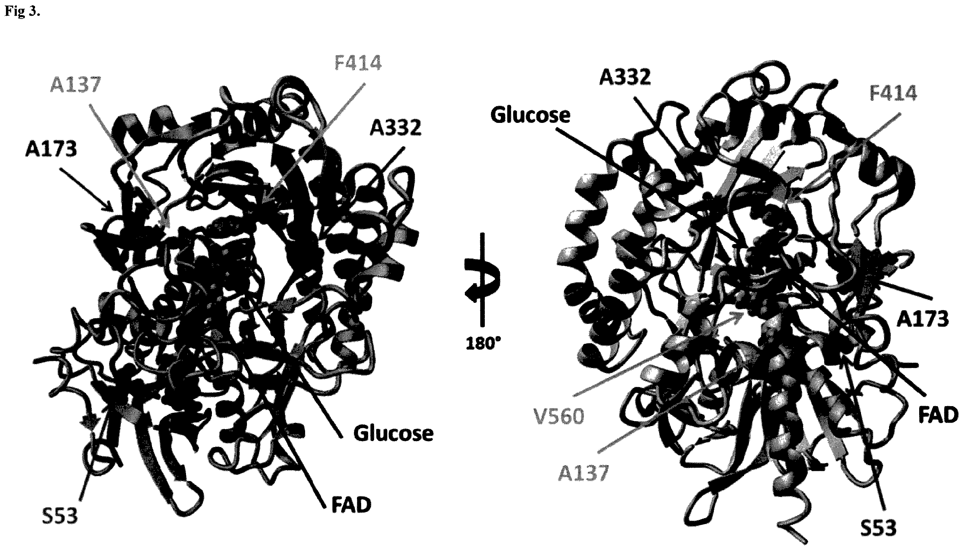

   Fig. 3 shows the distinct location of the six positions for amino acid substitutions in the protein structure pursuant to the invention.
**Figure 4**

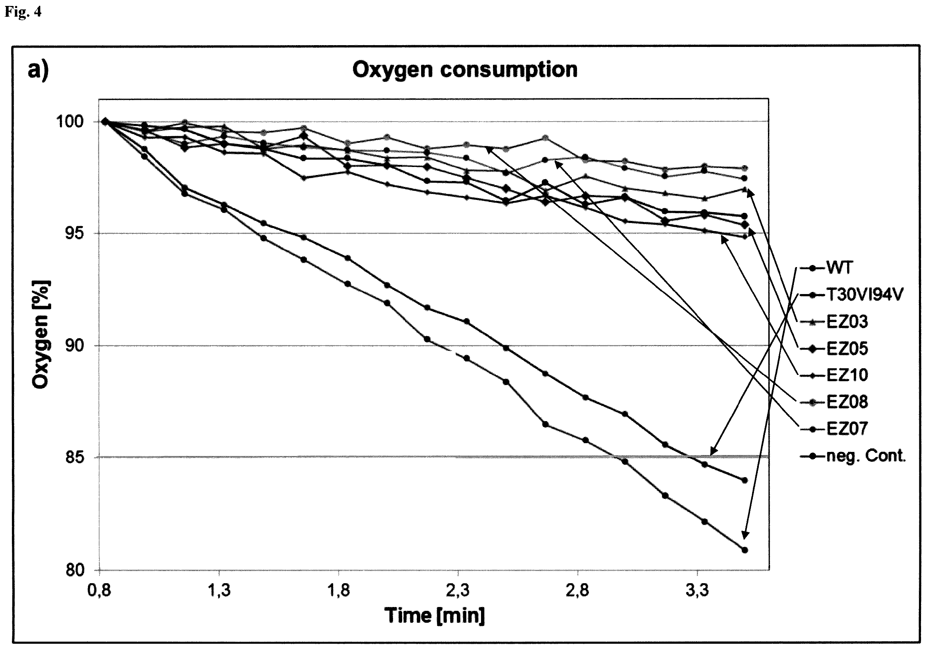

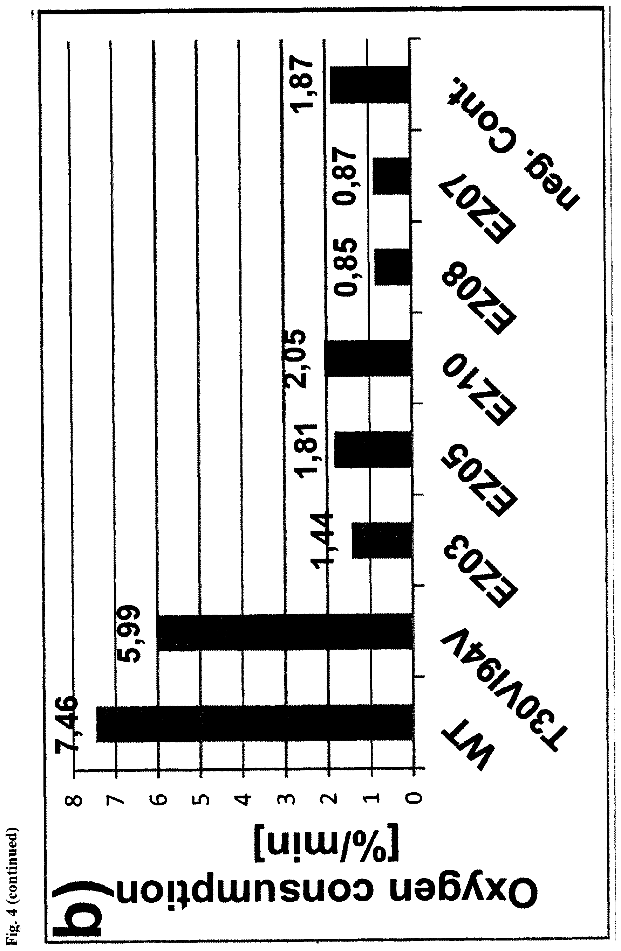

   Fig. 4 shows oxygen consumption rates of different GOx-variants pursuant to the invention: a) progression of the relative oxygen concentration as function of time; b) relative oxygen consumption rate per minute. The assay implementation is described in the Materials and Methods section under item hh). The enzymes were normalized to 2 U/L.
**Figure 5**

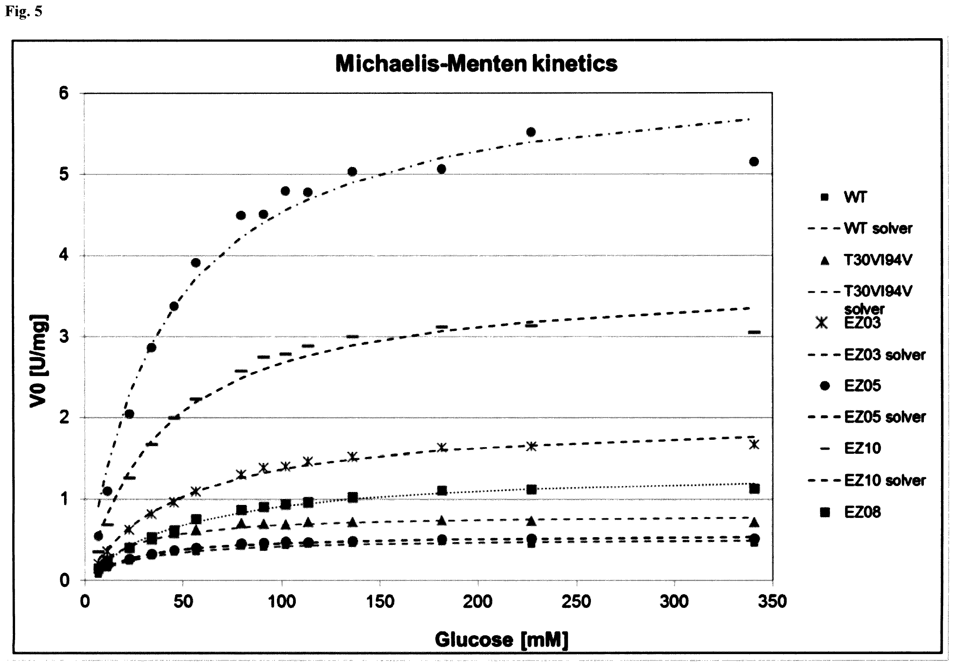

   Fig. 5 depicts Michaelis-Menten kinetics of different GOx-variants pursuant to the invention. For the activity determination the mediator assay for characterization was applied as outlined in the Material and Methods section under item ff). The dashed lines were calculated in Microsoft Excel applying the least square method.
**Figure 6**

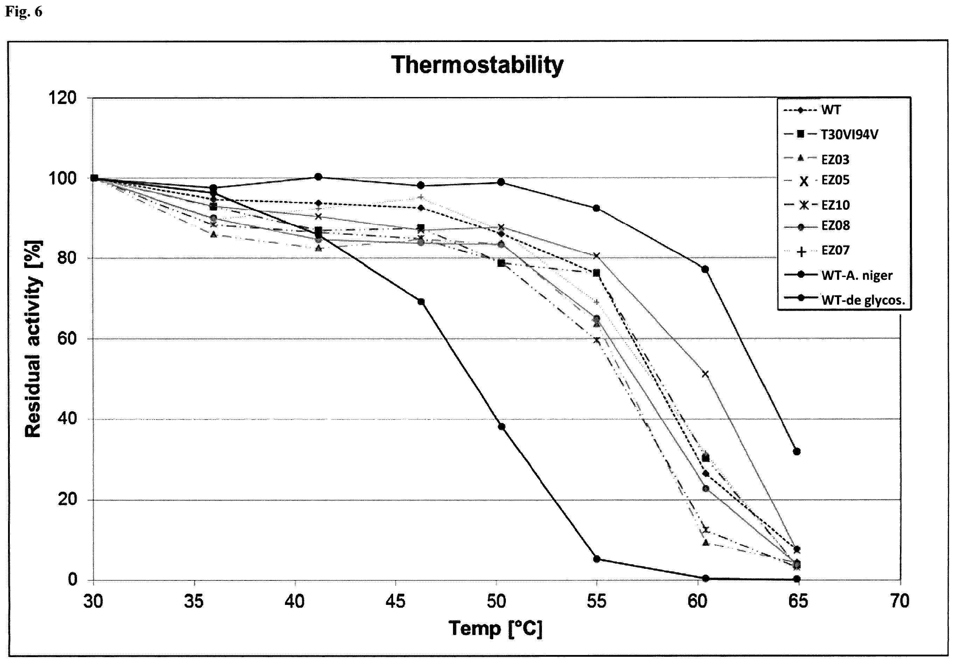

   Fig. 6 shows the thermostability properties of different GOx-variants pursuant to the invention. The assay was performed as outlined in the Material and Methods section under item ii). As additional controls de-glycosylated and hyper-glycosylated GOxs were used.
**Figure 7**

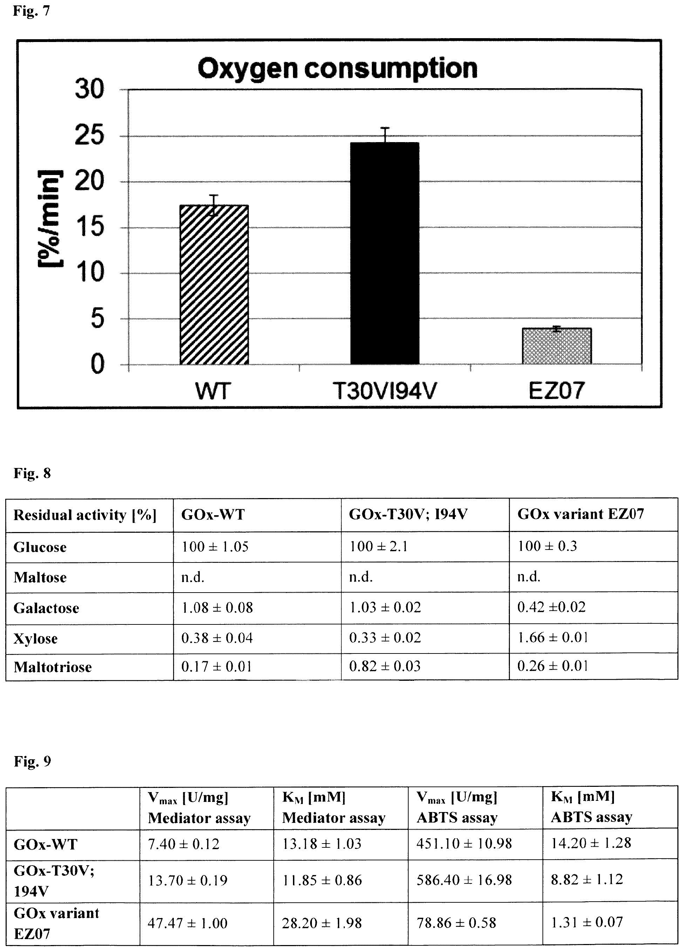

   Fig. 7 shows the relative oxygen consumption rate of the GOx-WT, the GOx-T30V; I94V and the GOx-variant V of the invention (i.e. A173V; A332S; F414I; V560T besides the substitutions T30V; I94V). The assay implementation is described the Materials and Methods section under item hh). The enzyme concentrations were normalized to 1.7 mg/L.
**Figure 8**
   Fig. 8 shows the residual activity of GOx-WT, GOx-T30V; I94V and the GOx variant EZ07 on different sugars in comparison to glucose. For the determination of residual activities the mediator assay for characterization was applied as described in the Materials and Methods section under item ff). The substrate concentration was 181.8 mM in the reaction mixtures.
**Figure 9**
   Fig. 9 shows enzyme parameters for GOx-WT, GOx-T30V; I94V and the GOx variant EZ07. The parameters were calculated according to Michaelis-Menten kinetics applying the least square method in Microsoft Excel. For the activity determination the mediator assay for characterization was applied as described in the Materials and Methods section under item ff).
**Figure 10**

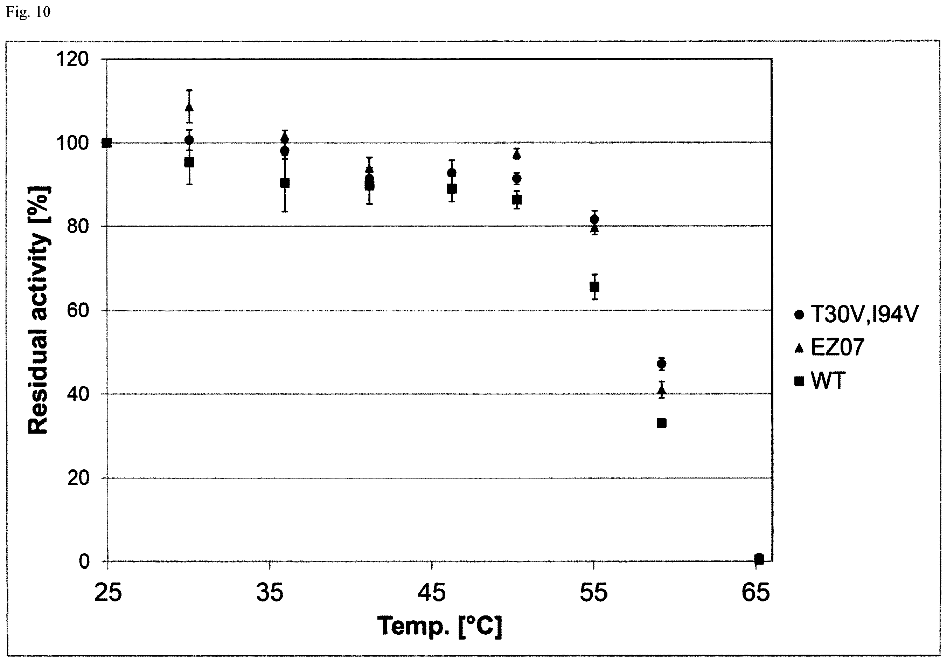

   Fig. 10 shows residual activity of GOx-WT, GOx-T30V; I94V and the GOx variant EZ07 after 15 min incubation at various temperatures between 25 and 67°C.

### Sequence Description

### Polypeptide Sequences

### Nucleotide Sequences

In the following example section, all reagents, restriction enzymes, and other materials were obtained from Roche Diagnostics Germany, unless other commercial sources are specified, and used according to the instructions given by the suppliers. Operations and methods employed for the purification, characterization and cloning of DNA are well known in the art *[*Ausubel, F., et al., in "Current protocols in molecular biology" (1994), Wiley*]* and can be adapted as required by the skilled artisan.

### Examples

The present invention will be illustrated in detail by way of the Examples below, although the present invention shall be not limited to those specific Examples.

The core of the present invention is the unexpected and surprising finding of specific GOx variants that exhibit improved properties over known GOxs in the field of blood glucose measurements, i.e. maintaining its specificity for the substrate glucose and significantly reducing the oxygen-consumption rates via a shift from oxidase activity towards dehydrogenase activity and/or having an increased activity for specific electron mediators other than oxygen.

To prove the GOx properties of the GOx variants in accordance with the invention, the inventors could no rely on the most of available activity assays that indicate the reduction of molecular oxygen to hydrogen peroxide. Specifically, for mediated electron transfer Zhu *et al.* (2006; 2007) established a product based GOx detection assay (hereinafter GODA) *[*Zhu, Z., Momeu, C., Zakhartsev, M., and Schwaneberg, U. (2006). Making glucose oxidase fit for biofuel cell applications by directed protein evolution. Biosensors and Bioelectronics 21, 2046-2051*;* Zhu, Z., Wang, M., Gautam, A., Nazor, J., Momeu, C., R, P., and U, S. (2007). Directed evolution of glucose oxidase from Aspergillus niger for ferrocenemethanol-mediated electron transfer. Biotechnology Journal 2, 241-248*].* That assays, indeed allow for the oxygen independent detection of gluconolactone, but still in the presence of oxygen it is not possible to detect specifically the mediated electron transfer by mediators other than oxygen, which is highly relevant in case of real-time blood glucose analytic devices.

In the following examples, the inventors tested therefore specific GOx variants of the invention, separately for its mediated electron transfer, the electron transfer by oxygen and its glucose specificity to prove the core of the invention. Further, the properties in terms of thermostability were also tested.

In consequence, the inventors used the well-known ABTS assay *[*Sun, L., Bulter, T., Alcalde, M., Petrounia, I.P., and Arnold, F.H. (2002). Modification of galactose oxidase to introduce glucose 6-oxidase activity. Chembiochem 3, 781-783*]* to detect the activity of the GOx variants on oxygen. Figure 1 shows the principle of the activity determination on oxygen.

### Testing for GOx variants

To test the appropriate amino acid substitutions for its improvement on the GOx properties in terms of glucose specificity, reduced oxygen-consumption rates and/or increased activity for specific mediators other than oxygen, specific GOx variants of the invention were tested
(i) for its oxygen-consumption rates, i.e. the enzymatic activity using oxygen as an electron acceptor in a colorimetric ABTS assay and
(ii) for mediated electron transfer by the Nitrosoaniline Mediator as exemplary mediator
(iii) for mediated electron transfer by Nitrosoaniline Mediator as exemplary mediator in the presence of different sugars as substrate, i.e. glucose, galactose, maltose, xylose, maltotriose.

For said mediated electron transfer a p- nitrosonaniline compound was used *[*Becker, O. (2005). Die Glucose-Dye-Oxidoreduktase in der klinischen Diagnostik. DISSERTATION thesis, Technische Universität Kaiserslautern, Kaiserslautern*].* Figure 2 shows the principle of the mediator assay, i.e. a PMO (Phosphomolybdic acid)-assay, in which the Nitrosoaniline Mediator N,N-bis(2-hydroxyethyl)-4-nitrosoaniline is applied.

In the above assay, two electrons, which come out of the mediator reaction, are transferred to the PMO. The PMO gets subsequently reduced. The color change from pale yellow, of the oxidised PMO, to dark blue, of the reduced PMO, was monitored by absorption at 700 nm.

To prove the specific GOx variants in accordance with the invention the above-described mediator assay was adapted to low GOx activities in yeast cell supernatants that show a broad linear detection range from 0.65 U/L to 22 U/L in potassium phosphate buffer (0.2 M, pH 7.0). A standard deviation over a 96-well plate of 10.2% was reached under said conditions. For the calculation of the standard deviation the background was considered by subtracting the values of the negative control (true standard deviation).

### Glucose oxidase variants

In accordance with the invention the double-mutant as described in Zhu *et al.* (2006; 2007) was chosen as the starting material for the herein provided specific amino acid substitutions. This double-mutant has the two substitutions T30V and I94V inherently present (SEQ ID NO:1). The GOx-T30V; I94V shows an improved thermal stability, an improved pH-stability as well as an increased kcat value (69.5/s to 137.7/s) *[*Zhu, Z., Wang, M., Gautam, A., Nazor, J., Momeu, C., R, P., and U, S. (2007). Directed evolution of glucose oxidase from Aspergillus niger for ferrocenemethanol-mediated electron transfer. Biotechnology Jounal 2, 241-248*].*

A hypoglycosylating *Saccharomyces cerevisiae* strain was chosen as an expression host *[*Lehle, L., Eiden, A., Lehnert, K., Haselbeck, A., and Kopetzki, E. (1995). Glycoprotein biosynthesis in Saccharomyces cerevisiae: ngd29, an N-glycosylation mutant allelic to ochl having a defect in the initiation of outer chain formation. FEBS Lett 370, 41-45*].*

After cultivation of the cell clones that bear specific amino acid substitutions in accordance with the invention, the mediator assay and the ABTS-assay was applied in parallel. Clones, which showed increased mediator activity, reduced oxygen activity or both were selected for further investigation. Therefore, the selected clones and the controls were cultivated and screened 12 times. Subsequently, the GOx-genes of improved variants in accordance with the invention were isolated and sequenced. Afterwards, said genes of selected variants served as templates for further specific amino acid substitutions.

### Characterization of the amino acid positions

The inventors further characterized the specific amino acid positions of the herein provided GOx variants, i.e. S53; A137; A173; A332; F414; and V560. These positions are depicted in Figure 3.

### Pre-characterization

To further investigate specific GOx variants the inventors conducted a pre-characterization, considering the following properties:
1) oxygen consumption
2) specificity for glucose as substrate
3) Michaelis-Menten kinetics (Mediator activity/glucose affinity) and
4) thermo stability.

The cultivation of the corresponding cell clones took place in conventional shaking flasks. The supernatants were concentrated and buffered in 0.2 M potassium phosphate, pH 7.

### 1) Oxygen consumption rate

The oxygen consumption was determined indirectly by the oxidation of the chromogenic substrate ABTS as outlined under item gg) in the below Materials and Methods section. To prove this data the oxygen consumption was measured directly using an optical oxygen probe. The GOx-variants were normalized to 2 U/L in the reaction approach.

### 2) Specificity

As already outlined above, the GOx specificity for the substrate glucose plays a major role in the enzymatic glucose determination since there are clinical relevant sugars next to glucose, such as the four sugars galactose, maltose, xylose and maltoriose. Therefore, to prove specificity of the herein provided GOx variants said sugars were chosen and compared to glucose. For these studies the mediator assay was used, 181.8 mM of each sugar was applied.

### 3) Mediator activity / glucose affinity

For a more detailed study on the mediator activity and for a determination of glucose affinity, the inventors applied testings of the herein disclosed variants for Michaelis-Menten kinetics.

### 4) Thermostability

The thermostability of the selected GOx variants of the invention was analyzed applying the mediator assay as outlined in the Materials and Methods section under item ff). As additional controls a hyper-glycosylated GOx, expressed in *Aspergillus niger* and the de-glycosylated GOx-WT were also analyzed.

### Final characterization of the GOx variant GOx-EZ07

For final characterization, the respective GOx-EZ07 expressing *S. cerevsiae* strain was cultivated in a 10 L fermenter, the obtained GOx-EZ07 was subsequently purified. The purity of the final enzyme preparation was higher than 90% in 50 mM potassium phosphate buffer pH 7. Like in the pre-characterization phase the oxygen consumption, the specificity, the activity and the glucose affinity were studied. For the specific determination of GOx concentration an ELISA was applied (see Material and Methods section under item ee).
The variant GOx-EZ07 was also characterized in terms of
1a) oxygen consumption
2a) specificity for glucose as substrate
3a) Michaelis-Menten kinetics (mediator activity/glucose affinity) and
4a) thermostability.

### 1a) Oxygen consumption of GOx-EZ07

The measurement was implemented as described in 1). All tested enzymes (GOx-WT; GOx-T30V; I94V, and the GOx-EZ07) were purified a standard protocol as outlined in the Materials and Methods section under item dd) and adjusted to a protein concentration of 5 µg/mL.

The following oxygen consumption rates were detected [%/min]: GOx-WT: 17.44; GOx-T30V; I94V: 24.22 and the GOx-EZ07 of the invention: 3.86. The residual oxygen activity of the GOx-EZ07 is 15.9% compared with GOx-T30V;I94V and 22.13 % compared with GOx-WT.

### 2a) Specificity of GOx-EZ07

The measurement was implemented as described in 2).

### 3a) Michaelis-Menten kinetics of GOx-EZ07

The measurement was implemented as described in 3).

### 4a) Thermostability

The measurement was implemented as described in 4).

### Results

The inventors found six distinct amino acid positions S53; A137; A173; A332; F414; and V560, which are responsible for responsible for reduced oxygen-consumption rates and/or increased mediator activity for mediators other than oxygen. Further, the inventors found cooperative effects among the six amino acid positions that enable the person skilled in the art to design GOx variants in terms of glucose specificity, significantly reduced oxygen-consumption rates, and/or increased activity for mediators other than oxygen.

In particular, the inventors found the positions V560 and F414, either alone or in combination, to be responsible for significantly reducing the oxygen-consumption rates and/or being responsible for significantly increasing the mediator activity. In addition, the positions A173 and A332 were found to be responsible for both in combination, i.e. a significant increase in mediator activity and concomitantly a significant reduction in oxygen-consumption rates of the herein provided GOx variants. The positions S53 and A137 were found to be responsible for significantly increasing the mediator activity for specific mediators other than oxygen. Further, the inventors found specific amino acids to be suitable for the GOx variants provided by the instant invention.

**Table 1 shows exemplarily a suitable set of amino acids for the positions 173, 332, 414, and 560 and the corresponding degenerated codons.**

| Residue | Reduced set of amino acids | Degenerated codons |
|---|---|---|
| 173 | AITV | RYT |
| 332 | SN | ART |
| 414 | RNDCGHILFSTV | NDT |
| 560 | AILMPTV | VYK |

The specific activities of the tested GOx variants according to the invention are shown in Table 2 by a respective EZ numbering. The table is a comparison of the tested GOx variants according to the invention in respect to mediator activity via the mediator assay and oxygen-consumption properties via the ABTS assay.

The results are shown in the form of a ratio (quotient) between the GOx variants according to the invention and the parent mutant, i.e. the GOx-T30V; I94V as starting material. The results of the different microtiter-plates were comparable. The ratio is based on the specific activities [U/mg], determined by conventional ELISA technique as outlined in the Materials and Methods section under item ee). The table also shows the exact substitutions of the tested amino acid positions in accordance with the present invention.

**Table 2: Specific GOx variants of the invention with an EZ number code, tested for mediator activity and for (residual) oxygen activity.**

| **GOx-variant** | **A 173** | **A 332** | **A 137** | **F 414** | **V 560** | **S 53** | **Mediator activity GOx-variant / GOx-T30V; I94V** | **Oxygen activity GOx-variant / GOx-T30V; I94V** |
|---|---|---|---|---|---|---|---|---|
| **Gox-T30V; I94V (Reference)** | - | - | - | - | - | - | **1.0** | **1.0** |
| **EZ03** | **T** | - | - | **L** | - | - | 1.5 | 0.1 |
| **EZ05** | - | **S** | - | **-** | **A** | **-** | 1.1 | 0.1 |
| **EZ12** | - | **S** | - | **-** | **-** | **F** | 1.7 | 1.5 |
| **EZ06** | **I** | **S** | - | **L** | **-** | **-** | 1.6 | 0.3 |
| **EZ07** | **V** | **S** | - | **I** | **T** | **-** | 4.4 | 0.1 |
| **EZ08** | **V** | **N** | **-** | **-** | **P** | - | 1.0 | 0.1 |
| **EZ10** | **V** | **N** | **-** | **V** | **L** | - | 2.2 | 0.1 |
| **EZ11** | - | **S** | - | - | **P** | - | 1.2 | 0.1 |
| **EZ15** | - | - | **L** | - | - | - | 6.0 | 4.0 |

The results show that the different specific variants (EZ03, EZ05, EZ12, EZ06, EZ07, EZ08, EZ10, EZ11, EZ15) having the essential properties according to the invention, i.e. the reduced oxygen affinity leading to significantly reduced oxygen consumption rates and/or the increased mediator activity in comparison to the parent mutant GOx-T30V; I94V. The variants EZ03, EZ05, EZ07, EZ08, and EZ10 were undergoing further studies.
For instance the inventors found the GOx-EZ07 and EZ10 show both significant improvements for increased mediator activity as well as a significant decrease in the oxygen assay in comparison to the parent mutant.

Particularly the inventors found the variant EZ07 as being a significantly improved GOx variant in accordance with the invention without any limitation to the corresponding specific amino acid substitutions of said variant. EZ07 shows a 4.4 times increase in the mediator assay in comparison to the parent mutant and only a 0.1 times oxygen activity of the oxygen activity of the parent mutant in the ABTS-assay. By contrast, the GOx variants EZ12 and EZ15 show concomitant increases for both, mediator- and oxygen activity in comparison to the parent mutant. EZ12 carries an additional substitution on position S53 compared with the parent mutant (see Table 2). Further, EZ15 were found to have a 6-times increase in mediator activity due to the amino acid substitution A137L.

### Characterization of the amino acid positions

The positions A173 and A332 are far away from the active site. A173 is a surface position; A332 is located close to the substrate entrance channel. The two positions F414 and V560, which have an influence to the oxygen activity, are located close to the active site. F414 is above the glucose-binding site and V560 is sitting next to glucose and FAD. Both of the two activity positions A137 and S53 are surface positions near the FAD.

### Pre-characterization

The results for the selected pre-characterized variants are as follows:

### 1) Oxygen consumption rate

Figure 4 shows the progression of the oxygen content in the reaction mixture as a function of time (a) and the oxygen consumption rate per minute (b). As controls the GOx-T30V; I94V, GOx-WT and the supernatant of the negative control strain were also analyzed.

The GOx-WT and GOx-T30V; I94V show a similar behaviour. Thus, all the here tested GOx variants (EZ03, EZ05, EZ07, EZ08, EZ10) have a significant reduced oxygen consumption rate. Moreover, as a surprising and unexpected finding of the present invention, the tested variants cannot be clearly distinguished from the negative control, what indicates that the respective oxygen activities are even below the detection range of the assay under the selected conditions.

### 2) Specificity

Table 3 shows the residual GOx enzyme activities referring to glucose.

**Table 3: Residual activity of GOx-variants of the invention for different sugars. For determination of residual activities the mediator assay for characterization was applied. The substrate concentration was 181.8 mM in the respective reaction mixtures. The GOx-WT and the GOx-T30V; I94V activities served as references.**

| Residual activity [%] | Glucose | Galactose | Maltose | Xylose | Maltotriose |
|---|---|---|---|---|---|
| GOx-WT | 100 | 1.4 | 0 | 2.6 | 0 |
| GOx-T30V; I94V | 100 | 1.6 | 0 | 2.6 | 0 |
| EZ03 | 100 | 2.9 | 0 | 5.3 | 0 |
| EZ05 | 100 | 3.3 | 0.3 | 3.8 | 0 |
| EZ10 | 100 | 1.1 | 0 | 3.1 | 0 |
| EZ08 | 100 | 4.0 | 0.2 | 4.7 | 0 |
| EZ07 | 100 | 0 | 0 | 3.6 | 0 |

In comparison to the GOx-WT and the parent mutant GOx-T30V; I94V according to SEQ ID NO: 1, none of the GOx variants of the invention showed significant interferences with maltose or maltotriose. EZ07 and EZ10 have the best results for all tested sugars.

### 3) Mediator activity / glucose affinity

The Michaelis-Menten kinetics are represented in Figure 5. The table 4 shows the calculated values for Vₘₐₓ and K_{M}.

**Table 4: Enzyme parameters for different GOx-variants. The parameters were calculated according to Michaelis-Menten applying the least square method in Microsoft Excel.**

| GOx variant | Vₘₐₓ | K_{M} |
|---|---|---|
| GOx-WT | 0.53 | 25.90 |
| GOx-T30V; I94V | 0.83 | 21.84 |
| EZ03 | 2.00 | 46.62 |
| EZ05 | 0.57 | 24.83 |
| EZ07 | 6.35 | 40.26 |
| EZ08 | 1.37 | 49.69 |
| EZ10 | 3.75 | 40.14 |

Table 4 shows that EZ07 has at one hand an approximately 7.5 times higher Vₘₐₓ value in comparison to the parent mutant GOx-T30I; I94V, but on the other hand an almost two times higher K_{M}-value. However, the high K_{M} value is a result of the high Vₘₐₓ value. In Figure 5 it can be clearly seen that the performance of GOx-EZ07 at low substrate concentrations is comparable to the WT and the parent mutant T30V, I94V. The GOx-EZ10 reached similar results but a lower Vₘₐₓ value.

### 4) Thermostability

In Figure 6 the residual activity is diagrammed for eight different temperatures.

The tested GOx variants pursuant to the invention, the GOx-WT and the GOx-T30V; I94V parent mutant show similar results. A significant drop down in the thermostability can be observed for the de-glycosylated GOx-WT; however, the hyper-glycosylated molecule shows a higher thermostability.

### Conclusion

In conclusion of the specific testings 1) to 4) during all pre-characterization studies the GOx-EZ07 and the GOx-EZ10 variant showed the most significant results. A significant drop down in the oxygen activity as well as significant increase in the mediator activity was achieved according to the pre-characterization results. The specificity of GOx-EZ07 and GOx-EZ10 are almost unchanged only a slight activity change on xylose was detected. Since the activity of the EZ07 is approximately 1.7 times higher than the activity of EZ10, the GOx-EZ07 was chosen for the final characterization.

It should be emphasized that the above results were generated using unpurified GOx variants of the invention. Accordingly, the person skilled in the art is aware that the results may vary after using similar purified GOx variants. Also the specific protein determination in cell supernatants applying ELISA may be affected by impurities.

### Final characterization of the variant GOx-EZ07

For final characterization the purified GOx-EZ07 variant was chosen.

### 1a) Oxygen consumption of GOx-EZ07

The relative oxygen consumption rate of the GOx-WT, GOx-T30V;I94V and the GOx-EZ07 are depicted in Figure 7.

Under the selected conditions the oxygen consumption rate of GOx-EZ07 is more than 10-fold reduced compared to the GOx-WT what confirms the data of the pre-characterization study.

### 2a) Specificity of GOx EZ07

Figure 8 shows the residual activity of the GOx-WT, GOx-T30V; I94V and the GOx-EZ07 referring to glucose (100%). Figure 8 shows that there is no significant change in the specificity of GOx-EZ07 compared to GOx-T30V; I94V and GOx-WT.

### 3a) Michaelis-Menten kinetics of GOx variant EZ07

Figure 9 compares the kinetics of GOx-WT, GOx-T30V; I94V and the GOx-EZ07 in the mediator assay.

According to Figure 9 the GOx variant EZ07 shows 641.5 % residual activity in the mediator assay and 17.5 % residual activity in the ABTS assay in comparison to the GOx-WT resulting in a 37 times reduced oxidase activity.

### 4a) Thermostability of the GOx-EZ07

Figure 10 indicates that the thermal stability of the GOx-EZ07 was maintained by the respective substitutions, when compared to the GOx-WT and the GOx-T30V; I94V.

### Summary for the GOx variants

Six amino acid positions were identified in the parent mutant GOx-T30V, I94V, which show effects on glucose specificity of the herein provided GOx variants, on enzyme activity for mediated electron transfer and on oxygen activity. All positions were saturated individually. To study cooperative effects positions clustered around the active site (S53; A137; A173; A332; F414; V560) were saturated simultaneously. The most significant variants for improved mediator activity and/or reduced oxygen consumption rates in comparison to the parent mutant properties were chosen for the pre-characterization. It turned out that the variant GOx-EZ07 (having the additional substitutions A173V; A332S; F414I; and V560T) shows the most significant results for the tested properties glucose specificity, mediator activity and oxygen consumption rates in the sense of the present invention.

Thus, the GOx- EZ07 was intensively studied for its mediator activity, oxidase activity, glucose specificity as well as its thermal stability properties. The variant shows a 6.4-fold increase in specific activity for mediated electron transfer (GOx-WT: 7.4; GOx-EZ07: 47.5 U/mg) and a 5.7-fold reduced activity in the ABTS assay (GOx-WT: 451.1 U/mg; GOx-EZ07: 78.86 U/mg) resulting in a 36.5 times reduced oxidase activity. GOx-EZ07 has no significant changes in thermal stability or specificity.

### Materials and Methods

All chemicals were of analytical grade and purchased from Sigma-Aldrich (Taufkirchen, Germany), Applichem (Darmstadt, Germany) or Carl Roth (Karlsruhe, Germany). All enzymes were purchased from New England Biolabs (UK) and Sigma-Aldrich (Taufkirchen, Germany). The pYES2 shuttle vector and *Escherichia coli strain DH5á* were purchased from Invitrogen (Karlsruhe, Germany). The *Saccharomyces cerevisiae* strain 7087 (ngd29mnn1) was provided by Roche diagnostics *[*Lehle, L., Eiden, A., Lehnert, K., Haselbeck, A., and Kopetzki, E. (1995). Glycoprotein biosynthesis in Saccharomyces cerevisiae: ngd29, an N-glycosylation mutant allelic to ochl having a defect in the initiation of outer chain formation. FEBS Lett 370, 41-45*].* DNA was quantified using a NanoDrop photometer (NanoDrop Technologies, Wilmington, DE, USA). Sequencing and oligonucleotide synthesis was done by Eurofins MWG Operon (Ebersberg, Germany). Primers used are mentioned in Table 5 (Supporting Information).

### aa) Multiple- and single site saturation mutagenesis

For multiple site saturation mutagenesis the Omnichange protocol was applied *[*Dennig, A., Shivange, A.V., Marienhagen, J., and Schwaneberg, U. (2011). OmniChange: The Sequence Independent Method for Simultaneous Site-Saturation of Five Codons. PLoS ONE 6, e26222*].* 400 µl PCR reaction mixture contained 1 ng/µl of plasmid template, 1x Phusion buffer (New England Biolabs, Frankfurt, Germany), 1 U of Phusion polymerase and 200 µM dNTP's. The reaction mixture was divided in four equal volumes and 250 nM of forward primer and reverse primers, targeting specific sites, were added for fragment amplifications. In the mixture for the vector backbone amplification, additional dNTP's were added to reach final concentration of 300 µM. For Fragment 1, targeting A173, pimers P7 and P8 were added in the master mix. While for A332 saturation, P9 and P10 were used. F414 site was targeted by using P11 and P12. The V560 site was included in vector backbone as it was amplified by P13 and P14. The PCR program for amplification of fragments was 98°C for 45 sec (1 cycle); 98°C for 15 sec; 65°C for 30 sec; 72 °C for 30 sec (20 cycles); 72 °C for 5 min (1 cycle). For vector backbone amplification extension time of 4 min was applied. All fragments were column purified and the concentration was measured on NanoDrop photometer (NanoDrop Technologies, Wilmington, DE, USA). The PCR product was then subjected to overnight *Dpn*I digestion (5 U each).

Digestion and ligation of fragments: The concentration of insert fragments was adjusted to 0.06 pmol/ul. While vector backbone concentration was adjusted to 0.02 pmol/ul (dilution factor with *Dpn*I digestion was also taken into consideration). 1 µl of cleavage solution (50 % (500 mM Tris pH 9.0, 30% (100mM Iodine in ethanol) and 20% dH2O) was added to 4 µl of each fragment. The reaction was then incubated at 70°C for 5 min. The cleaved fragments were mixed together and incubated at room temperature (RT) for 5 min before transformation of chemical competent *E. coli* DH5á for library amplification. For yeast transformation the gene library was isolated from *E. coli* DH5á using the plasmid isolation kit "NucleoSpin® Plasmid" (MACHERY-NAGEL, Düren, Germany).

For individual site saturation mutagenesis (SSM) a two-step PCR protocol has been followed *[*Sambrook, J., and Russel, D. (2001). Molecular cloning: a laboratory manual, Volume 2 (Cold Spring Harbor Laboratory Press*)].* 50 µl PCR reaction mixture contained 1 ng/µl of plasmid template, 1x Phusion buffer (New England Biolabs, Frankfurt, Germany), 1 U of Phusion polymerase and 300 µM dNTP's. The reaction mixture was divided in two equal volumes and 400 nM of forward primer and reverse primer of the targeted site were added to separate reaction mixtures. PCR program was 98°C for 45 sec (1 cycle); 98°C for 15 sec; 65°C for 30 sec; 72 °C for 4 min (5 cycles); 72 °C for 5 min (1 cycle). After first step, both reactions were pooled and the same program was carried out with 15 more repetitions for the amplifying cycles. The following primers were used: P15/P16 for position 173, P17/P18 for position 332, P19/P20 for position 414 and P21/22 for position 560. The PCR product was column purified and subsequently subjected for *Dpn*I digestion.

### bb) Gene cloning and Yeast cell transformation

The cloning of the mutated GOx-genes into the pYES2 vector backbone was performed according to a recombination mediated ligase free method *[*Oldenburg, K.R., Vo, K.T., Michaelis, S., and Paddon, C. (1997). Recombination-mediated PCR-directed plasmid construction in vivo in yeast. Nucleic Acids Research 25, 451-452*].* Firstly, 2 µg of pYES2-GOx T30V, I94V double-mutant was linearized by SalI/BamHI digestion (10 U/µg DNA, 6 h, 37°C) and subsequently purified by gel-extraction employing the NucleoSpin® Extract II - kit (MACHERY-NAGEL, Düren, Germany). The primers, used for library amplification, were designed in a way that the whole primer sequence is complementary to the linearized vector backbone.

Secondly, the linearized pYES2-vector and the amplified gene library were mixed in a ratio of 1:3 (250 ng/750 ng). This DNA-mix was used for the transformation of *Saccharomyces cerevisiae* 7087 employing a lithium acetate method *[*Gietz, R.D., and Schiestl, R.H. (2007). High-efficiency yeast transformation using the LiAc/SS carrier DNA/PEG method. Nature Protocols 2, 31-34*].* Transformants were grown on SC-U selective plates containing 2% glucose. For the transformation of circular plasmids 300 ng DNA was used.

### cc) Cultivation and expression in 96-well plates

Single colonies grown on SC-U selective agar plates containing 2% glucose were transferred into 96-well microtiter plates (PS-F-bottom, greiner bio-one) containing 100 µL SC-U media (1% glucose) per well using toothpicks. The cultivation of the pre-culture took place in a plate shaker (Infors GmbH, Eisenach, Germany) under the following conditions: 900 rpm, 30°C, 70% humidity, 24 h. A certain amount of pre-culture was transferred from each well to 500 µL SC-U auto-induction media (0.5% glucose / 2% galactose) in a deep well plate (riplate-rectangular, ritter). The main-culture was cultivated under the same conditions than the pre-culture, but for 72 h. Plates were centrifuged at 4000 rpm and RT for 20 min. The resultant supernatants were used for activity determinations.

### dd) Production and purification of recombinant GOx

Pre-cultivation: 500 mL SynY media (2% glucose) were inoculated to OD 600 = 0.2 using an overnight-culture of Sc7087/pYES2-GOx, the cultivations took place in 5L shaking flasks for 12 h at 30°C and 250 rpm. Main-cultivation: 10 L SynY media (1% glucose) was inoculated with 500 mL pre-culture. For the cultivation a 10 L fermenter was used applying the following conditions: Temperature: 30°C; stirrer: 400 rpm; air flow: 1 vvm; time: 48h.

Cell separation and buffer exchange: The GOx containing supernatant was recovered by micro-filtration. For this purpose the cross-flow filtration unit SartoJet (Sartorius Stedim Biotech GmbH, Göttingen, Germany) was used with one filter cassette (SARTOCON Slice, Hydrosart 0.45 µm, Sartorius Stedim Biotech GmbH, Göttingen, Germany). The feed pressure was adjusted to 2.0 bar and the retentate pressure to 1.0 bar. The same filtration system was used for buffer exchange and sample concentration applying a ultra-filtration cassette (SARTOCON Slice, Hydrosart 10 kDa, Sartorius Stedim Biotech GmbH, Göttingen, Germany) under the following conditions: Buffer 50 mM NaH₂PO₄, pH 6; Buffer exchange volumes: 7; feed pressure: 2.0 bar; retentate pressure: 1.0 bar; end volume 0.5 L.

For enzyme purification anion-exchange chromatography was applied, using the ÄKTA pilot system and a FinLine Pilot 35 column (GE Healthcare, Munich, Germany). The column was packed with 220 mL resin (Fractogel TSK DEAE-650s Merck), a flow rate of 62.5 cm/h was selected. Equilibration: 440 mL 50 mM sodium phosphate buffer pH6; sample load: 0.5 L retentate; wash: 440 mL50 mM sodium phosphate buffer pH6; elution: 95% 50 mM sodium phosphate buffer pH6 / 5% 1M NaCl (step gradient). The GOx containing fractions were pooled, subsequently a final buffer exchange against 50 mM potassium phosphate buffer pH 7 took place (Amicon® Ultracel-30k Millipore, Cork, Ireland).

### ee) Normalization of protein concentrations

An enzyme-linked immunosorbent assay (ELISA) was used for the specific determination of GOx concentrations. Each well of a streptavidin coated microtiter plate (Roche - Material No. 11643673001) was filled with 100 µL of the primary antibody solution (2 µg/mL; Rabbit PAK GOD-Biotin Acris R1083B), the plate was incubated for 1 h at room temperature. After a subsequent washing step (4 times 350 µL 9 g/L NaCl_0.1 % Tween 20) 100 mL of Enzyme sample was pipetted into the wells, followed by a second incubation step. The washing step was repeated and the plate was filled with 100 µL of the secondary antibody solution (10 µg/mL; Rabbit PAK GOD-HRP Acris R1083HRP). After a third incubation and washing step the plate was filled with 100 µL of a ABTS/H₂O₂ solution (11684302001 Roche Diagnostics GmbH, Mannheim, Germany). The plate was incubated for 30 min at room temperature before the absorption at 405 nm was determined. Glucose oxidase from *Aspergillus niger* was used as internal standard (G7141-50KU SIGMA-ALDRICH).

### ff) Mediator assay

For the liquid handling multi-channel pipettes from Brand (Transferpette S-8) and Eppendorf (Research pro) were used. 75 µL of sample (prepared supernatants / enzyme solution) were transferred to a 96-well flat-bottom microplate (greiner bio-one). 100 µL of mediator solution (19.05 mM N,N-bis(2-hydroxyethyl)-4-nitrosoaniline *[*Becker, O. (2005). Die Glucose-Dye-Oxidoreduktase in der klinischen Diagnostik. DISSERTATION thesis, Technische Universität Kaiserslautern, Kaiserslautern*],* Roche - Material No. 100088314; 5% (w/w) Polyvinylpyrrolidone, Roche - 10003476964; pH 7) and 20 µL of 25 mM Phosphomolybdic acid (Roche, Genisys-nr.: 11889893001) were added. The reactions were started by adding 25 µL substrate solution and subsequently shaking of the plate at 1000 rpm for 1 min. The kinetic of Phosphomolybdic acid reduction was monitored at 700 nm using the microplate-reader Tecan Sunrise (Tecan Trading AG, Switzerland). For kinetic analysis Vmax and KM values were determined from initial velocity data plotted as a function of substrate concentrations with a linear correlation coefficient of >0.99.

### gg) ABTS assay

For the liquid handling multi-channel pipettes from Brand (Transferpette S-8) and Eppendorf (Research pro) were used. 75 µL of sample (prepared supernatants / enzyme solution) were transferred to a 96-well flat-bottom microplate (greiner bio-one) containing 100 µL of phosphate buffer (pH 7). 20 µL of reaction mixture were added to each well resulting in the following concentrations: 0.91 U/mL HRP; 2.3 mM ABTS. The reactions were started by adding 25 µL substrate solution and subsequently shaking the plate at 1000 rpm for 30 sec. The oxidation of ABTS was kinetically determined at 414 nm using the microplate reader FLUOstar Omega (BMG LABTECH). For kinetic analysis Vₘₐₓ and K_{M} values were determined from initial velocity data plotted as a function of substrate concentrations with a linear correlation coefficient of > 0.99.

### hh) Oxygen consumption assay

For the direct determination of the oxygen consumption the optical oxygen probe "Fibox3 - Minisensor Oxygen Meter" (Precision Sensing GmbH, Regensburg, Germany) was used. The 1540 µL reaction mixture consisted of 840 µL phosphate buffer (0.2 M / pH 7), 175 µL substrate solution and 525 µL GOx-solution. The oxygen consumption (%/min) was kinetically determined.

### ii) Thermal stability

100 µL of the enzyme solution were incubated at the corresponding temperature for 15 min and subsequently chilled on ice for 5 min. 75 µL were used for the activity determinations. The following temperatures were studied: 30-, 35-, 40-, 45-, 50-, 55-, 60-, and 65°C.

**Table 5: Primer used in experimental section**

| **Primer** | **Sequence** |
|---|---|
| **P1** | GGC GTG AAT GTA AGC GTG ACA TA |
| **P2** | CACACTACCGCACTCCGTCG CCG GAT CGG ACT ACT AGC AG |
| **P3** | CCG GAT CGG ACT ACT AGC AG |
| **P4** | GTGTGATGGCGTGAGGCAGC GGC GTG AAT GTA AGC GTG ACA TA |
| **P5** | |
| **P6** | |
| **P7** | ggtactgtccatRYTGGACCCCGCGACAC |
| **P8** | ggtggtctggtcCTGCAGGTTCAAG |
| **P9** | gaccagaccaccARTACCGTCCGCTCCC |
| **P10** | gagttccgagtaCGCGACGTTGTGG |
| **P11** | tactcggaactcNDTCTCGACACTGCC |
| **P12** | atgggacgacatTTGCGTAGGAGG |
| **P13** | atgtcgtcccatVYKATGACGGTGTTCTA |
| **P14** | atggacagtaccATTAACACCATG |
| **P15** | GGT ACT GTC CAT NNK GGA CCC CGC GAC AC |
| **P16** | GTGTCGCGG GGT CCMNNA TGG ACAGTA CC |
| **P17** | GAC CAG ACC ACC NNK ACC GTC CGC TCC C |
| **P18** | GGG AGC GGA CGG TMN NGG TGG TCT GGT C |
| **P19** | TAC TCG GAA CTC NNK CTC GAC ACT GCC |
| **P20** | GGC AGT GTC GAG MNN GAG TTC CGA GTA |
| **P21** | ATG TCG TCC CAT NNK ATG ACG GTG TTC TA |
| **P22** | TAG AAC ACC GTC ATM NNA TGG GAC GAC AT |

## Claims

1. A glucose oxidase according to SEQ ID NO: 1, selected from the group consisting of
a) a glucose oxidase according to SEQ ID NO: 1, having besides the two amino acid substitutions T30V and I94V, at least one additional amino acid substitution in any of the five positions selected from the group S53; A173; A332; F414; V560 in said SEQ ID NO: 1; or
b) a glucose oxidase that exhibits at least 70%, particularly at least 80%, or > 90% amino acid sequence identity to the glucose oxidase according to a) provided that the glucose oxidase of b) is having besides the two amino acid substitutions T30V and I94V, at least one additional amino acid substitution in any of the five positions selected from the group S53; A173; A332; F414; V560 according to SEQ ID NO: 1,
and provided that the glucose oxidase according to b) exhibits at least 70%, particularly at least 80%, or > 90% of the enzyme activity of the glucose oxidase according to a), and exhibits at least 70%, particularly at least 80%, or > 90% of the enzyme specificity for glucose of the glucose oxidase according to a),
and provided that the glucose oxidase according to b) exhibits at least a 5-fold reduced activity for oxygen as electron acceptor of the glucose oxidase according to SEQ ID NO: 1 or exhibits at least a 1.5-fold increased activity for electron mediators other than oxygen of the glucose oxidase according to SEQ ID NO: 1, or both; or
c) an active fragment of a glucose oxidase according to a) or b), provided that in the active fragment according to c) the amino acid substitutions as outlined under a) or b) are preserved when compared to the glucose oxidase according to a) or b),
and provided that the glucose oxidase according to c) exhibits at least 70%, particularly at least 80%, or > 90% of the enzyme activity of the glucose oxidase according to a), and exhibits at least 70%, particularly at least 80%, or > 90% of the enzyme specificity for glucose of the glucose oxidase according to a),
and provided that the glucose oxidase according to c) exhibits at least a 5-fold reduced activity for oxygen as electron acceptor of the glucose oxidase according to SEQ ID NO: 1 or exhibits at least a 1.5-fold increased activity for electron mediators other than oxygen of the glucose oxidase according to SEQ ID NO: 1, or both, and wherein the amino acids for additional substitution(s) are selected from the group
Phe for the position S53; and/or
Ile, Thr, Val for the position A173; and/or
Ser, Val, Thr for the position A332; and/or
Ile, Leu, Met, Val for the position F414; and/or
Leu, Pro, Thr, for the position V560.

2. A glucose oxidase according to claim 1, wherein
• said activity for oxygen as electron acceptor is determined by ABTS assay, comprising the steps of
a) 75 µL of sample enzyme solution are transferred to a 96-well flat-bottom microplate containing 100 µL of phosphate buffer (pH 7)
b) 20 µL of reaction mixture is added to each well resulting in the following concentrations: 0.91 U/mL HRP; 2.3 mM ABTS (2,2'-azino-bis(3-ethylbenzothiazoline-6-sulphonic acid))
c) the reaction starts by adding 25 µL glucose substrate solution and subsequent shaking of the plate at 1000 rpm for 30 sec
d) the oxidation of ABTS is kinetically determined at 414 nm using a microplate reader
• said activity for electron mediators other than oxygen is determined by mediator assay, comprising the steps of
a) 75µL sample of enzyme solution is transferred to a 96-well flat-bottom microplate
b) 100 µL of mediator solution (19.05 mM N,N-bis(2-hydroxyethyl)-4-nitrosoaniline); 5% (w/w) Polyvinylpyrrolidone, pH 7 and 20 µL of 25 mM Phosphomolybdic acid are added
c) the reaction starts by adding 25 µL glucose substrate solution and subsequent shaking of the plate at 1000 rpm for 1 min
d) the kinetic of Phosphomolybdic acid reduction is monitored at 700 nm using a microplate-reader.

3. A glucose oxidase according to claim 1 or 2, wherein said electron mediators other than oxygen are selected from the group comprising nitrosoanilines and derivatives thereof, azo-compounds, phenazines and derivatives thereof, phenothiazines and derivatives thereof, phenoxazines and derivatives thereof, ferrocenes and derivatives thereof, potassium ferricyanide, Ru- and Os-complexes, quinones and derivatives thereof, indophenols, viologens, tetrathiafulvalene and derivatives thereof, and phthalocyanines.

4. A glucose oxidase according to any of the preceding claims, having besides the two substitutions T30V and I94V according to the SEQ ID NO: 1, additional two, or three, or four, or five amino acid substitutions in any of the five positions selected from the group S53; A173; A332; F414; V560 according to the SEQ ID NO: 1.

5. A glucose oxidase according to any of the preceding claims, wherein besides the substitutions T30V and I94V according to the SEQ ID NO: 1, at least one additional amino acid substitution in the position(s) F414 and/or V560 is/are combined with at least one amino acid substitution in the position(s) A173 and/or A332.

6. A glucose oxidase according to any of the preceding claims, having besides the substitutions T30V and I94V, the additional amino acid substitutions A173I; A332S; and F414L according to SEQ ID NO: 4.

7. A glucose oxidase according to any of the preceding claims, having besides the substitutions T30V and I94V, the additional amino acid substitutions A173V; A332S; F414I; and V560T according to SEQ ID NO: 3.

8. A glucose oxidase according to any of the preceding claims, having besides the substitutions T30V and I94V, the additional amino acid substitutions A173V; A332N; F414V; and V560L according to SEQ ID NO: 6.

9. A glucose oxidase according to any of the preceding claims, exhibiting a glucose specificity of at least 99.9% and/or a galactose specificity < 4% and/or a maltose specificity < 0.3% and/or a xylose specificity < 6% and/or a maltotriose specificity < 0.1%, when determined by mediator assay, comprising the steps of
a) 75µL sample of enzyme solution is transferred to a 96-well flat-bottom microplate
b) 100 µL of mediator solution (19.05 mM N,N-bis(2-hydroxyethyl)-4-nitrosoaniline); 5% (w/w) Polyvinylpyrrolidone, pH 7 and 20 µL of 25 mM Phosphomolybdic acid are added
c) the reaction starts by adding 25 µL of the respective sugar substrate solution and subsequent shaking of the plate at 1000 rpm for 1 min
d) the kinetic of Phosphomolybdic acid reduction is monitored at 700 nm using a microplate-reader.

10. A glucose oxidase according to any of the preceding claims, exhibiting an activity of > 400%, or > 500%, or > 600% for Nitrosoaniline Mediator for electron transfer when compared to Nitrosoanilin Mediator activity of the GOx according to SEQ ID NO: 1 by means of mediator assay, comprising the steps of
a) 75µL sample of enzyme solution is transferred to a 96-well flat-bottom microplate
b) 100 µL of mediator solution (19.05 mM N,N-bis(2-hydroxyethyl)-4-nitrosoaniline); 5% (w/w) Polyvinylpyrrolidone, pH 7 and 20 µL of 25 mM Phosphomolybdic acid are added
c) the reaction starts by adding 25 µL glucose substrate solution and subsequent shaking of the plate at 1000 rpm for 1 min
d) the kinetic of Phosphomolybdic acid reduction is monitored at 700 nm using a microplate-reader.
and an oxygen activity of ≤ 30%, or < 25%, or < 20%, particularly < 15%, or ≤ 10% when compared to the oxygen activity of the GOx according to SEQ ID NO:1 by means of ABTS assay, comprising the steps of
a) 75 µL of sample enzyme solution are transferred to a 96-well flat-bottom microplate containing 100 µL of phosphate buffer (pH 7)
b) 20 µL of reaction mixture is added to each well resulting in the following concentrations: 0.91 U/mL HRP; 2.3 mM ABTS (2,2'-azino-bis(3-ethylbenzothiazo line-6-sulphonic acid))
c) the reaction starts by adding 25 µL glucose substrate solution and subsequent shaking of the plate at 1000 rpm for 30 sec
d) the oxidation of ABTS is kinetically determined at 414 nm using a microplate reader.

11. An isolated polynucleotide encoding a glucose oxidase according to any of the preceding claims or an active fragment thereof.

12. A method of detecting, determining or measuring glucose in an *ex vivo* sample by a glucose oxidase according to the claims 1 to 11 or an active fragment thereof, said detection, determination or measurement comprising contacting an *ex vivo* sample with said glucose oxidase or an active fragment thereof.

13. The method of claim 12, wherein said detection, determination or measurement of glucose is performed using a sensor or a test strip device.

## Patentansprüche

1. Glucoseoxidase gemäß SEQ ID NO: 1, ausgewählt aus der Gruppe bestehend aus:
a) einer Glucoseoxidase gemäß SEQ ID NO: 1, die neben den zwei Aminosäuresubstitutionen T30V und I94V mindestens eine zusätzliche Aminosäuresubstitution in Positionen ausgewählt aus der Gruppe der fünf Positionen S53, A173, A332, F414, V560 in dieser SEQ ID NO: 1 aufweist; oder
b) einer Glucoseoxidase, die mindestens 70 %, insbesondere mindestens 80 %, oder > 90 % Aminosäuresequenzidentität aufweist zu der Glucoseoxidase gemäß a), unter der Voraussetzung, dass die Glucoseoxidase aus b) neben den zwei Aminosäuresubstitutionen T30V und I94V mindestens eine zusätzliche Aminosäuresubstitution in Positionen ausgewählt aus der Gruppe der fünf Positionen S53, A173, A332, F414, V560 nach SEQ ID NO: 1 aufweist;
und unter der Voraussetzung, dass die Glucoseoxidase gemäß b) mindestens 70 %, insbesondere mindestens 80 %, oder > 90 % der Enzymaktivität der Glucoseoxidase gemäß a) aufweist und mindestens 70 %, insbesondere mindestens 80 %, oder > 90 % der Enzymspezifität der Glucoseoxidase gemäß a) für Glucose aufweist,
und unter der Voraussetzung, dass die Glucoseoxidase gemäß b) eine mindestens 5-fach reduzierte Aktivität mit Sauerstoff als Elektronenakzeptor aufweist verglichen mit der Glucoseoxidase gemäß SEQ ID NO: 1 oder eine mindestens 1,5-fach erhöhte Aktivität mit von Sauerstoff verschiedenen Elektronenüberträgern aufweist verglichen mit der Glucoseoxidase gemäß SEQ ID NO: 1 oder beides; oder
c) einem aktiven Fragment einer Glucoseoxidase gemäß a) oder b), unter der Voraussetzung, dass im aktiven Fragment gemäß c) die Aminosäuresubstitutionen wie unter a) oder b) beschrieben erhalten bleiben im Vergleich zu der Glucoseoxidase gemäß a) oder b),
und unter der Voraussetzung, dass die Glucoseoxidase gemäß c) mindestens 70 %, insbesondere mindestens 80 %, oder > 90 % der Enzymaktivität der Glucoseoxidase gemäß a) aufweist und mindestens 70 %, insbesondere mindestens 80 %, oder > 90 % der Enzymspezifität der Glucoseoxidase gemäß a) für Glucose aufweist,
und unter der Voraussetzung, dass die Glucoseoxidase gemäß c) eine mindestens 5-fach reduzierte Aktivität mit Sauerstoff ais Elektronenakzeptor aufweist verglichen mit der Glucoseoxidase gemäß SEQ ID NO: 1 oder eine mindestens 1,5-fach erhöhte Aktivität mit von Sauerstoff verschiedenen Elektronenüberträgern aufweist verglichen mit der Glucoseoxidase gemäß SEQ ID NO: 1 oder beides, und wobei die Aminosäuren für die zusätzliche(n) Substitution(en) ausgewählt sind aus der Gruppe
Phe für die Position S53; und/oder
Ile, Thr, Val für die Position A173; und/oder
Ser, Val, Thr für die Position A332; und/oder
Ile, Leu, Met, Val für die Position F414; und/oder
Leu, Pro, Thr für die Position V560.

2. Glucoseoxidase nach Anspruch 1, wobei
• die Aktivität mit Sauerstoff als Elektronenakzeptor per ABTS-Assay bestimmt wird, umfassend die Schritte
a) 75 µl der Enzymlösungsprobe werden in eine 96-Well-Flachboden-Mikrotiterplatte transferiert, die 100 µl Phosphatpuffer (pH 7) enthält
b) in jedes Well werden 20 µl des Reaktionsgemischs gegeben, was zu folgenden Konzentrationen führt: 0,91 U/ml HRP; 2,3 mM ABTS (2,2'-Azino-bis(3-ethylbenzthioazolin-6-sulfonsäure))
c) die Reaktion startet durch Zugabe von 25 µl Glucosesubstratlösung und nachfolgendes Schütteln der Platte mit 1000 U/min für 30 Sek.
d) die Oxidation von ABTS wird kinetisch bestimmt bei 414 nm mit einem Mikrotiterplatten-Lesegerät
• die Aktivität mit von Sauerstoff verschiedenen Elektronenüberträgern per Mediator-Assay bestimmt wird, umfassend die Schritte
a) 75-µl-Probe der Enzymlösung wird in eine 96-Well-Flachboden-Mikrotiterplatte transferiert
b) 100 µl der Mediatorlösung (19,05 mM N,N-Bis(2-hydroxyethyl)-4-nitrosoanilin), 5 % (w/w) Polyvinylpyrrolidon pH 7 und 20 µl 25 mM Phosphormolybdänsäure werden hinzugefügt
c) die Reaktion startet durch Zugabe von 25 µl Glucosesubstratlösung und nachfolgendes Schütteln der Platte mit 1000 U/min für 1 min
d) die Kinetik der Reduktion der Phosphormolybdänsäure wird bei 700 nm mit einem Mikrotiterplatten-Lesegerät verfolgt.

3. Glucoseoxidase nach Anspruch 1 oder 2, wobei die von Sauerstoff verschiedenen Elektronenüberträger ausgewählt sind aus der Gruppe umfassend Nitrosoaniline und ihre Derivate, Azo-Verbindungen, Phenazine und ihre Derivate, Phenothiazine und ihre Derivate, Phenoxazine und ihre Derivate, Ferrocene und ihre Derivate, Kaliumferricyanid, Ru- und Os-Komplexe, Chinone und ihre Derivate, Indophenole, Viologene, Tetrathiafulvalen und seine Derivate sowie Phthalocyanine.

4. Glucoseoxidase nach einem der vorhergehenden Ansprüche, die neben den zwei Substitutionen T30V und I94V nach SEQ ID NO: 1 zusätzlich zwei oder drei oder vier oder fünf Aminosäuresubstitutionen in Positionen ausgewählt aus der Gruppe der fünf Positionen S53, A173, A332, F414, V560 nach SEQ ID NO: 1 aufweist.

5. Glucoseoxidase nach einem der vorhergehenden Ansprüche, wobei neben den Substitutionen T30V und I94V nach SEQ ID NO: 1 mindestens eine zusätzliche Aminosäuresubstitution in der/den Position(en) F414 und/oder V560 kombiniert ist mit mindestens einer Aminosäuresubstitution in der/den Position(en) A173 und/oder A332.

6. Glucoseoxidase nach einem der vorhergehenden Ansprüche, die neben den Substitutionen T30V und I94V die zusätzlichen Aminosäuresubstitutionen A173I, A332S und F414L nach SEQ ID NO: 4 aufweist.

7. Glucoseoxidase nach einem der vorhergehenden Ansprüche, die neben den Substitutionen T30V und I94V die zusätzlichen Aminosäuresubstitutionen A173V, A332S, F414I und V560T nach SEQ ID NO: 3 aufweist.

8. Glucoseoxidase nach einem der vorhergehenden Ansprüche, die neben den Substitutionen T30V und I94V die zusätzlichen Aminosäuresubstitutionen A173V, A332N, F414V und V560L nach SEQ ID NO: 6 aufweist.

9. Glucoseoxidase nach einem der vorhergehenden Ansprüche, die eine Gfucosespezifität von mindestens 99,9 % und/oder eine Galactosespezifität < 4 % und/oder eine Maltosespezifität < 0,3 % und/oder eine Xylosespezifität < 6 % und/oder eine Maltotriosespezifität < 0,1 % aufweist bei Bestimmung mittels Mediator-Assay, umfassend die Schritte
a) 75-µl-Probe der Enzymlösung wird in eine 96-Well-Flachboden-Mikrotiterplatte transferiert
b) 100 µl der Mediatorlösung (19,05 mM N,N-Bis(2-hydroxyethyl)-4-nitrosoanilin), 5 % (w/w) Polyvinylpyrrolidon pH 7 und 20 µl 25 mM Phosphormolybdänsäure werden hinzugefügt
c) die Reaktion startet durch Zugabe von 25 µl Lösung des jeweiligen Zuckersubstrats und nachfolgendes Schütteln der Platte mit 1000 U/min für 1 min
d) die Kinetik der Reduktion der Phosphormolybdänsäure wird bei 700 nm mit einem Mikrotiterplatten-Lesegerät verfolgt.

10. Glucoseoxidase nach einem der vorhergehenden Ansprüche, die eine Aktivität von > 400 % oder > 500 % oder > 600 % für den Nitrosoanilin-Mediator zum Elektronentransfer im Vergleich zur Nitrosoanilin-Mediator-Aktivität der GOx gemäß SEQ ID NO: 1 mittels Mediator-Assay aufweist, umfassend die Schritte
a) 75-µl-Probe der Enzymlösung wird in eine 96-Well-Flachboden-Mikrotiterplatte transferiert
b) 100 µl der Mediatorlösung (19,05 mM N,N-Bis(2-hydroxyethyl)-4-nitrosoanilin), 5 % (w/w) Polyvinylpyrrolidon pH 7 und 20 µl 25 mM Phosphormolybdänsäure werden hinzugefügt
c) die Reaktion startet durch Zugabe von 25 µl Glucosesubstratlösung und nachfolgendes Schütteln der Platte mit 1000 U/min für 1 min
d) die Kinetik der Reduktion der Phosphormolybdänsäure wird bei 700 nm mit einem Mikrotiterplatten-Lesegerät verfolgt.
und eine Sauerstoffaktivität von ≤ 30 % oder < 25 % oder < 20 %, insbesondere < 15 % oder ≤ 10 % im Vergleich zur Sauerstoffaktivität der GOx gemäß SEQ ID NO: 1 mittels ABTS-Assay, umfassend die Schritte
a) 75 µl der Enzymlösungsprobe werden in eine 96-Well-Flachboden-Mikrotiterplatte transferiert, die 100 µl Phosphatpuffer (pH 7) enthält
b) in jedes Well werden 20 µl des Reaktionsgemischs gegeben, was zu folgenden Konzentrationen führt: 0,91 U/ml HRP; 2,3 mM ABTS (2,2'-Azino-bis(3-ethylbenzthioazolin-6-sutfonsäure))
c) die Reaktion startet durch Zugabe von 25 µl Glucosesubstratlösung und nachfolgendes Schütteln der Platte mit 1000 U/min für 30 Sek.
d) die Oxidation von ABTS wird kinetisch bestimmt bei 414 nm mit einem Mikrotiterplatten-Lesegerät.

11. Isoliertes Polynukleotid, das eine Glucoseoxidase nach einem der vorhergehenden Ansprüche oder ein aktives Fragment davon kodiert.

12. Verfahren zum Nachweis, zur Bestimmung oder zur Messung von Glucose in einer Ex-vivo-Probe mittels einer Glucoseoxidase nach den Ansprüchen 1 bis 11 oder eines aktiven Fragments davon, wobei der Nachweis, die Bestimmung oder die Messung das Inkontaktbringen einer Ex-vivo-Probe mit der Glucoseoxidase oder einem Fragment davon umfasst.

13. Verfahren nach Anspruch 12, wobei die Bestimmung oder Messung von Glucose mittels eines Sensors oder eines Teststreifengeräts durchgeführt wird.

## Revendications

1. Glucose-oxydase selon SEQ ID NO : 1, choisi dans le groupe composé d'une
a) glucose-oxydase selon SEQ ID NO : 1, comportant, en plus des deux substitutions d'acides aminés T30V et I94V, au moins une substitution additionnelle d'acide aminé dans l'une quelconque des cinq positions choisies dans le groupe S53; A173; A332; F414; V560 dans ladite SEQ ID NO : 1; ou
b) glucose-oxydase présentant au moins 70 %, particulièrement au moins 80 %, ou > 90 % d'identité de séquence d'acide aminé avec la glucose-oxydase selon a) pourvu que la glucose-oxydase de b) comporte, en plus des deux substitutions d'acides aminés T30V et I94V, au moins une substitution d'acide aminé dans l'une quelconque des cinq positions choisies dans le groupe S53; A173; A332; F414; V560 selon SEQ ID NO : 1;
et pourvu que la glucose-oxydase selon b) présente au moins 70 %, particulièrement au moins 80 %, ou > 90 % de l'activité enzymatique de la glucose-oxydase selon a), et présente au moins 70 %, particulièrement au moins 80 %, ou > 90 % de la spécificité enzymatique pour le glucose de la glucose-oxydase selon a);
et pourvu que la glucose-oxydase selon b) présente au moins une activité cinq fois réduite de l'oxygène en tant qu'accepteur d'électrons de la glucose-oxydase selon SEQ ID NO : 1 ou présente au moins une activité une fois et demie accrue pour des médiateurs d'électrons autres que l'oxygène de la glucose-oxydase selon SEQ ID NO : 1, ou les deux; ou
c) un fragment actif d'une glucose-oxydase selon a) ou b), pourvu que dans le fragment actif selon c), les substitutions d'acides aminés telles qu'indiquées à a) ou b) soient préservées par rapport à la glucose-oxydase selon a) ou b);
et pourvu que la glucose-oxydase selon c) présente au moins 70 %, particulièrement au moins 80 %, ou > 90 % de l'activité enzymatique de la glucose-oxydase selon a), et présente au moins 70 %, particulièrement au moins 80 %, ou > 90 % de la spécificité enzymatique pour le glucose de la glucose-oxydase selon a);
et pourvu que la glucose-oxydase selon c) présente au moins une activité cinq fois réduite de l'oxygène en tant qu'accepteur d'électrons de la glucose-oxydase selon SEQ ID NO : 1 ou présente au moins une activité une fois et demie accrue pour des médiateurs d'électrons autres que l'oxygène de la glucose-oxydase selon SEQ ID NO : 1, ou les deux, et les acides aminés de la ou les substitutions additionnelles étant choisis dans le groupe
Phe pour la position 553; et/ou
Ile, Thr, Val pour la position A173; et/ou
Ser, Val, Thr pour la position A332; et/ou
Ile, Leu, Met, Val pour la position F414; et/ou
Leu, Pro, Thr, pour la position V560.

2. Glucose-oxydase selon la revendication 1,
• ladite activité pour l'oxygène en tant qu'accepteur d'électrons étant déterminée par essai ABTS, comprenant les étapes suivantes :
a) 75 µL d'échantillon de solution enzymatique sont transférés à une microplaque à fond plat de 96 puits contenant 100 µL de tampon de phosphate (pH 7);
b) 20 µL de mélange de réaction sont ajoutés à chaque puits, donnant les concentrations suivantes : 0,91 U/mL HRP; 2,3 mM ABTS (acide 2,2'-azino-bis(3-éthylbenzothiazoline-6-sulfonique));
c) la réaction commence par l'ajout de 25 µL de solution de substrat de glucose et agitation subséquente de la plaque à 1000 tr/min pendant 30 secondes;
d) l'oxydation de l'ABTS est déterminée cinétiquement à 414 nm à l'aide d'un lecteur de microplaques.
• ladite activité des médiateurs d'électrons autres que l'oxygène est déterminée par dosage médiateur, comprenant les étapes :
a) 75 µL d'échantillon de solution enzymatique sont transférés à une microplaque à fond plat de 96 puits;
b) 100 µL de solution médiatrice (19,05 mM N,N-bis(2-hydroxyéthyl)-4-nitrosoaniline) ; 5 % (p/p) de polyvinylpyrrolidone, pH 7 et 20 µL de 25 mM d'acide phosphomolybdique sont ajoutés;
c) la réaction commence par ajout de 25 µL de solution de substrat de glucose et l'agitation subséquente de la plaque à 1000 tr/min pendant 1 minute;
d) la cinétique de la réduction de l'acide phosphomolybdique est surveillée à 700 nm à l'aide d'un lecteur de microplaques.

3. Glucose-oxydase selon la revendication 1 ou 2, lesdits médiateurs d'électrons autres que l'oxygène étant choisis dans le groupe comprenant les nitrosoanilines et leurs dérivés, les composés azoïques, les phénazines et leurs dérivés, les phénothiazines et leurs dérivés, les phénothiazines et leurs dérivés, les phénoxazines et leurs dérivés, les ferrocènes et leurs dérivés, les ferricyanures de potassium, les complexes Ru- et Os-, les quinones et leurs dérivés, les indophénols, les viologènes, le tétrathiafulvalène et ses dérivés, et les phtalocyanines.

4. Glucose-oxydase selon l'une quelconque des revendications précédentes, comportant, en plus des deux substitutions T30V et I94V selon SEQ ID NO : 1, deux, trois, quatre ou cinq substitutions additionnelles d'acides aminés dans chacune des cinq positions choisies dans le groupe S53 ; A173 ; A332 ; F414 ; V560 selon SEQ ID NO : 1.

5. Glucose-oxydase selon l'une quelconque des revendications précédentes, outre les substitutions T30V et I94V selon SEQ ID NO : 1, au moins une substitution additionnelle d'acide aminé dans la ou les positions F414 et/ou V560 étant associé à au moins une substitution d'acide aminé dans la ou les positions A173 et/ou A332.

6. Glucose-oxydase selon l'une quelconque des revendications précédentes, comportant, en plus des substitutions T30V et I94V, les substitutions additionnelles d'acides aminés A173I; A332S; et F414L selon SEQ ID NO : 4.

7. Glucose-oxydase selon l'une quelconque des revendications précédentes, comportant, en plus des substitutions T30V et I94V, les substitutions additionnelles d'acides aminés A173V; A332S; F414I; et V560T selon SEQ ID NO : 3.

8. Glucose-oxydase selon l'une quelconque des revendications précédentes, comportant, en plus des substitutions T30V et I94V, les substitutions additionnelles d'acides aminés A173V; A332N; F414V; et V560L selon la SEQ ID NO : 6.

9. Glucose-oxydase selon l'une quelconque des revendications précédentes, présentant une spécificité de glucose d'au moins 99,9 % et/ou une spécificité de galactose < 4 % et/ou une spécificité de maltose < 0,3 % et/ou une spécificité de xylose < 6 % et/ou une spécificité de maltotriose < 0,1 %, lorsque déterminée par essai médiateur, comprenant les étapes :
a) 75 µL d'échantillon de solution enzymatique sont transférés à une microplaque à fond plat de 96 puits;
b) 100 µL de solution médiatrice (19,05 mM N,N-bis(2-hydroxyéthyl)-4-nitrosoaniline) ; 5 % (p/p) de polyvinylpyrrolidone, pH 7 et 20 µL de 25 mM d'acide phosphomolybdique sont ajoutés;
c) la réaction commence par ajout de 25 µL de solution de substrat de sucre respective et l'agitation subséquente de la plaque à 1000 tr/min pendant 1 minute;
d) la cinétique de la réduction de l'acide phosphomolybdique est surveillée à 700 nm à l'aide d'un lecteur de microplaques.

10. Glucose-oxydase selon l'une quelconque des revendications précédentes, présentant une activité du médiateur de nitrosoaniline > 400 %, ou > 500 % ou > 600 % du transfert d'électrons par rapport à l'activité du médiateur de nitrosoaniline du GOx selon SEQ ID NO : 1 au moyen de l'essai médiateur, comprenant les étapes :
a) 75 µL d'échantillon de solution enzymatique sont transférés à une microplaque à fond plat de 96 puits;
b) 100 µL de solution médiatrice (19,05 mM N,N-bis(2-hydroxyéthyl)-4-nitrosoaniline) ; 5 % (p/p) de polyvinylpyrrolidone, pH 7 et 20 µL de 25 mM d'acide phosphomolybdique sont ajoutés;
c) la réaction commence par ajout de 25 µL de solution de substrat de glucose et l'agitation subséquente de la plaque à 1000 tr/min pendant 1 minute;
d) la cinétique de la réduction de l'acide phosphomolybdique est surveillée à 700 nm à l'aide d'un lecteur de microplaques,
et une activité d'oxygène de ≤ 30 %, ou < 25 %, ou < 20 %, particulièrement < 15 %, ou ≤ 10% par rapport à l'activité d'oxygène de la GOx selon SEQ ID NO : 1 au moyen d'un dosage ABTS, comprenant les étapes suivantes :
a) 75 µL d'échantillon de solution enzymatique sont transférés à une microplaque à fond plat de 96 puits contenant 100 µL de tampon de phosphate (pH 7);
b) 20 µL de mélange de réaction sont ajoutés à chaque puits, donnant les concentrations suivantes : 0,91 U/mL HRP; 2,3 mM ABTS (acide 2,2'-azino-bis(3-éthylbenzothiazoline-6-sulfonique));
c) la réaction commence par ajout de 25 µL de solution de substrat de glucose et agitation subséquente de la plaque à 1000 tr/min pendant 30 secondes;
d) l'oxydation de l'ABTS est déterminée cinétiquement à 414 nm à l'aide d'un lecteur de microplaques.

11. Polynucléotide isolé codant pour une glucose-oxydase selon l'une quelconque des revendications précédentes ou un fragment actif de celle-ci.

12. Procédé de détection, de détermination ou de mesure du glucose dans un échantillon ex vivo par une glucose-oxydase selon les revendications 1 à 11 ou un fragment actif de celle-ci, lesdites détection, détermination ou mesure comprenant la mise en contact d'un échantillon ex vivo avec ladite glucose-oxydase ou un fragment actif de celle-ci.

13. Procédé selon la revendication 12, lesdites détection, détermination ou mesure du glucose étant effectuées à l'aide d'un capteur ou d'un dispositif de bande de test.


### SEQUENCE LISTING

<110> Roche Diagnostics GmbH
   F. Hoffmann-La Roche AG
<120> Novel glucose oxidases derived from Aspergillus niger
<130> R75001WO
<150> 13152935.6
   <151> 2013-01-28
<160> 17
<170> PatentIn version 3.3
<210> 1
   <211> 583
   <212> PRT
   <213> Aspergillus niger
<400> 1
   Ser Asn Gly Ile Glu Ala Ser Leu Leu Thr Asp Pro Lys Asp Val Ser
   1               5                   10                  15
   Gly Arg Thr Val Asp Tyr Ile Ile Ala Gly Gly Gly Leu Val Gly Leu
               20                  25                  30
   Thr Thr Ala Ala Arg Leu Thr Glu Asn Pro Asn Ile Ser Val Leu Val
           35                  40                  45
   Ile Glu Ser Gly Ser Tyr Glu Ser Asp Arg Gly Pro Ile Ile Glu Asp
       50                  55                  60
   Leu Asn Ala Tyr Gly Asp Ile Phe Gly Ser Ser Val Asp His Ala Tyr
   65                  70                  75                  80
   Glu Thr Val Glu Leu Ala Thr Asn Asn Gln Thr Ala Leu Val Arg Ser
                   85                  90                  95
   Gly Asn Gly Leu Gly Gly Ser Thr Leu Val Asn Gly Gly Thr Trp Thr
               100                 105                 110
   Arg Pro His Lys Ala Gln Val Asp Ser Trp Glu Thr Val Phe Gly Asn
           115                 120                 125
   Glu Gly Trp Asn Trp Asp Asn Val Ala Ala Tyr Ser Leu Gln Ala Glu
       130                 135                 140
   Arg Ala Arg Ala Pro Asn Ala Lys Gln Ile Ala Ala Gly His Tyr Phe
   145                 150                 155                 160
   Asn Ala Ser Cys His Gly Val Asn Gly Thr Val His Ala Gly Pro Arg
                   165                 170                 175
   Asp Thr Gly Asp Asp Tyr Ser Pro Ile Val Lys Ala Leu Met Ser Ala
               180                 185                 190
   Val Glu Asp Arg Gly Val Pro Thr Lys Lys Asp Phe Gly Cys Gly Asp
           195                 200                 205
   Pro His Gly Val Ser Met Phe Pro Asn Thr Leu His Glu Asp Gln Val
       210                 215                 220
   Arg Ser Asp Ala Ala Arg Glu Trp Leu Leu Pro Asn Tyr Gln Arg Pro
   225                 230                 235                 240
   Asn Leu Gln Val Leu Thr Gly Gln Tyr Val Gly Lys Val Leu Leu Ser
                   245                 250                 255
   Gln Asn Gly Thr Thr Pro Arg Ala Val Gly Val Glu Phe Gly Thr His
               260                 265                 270
   Lys Gly Asn Thr His Asn Val Tyr Ala Lys His Glu Val Leu Leu Ala
           275                 280                 285
   Ala Gly Ser Ala Val Ser Pro Thr Ile Leu Glu Tyr Ser Gly Ile Gly
       290                 295                 300
   Met Lys Ser Ile Leu Glu Pro Leu Gly Ile Asp Thr Val Val Asp Leu
   305                 310                 315                 320
   Pro Val Gly Leu Asn Leu Gln Asp Gln Thr Thr Ala Thr Val Arg Ser
                   325                 330                 335
   Arg Ile Thr Ser Ala Gly Ala Gly Gln Gly Gln Ala Ala Trp Phe Ala
               340                 345                 350
   Thr Phe Asn Glu Thr Phe Gly Asp Tyr Ser Glu Lys Ala His Glu Leu
           355                 360                 365
   Leu Asn Thr Lys Leu Glu Gln Trp Ala Glu Glu Ala Val Ala Arg Gly
       370                 375                 380
   Gly Phe His Asn Thr Thr Ala Leu Leu Ile Gln Tyr Glu Asn Tyr Arg
   385                 390                 395                 400
   Asp Trp Ile Val Asn His Asn Val Ala Tyr Ser Glu Leu Phe Leu Asp
                   405                 410                 415
   Thr Ala Gly Val Ala Ser Phe Asp Val Trp Asp Leu Leu Pro Phe Thr
                   420                 425                 430
   Arg Gly Tyr Val His Ile Leu Asp Lys Asp Pro Tyr Leu His His Phe
               435                 440                 445
   Ala Tyr Asp Pro Gln Tyr Phe Leu Asn Glu Leu Asp Leu Leu Gly Gln
       450                 455                 460
   Ala Ala Ala Thr Gln Leu Ala Arg Asn Ile Ser Asn Ser Gly Ala Met
   465                 470                 475                 480
   Gln Thr Tyr Phe Ala Gly Glu Thr Ile Pro Gly Asp Asn Leu Ala Tyr
                   485                 490                 495
   Asp Ala Asp Leu Ser Ala Trp Thr Glu Tyr Ile Pro Tyr His Phe Arg
                   500                 505                 510
   Pro Asn Tyr His Gly Val Gly Thr Cys Ser Met Met Pro Lys Glu Met
               515                 520                 525
   Gly Gly Val Val Asp Asn Ala Ala Arg Val Tyr Gly Val Gln Gly Leu
       530                 535                 540
   Arg Val Ile Asp Gly Ser Ile Pro Pro Thr Gln Met Ser Ser His Val
   545                 550                 555                 560
   Met Thr Val Phe Tyr Ala Met Ala Leu Lys Ile Ser Asp Ala Ile Leu
                   565                 570                 575
   Glu Asp Tyr Ala Ser Met Gln
                   580
<210> 2
   <211> 583
   <212> PRT
   <213> Aspergillus niger
<400> 2
   Ser Asn Gly Ile Glu Ala Ser Leu Leu Thr Asp Pro Lys Asp Val Ser
   1               5                   10                  15
   Gly Arg Thr Val Asp Tyr Ile Ile Ala Gly Gly Gly Leu Thr Gly Leu
                   20                  25                  30
   Thr Thr Ala Ala Arg Leu Thr Glu Asn Pro Asn Ile Ser Val Leu Val
               35                  40                  45
   Ile Glu Ser Gly Ser Tyr Glu Ser Asp Arg Gly Pro Ile Ile Glu Asp
       50                  55                  60
   Leu Asn Ala Tyr Gly Asp Ile Phe Gly Ser Ser Val Asp His Ala Tyr
   65                  70                  75                  80
   Glu Thr Val Glu Leu Ala Thr Asn Asn Gln Thr Ala Leu Ile Arg Ser
                   85                  90                  95
   Gly Asn Gly Leu Gly Gly Ser Thr Leu Val Asn Gly Gly Thr Trp Thr
               100                 105                 110
   Arg Pro His Lys Ala Gln Val Asp Ser Trp Glu Thr Val Phe Gly Asn
           115                 120                 125
   Glu Gly Trp Asn Trp Asp Asn Val Ala Ala Tyr Ser Leu Gln Ala Glu
       130                 135                 140
   Arg Ala Arg Ala Pro Asn Ala Lys Gln Ile Ala Ala Gly His Tyr Phe
   145                 150                 155                 160
   Asn Ala Ser Cys His Gly Val Asn Gly Thr Val His Ala Gly Pro Arg
                   165                 170                 175
   Asp Thr Gly Asp Asp Tyr Ser Pro Ile Val Lys Ala Leu Met Ser Ala
               180                 185                 190
   Val Glu Asp Arg Gly Val Pro Thr Lys Lys Asp Phe Gly Cys Gly Asp
           195                 200                 205
   Pro His Gly Val Ser Met Phe Pro Asn Thr Leu His Glu Asp Gln Val
       210                 215                 220
   Arg Ser Asp Ala Ala Arg Glu Trp Leu Leu Pro Asn Tyr Gln Arg Pro
   225                 230                 235                 240
   Asn Leu Gln Val Leu Thr Gly Gln Tyr Val Gly Lys Val Leu Leu Ser
                   245                 250                 255
   Gln Asn Gly Thr Thr Pro Arg Ala Val Gly Val Glu Phe Gly Thr His
               260                 265                 270
   Lys Gly Asn Thr His Asn Val Tyr Ala Lys His Glu Val Leu Leu Ala
           275                 280                 285
   Ala Gly Ser Ala Val Ser Pro Thr Ile Leu Glu Tyr Ser Gly Ile Gly
       290                 295                 300
   Met Lys Ser Ile Leu Glu Pro Leu Gly Ile Asp Thr Val Val Asp Leu
   305                 310                 315                 320
   Pro Val Gly Leu Asn Leu Gln Asp Gln Thr Thr Ala Thr Val Arg Ser
                   325                 330                 335
   Arg Ile Thr Ser Ala Gly Ala Gly Gln Gly Gln Ala Ala Trp Phe Ala
               340                 345                 350
   Thr Phe Asn Glu Thr Phe Gly Asp Tyr Ser Glu Lys Ala His Glu Leu
           355                 360                 365
   Leu Asn Thr Lys Leu Glu Gln Trp Ala Glu Glu Ala Val Ala Arg Gly
       370                 375                 380
   Gly Phe His Asn Thr Thr Ala Leu Leu Ile Gln Tyr Glu Asn Tyr Arg
   385                 390                 395                 400
   Asp Trp Ile Val Asn His Asn Val Ala Tyr Ser Glu Leu Phe Leu Asp
                   405                 410                 415
   Thr Ala Gly Val Ala Ser Phe Asp Val Trp Asp Leu Leu Pro Phe Thr
               420                 425                 430
   Arg Gly Tyr Val His Ile Leu Asp Lys Asp Pro Tyr Leu His His Phe
           435                 440                 445
   Ala Tyr Asp Pro Gln Tyr Phe Leu Asn Glu Leu Asp Leu Leu Gly Gln
       450                 455                 460
   Ala Ala Ala Thr Gln Leu Ala Arg Asn Ile Ser Asn Ser Gly Ala Met
   465                 470                 475                 480
   Gln Thr Tyr Phe Ala Gly Glu Thr Ile Pro Gly Asp Asn Leu Ala Tyr
                   485                 490                 495
   Asp Ala Asp Leu Ser Ala Trp Thr Glu Tyr Ile Pro Tyr His Phe Arg
               500                 505                 510
   Pro Asn Tyr His Gly Val Gly Thr Cys Ser Met Met Pro Lys Glu Met
           515                 520                 525
   Gly Gly Val Val Asp Asn Ala Ala Arg Val Tyr Gly Val Gln Gly Leu
       530                 535                 540
   Arg Val Ile Asp Gly Ser Ile Pro Pro Thr Gln Met Ser Ser His Val
   545 550 555 560
   Met Thr Val Phe Tyr Ala Met Ala Leu Lys Ile Ser Asp Ala Ile Leu
   565 570 575
   Glu Asp Tyr Ala Ser Met Gln
   580
<210> 3
   <211> 583
   <212> PRT
   <213> Aspergillus niger
<400> 3
   Ser Asn Gly Ile Glu Ala Ser Leu Leu Thr Asp Pro Lys Asp Val Ser
   1 5 10 15
   Gly Arg Thr Val Asp Tyr Ile Ile Ala Gly Gly Gly Leu Val Gly Leu
   20 25 30
   Thr Thr Ala Ala Arg Leu Thr Glu Asn Pro Asn Ile Ser Val Leu Val
   35 40 45
   Ile Glu Ser Gly Ser Tyr Glu Ser Asp Arg Gly Pro Ile Ile Glu Asp
   50 55 60
   Leu Asn Ala Tyr Gly Asp Ile Phe Gly Ser Ser Val Asp His Ala Tyr
   65 70 75 80
   Glu Thr Val Glu Leu Ala Thr Asn Asn Gln Thr Ala Leu Val Arg Ser
   85 90 95
   Gly Asn Gly Leu Gly Gly Ser Thr Leu Val Asn Gly Gly Thr Trp Thr
   100 105 110
   Arg Pro His Lys Ala Gln Val Asp Ser Trp Glu Thr Val Phe Gly Asn
   115 120 125
   Glu Gly Trp Asn Trp Asp Asn Val Ala Ala Tyr Ser Leu Gln Ala Glu
   130 135 140
   Arg Ala Arg Ala Pro Asn Ala Lys Gln Ile Ala Ala Gly His Tyr Phe
   145 150 155 160
   Asn Ala Ser Cys His Gly Val Asn Gly Thr Val His Val Gly Pro Arg
   165 170 175
   Asp Thr Gly Asp Asp Tyr Ser Pro Ile Val Lys Ala Leu Met Ser Ala
               180                 185                 190
   Val Glu Asp Arg Gly Val Pro Thr Lys Lys Asp Phe Gly Cys Gly Asp
           195                 200                 205
   Pro His Gly Val Ser Met Phe Pro Asn Thr Leu His Glu Asp Gln Val
       210                 215                 220
   Arg Ser Asp Ala Ala Arg Glu Trp Leu Leu Pro Asn Tyr Gln Arg Pro
   225                 230                 235                 240
   Asn Leu Gln Val Leu Thr Gly Gln Tyr Val Gly Lys Val Leu Leu Ser
                   245                 250                 255
   Gln Asn Gly Thr Thr Pro Arg Ala Val Gly Val Glu Phe Gly Thr His
               260                 265                 270
   Lys Gly Asn Thr His Asn Val Tyr Ala Lys His Glu Val Leu Leu Ala
           275                 280                 285
   Ala Gly Ser Ala Val Ser Pro Thr Ile Leu Glu Tyr Ser Gly Ile Gly
       290                 295                 300
   Met Lys Ser Ile Leu Glu Pro Leu Gly Ile Asp Thr Val Val Asp Leu
   305                 310                 315                 320
   Pro Val Gly Leu Asn Leu Gln Asp Gln Thr Thr Ser Thr Val Arg Ser
                   325                 330                 335
   Arg Ile Thr Ser Ala Gly Ala Gly Gln Gly Gln Ala Ala Trp Phe Ala
               340                 345                 350
   Thr Phe Asn Glu Thr Phe Gly Asp Tyr Ser Glu Lys Ala His Glu Leu
           355                 360                 365
   Leu Asn Thr Lys Leu Glu Gln Trp Ala Glu Glu Ala Val Ala Arg Gly
       370                 375                 380
   Gly Phe His Asn Thr Thr Ala Leu Leu Ile Gln Tyr Glu Asn Tyr Arg
   385                 390                 395                 400
   Asp Trp Ile Val Asn His Asn Val Ala Tyr Ser Glu Leu Ile Leu Asp
                   405                 410                 415
   Thr Ala Gly Val Ala Ser Phe Asp Val Trp Asp Leu Leu Pro Phe Thr
               420                 425                 430
   Arg Gly Tyr Val His Ile Leu Asp Lys Asp Pro Tyr Leu His His Phe
           435                 440                 445
   Ala Tyr Asp Pro Gln Tyr Phe Leu Asn Glu Leu Asp Leu Leu Gly Gln
       450                 455                 460
   Ala Ala Ala Thr Gln Leu Ala Arg Asn Ile Ser Asn Ser Gly Ala Met
   465                 470                 475                 480
   Gln Thr Tyr Phe Ala Gly Glu Thr Ile Pro Gly Asp Asn Leu Ala Tyr
                   485                 490                 495
   Asp Ala Asp Leu Ser Ala Trp Thr Glu Tyr Ile Pro Tyr His Phe Arg
               500                 505                 510
   Pro Asn Tyr His Gly Val Gly Thr Cys Ser Met Met Pro Lys Glu Met
           515                 520                 525
   Gly Gly Val Val Asp Asn Ala Ala Arg Val Tyr Gly Val Gln Gly Leu
       530                 535                 540
   Arg Val Ile Asp Gly Ser Ile Pro Pro Thr Gln Met Ser Ser His Thr
   545                 550                 555                 560
   Met Thr Val Phe Tyr Ala Met Ala Leu Lys Ile Ser Asp Ala Ile Leu
                   565                 570                 575
   Glu Asp Tyr Ala Ser Met Gln
               580
<210> 4
   <211> 583
   <212> **PRT**
   <213> Aspergillus niger
<400> 4
   Ser Asn Gly Ile Glu Ala Ser Leu Leu Thr Asp Pro Lys Asp Val Ser
   1               5                   10                  15
   Gly Arg Thr Val Asp Tyr Ile Ile Ala Gly Gly Gly Leu Val Gly Leu
               20                  25                  30
   Thr Thr Ala Ala Arg Leu Thr Glu Asn Pro Asn Ile Ser Val Leu Val
           35                  40                  45
   Ile Glu Ser Gly Ser Tyr Glu Ser Asp Arg Gly Pro Ile Ile Glu Asp
       50                  55                  60
   Leu Asn Ala Tyr Gly Asp Ile Phe Gly Ser Ser Val Asp His Ala Tyr
   65 70 75 80
   Glu Thr Val Glu Leu Ala Thr Asn Asn Gln Thr Ala Leu Val Arg Ser
   85 90 95
   Gly Asn Gly Leu Gly Gly Ser Thr Leu Val Asn Gly Gly Thr Trp Thr
   100 105 110
   Arg Pro His Lys Ala Gln Val Asp Ser Trp Glu Thr Val Phe Gly Asn
   115 120 125
   Glu Gly Trp Asn Trp Asp Asn Val Ala Ala Tyr Ser Leu Gln Ala Glu
   130 135 140
   Arg Ala Arg Ala Pro Asn Ala Lys Gln Ile Ala Ala Gly His Tyr Phe
   145 150 155 160
   Asn Ala Ser Cys His Gly Val Asn Gly Thr Val His Ile Gly Pro Arg
   165 170 175
   Asp Thr Gly Asp Asp Tyr Ser Pro Ile Val Lys Ala Leu Met Ser Ala
   180 185 190
   Val Glu Asp Arg Gly Val Pro Thr Lys Lys Asp Phe Gly Cys Gly Asp
   195 200 205
   Pro His Gly Val Ser Met Phe Pro Asn Thr Leu His Glu Asp Gln Val
   210 215 220
   Arg Ser Asp Ala Ala Arg Glu Trp Leu Leu Pro Asn Tyr Gln Arg Pro
   225 230 235 240
   Asn Leu Gln Val Leu Thr Gly Gln Tyr Val Gly Lys Val Leu Leu Ser
   245 250 255
   Gln Asn Gly Thr Thr Pro Arg Ala Val Gly Val Glu Phe Gly Thr His
   260 265 270
   Lys Gly Asn Thr His Asn Val Tyr Ala Lys His Glu Val Leu Leu Ala
   275 280 285
   Ala Gly Ser Ala Val Ser Pro Thr Ile Leu Glu Tyr Ser Gly Ile Gly
   290 295 300
   Met Lys Ser Ile Leu Glu Pro Leu Gly Ile Asp Thr Val Val Asp Leu
   305 310 315 320
   Pro Val Gly Leu Asn Leu Gln Asp Gln Thr Thr Ser Thr Val Arg Ser
                   325                 330                 335
   Arg Ile Thr Ser Ala Gly Ala Gly Gln Gly Gln Ala Ala Trp Phe Ala
               340                 345                 350
   Thr Phe Asn Glu Thr Phe Gly Asp Tyr Ser Glu Lys Ala His Glu Leu
           355                 360                 365
   Leu Asn Thr Lys Leu Glu Gln Trp Ala Glu Glu Ala Val Ala Arg Gly
       370                 375                 380
   Gly Phe His Asn Thr Thr Ala Leu Leu Ile Gln Tyr Glu Asn Tyr Arg
   385                 390                 395                 400
   Asp Trp Ile Val Asn His Asn Val Ala Tyr Ser Glu Leu Leu Leu Asp
                   405                 410                 415
   Thr Ala Gly Val Ala Ser Phe Asp Val Trp Asp Leu Leu Pro Phe Thr
               420                 425                 430
   Arg Gly Tyr Val His Ile Leu Asp Lys Asp Pro Tyr Leu His His Phe
           435                 440                 445
   Ala Tyr Asp Pro Gln Tyr Phe Leu Asn Glu Leu Asp Leu Leu Gly Gln
       450                 455                 460
   Ala Ala Ala Thr Gln Leu Ala Arg Asn Ile Ser Asn Ser Gly Ala Met
   465                 470                 475                 480
   Gln Thr Tyr Phe Ala Gly Glu Thr Ile Pro Gly Asp Asn Leu Ala Tyr
                   485                 490                 495
   Asp Ala Asp Leu Ser Ala Trp Thr Glu Tyr Ile Pro Tyr His Phe Arg
               500                 505                 510
   Pro Asn Tyr His Gly Val Gly Thr Cys Ser Met Met Pro Lys Glu Met
           515                 520                 525
   Gly Gly Val Val Asp Asn Ala Ala Arg Val Tyr Gly Val Gln Gly Leu
       530                 535                 540
   Arg Val Ile Asp Gly Ser Ile Pro Pro Thr Gln Met Ser Ser His Val
   545                 550                 555                 560
   Met Thr Val Phe Tyr Ala Met Ala Leu Lys Ile Ser Asp Ala Ile Leu
                   565                 570                 575
   Glu Asp Tyr Ala Ser Met Gln
   580
<210> 5
   <211> 583
   <212> PRT
   <213> Aspergillus niger
<400> 5
   Ser Asn Gly Ile Glu Ala Ser Leu Leu Thr Asp Pro Lys Asp Val Ser
   1 5 10 15
   Gly Arg Thr Val Asp Tyr Ile Ile Ala Gly Gly Gly Leu Val Gly Leu
   20 25 30
   Thr Thr Ala Ala Arg Leu Thr Glu Asn Pro Asn Ile Ser Val Leu Val
   35 40 45
   Ile Glu Ser Gly Ser Tyr Glu Ser Asp Arg Gly Pro Ile Ile Glu Asp
   50 55 60
   Leu Asn Ala Tyr Gly Asp Ile Phe Gly Ser Ser Val Asp His Ala Tyr
   65 70 75 80
   Glu Thr Val Glu Leu Ala Thr Asn Asn Gln Thr Ala Leu Val Arg Ser
   85 90 95
   Gly Asn Gly Leu Gly Gly Ser Thr Leu Val Asn Gly Gly Thr Trp Thr
   100 105 110
   Arg Pro His Lys Ala Gln Val Asp Ser Trp Glu Thr Val Phe Gly Asn
   115 120 125
   Glu Gly Trp Asn Trp Asp Asn Val Ala Ala Tyr Ser Leu Gln Ala Glu
   130 135 140
   Arg Ala Arg Ala Pro Asn Ala Lys Gln Ile Ala Ala Gly His Tyr Phe
   145 150 155 160
   Asn Ala Ser Cys His Gly Val Asn Gly Thr Val His Val Gly Pro Arg
   165 170 175
   Asp Thr Gly Asp Asp Tyr Ser Pro Ile Val Lys Ala Leu Met Ser Ala
   180 185 190
   Val Glu Asp Arg Gly Val Pro Thr Lys Lys Asp Phe Gly Cys Gly Asp
   195 200 205
   Pro His Gly Val Ser Met Phe Pro Asn Thr Leu His Glu Asp Gln Val
       210                 215                 220
   Arg Ser Asp Ala Ala Arg Glu Trp Leu Leu Pro Asn Tyr Gln Arg Pro
   225                 230                 235                 240
   Asn Leu Gln Val Leu Thr Gly Gln Tyr Val Gly Lys Val Leu Leu Ser
                   245                 250                 255
   Gln Asn Gly Thr Thr Pro Arg Ala Val Gly Val Glu Phe Gly Thr His
               260                 265                 270
   Lys Gly Asn Thr His Asn Val Tyr Ala Lys His Glu Val Leu Leu Ala
           275                 280                 285
   Ala Gly Ser Ala Val Ser Pro Thr Ile Leu Glu Tyr Ser Gly Ile Gly
       290                 295                 300
   Met Lys Ser Ile Leu Glu Pro Leu Gly Ile Asp Thr Val Val Asp Leu
   305                 310                 315                 320
   Pro Val Gly Leu Asn Leu Gln Asp Gln Thr Thr Asn Thr Val Arg Ser
                   325                 330                 335
   Arg Ile Thr Ser Ala Gly Ala Gly Gln Gly Gln Ala Ala Trp Phe Ala
               340                 345                 350
   Thr Phe Asn Glu Thr Phe Gly Asp Tyr Ser Glu Lys Ala His Glu Leu
           355                 360                 365
   Leu Asn Thr Lys Leu Glu Gln Trp Ala Glu Glu Ala Val Ala Arg Gly
       370                 375                 380
   Gly Phe His Asn Thr Thr Ala Leu Leu Ile Gln Tyr Glu Asn Tyr Arg
   385                 390                 395                 400
   Asp Trp Ile Val Asn His Asn Val Ala Tyr Ser Glu Leu Phe Leu Asp
                   405                 410                 415
   Thr Ala Gly Val Ala Ser Phe Asp Val Trp Asp Leu Leu Pro Phe Thr
               420                 425                 430
   Arg Gly Tyr Val His Ile Leu Asp Lys Asp Pro Tyr Leu His His Phe
           435                 440                 445
   Ala Tyr Asp Pro Gln Tyr Phe Leu Asn Glu Leu Asp Leu Leu Gly Gln
       450                 455                 460
   Ala Ala Ala Thr Gln Leu Ala Arg Asn Ile Ser Asn Ser Gly Ala Met
   465                 470                 475                 480
   Gln Thr Tyr Phe Ala Gly Glu Thr Ile Pro Gly Asp Asn Leu Ala Tyr
                   485                 490                 495
   Asp Ala Asp Leu Ser Ala Trp Thr Glu Tyr Ile Pro Tyr His Phe Arg
               500                 505                 510
   Pro Asn Tyr His Gly Val Gly Thr Cys Ser Met Met Pro Lys Glu Met
           515                 520                 525
   Gly Gly Val Val Asp Asn Ala Ala Arg Val Tyr Gly Val Gln Gly Leu
       530                 535                 540
   Arg Val Ile Asp Gly Ser Ile Pro Pro Thr Gln Met Ser Ser His Pro
   545                 550                 555                 560
   Met Thr Val Phe Tyr Ala Met Ala Leu Lys Ile Ser Asp Ala Ile Leu
                   565                 570                 575
   Glu Asp Tyr Ala Ser Met Gln
               580
<210> 6
   <211> 583
   <212> PRT
   <213> Aspergillus niger
<400> 6
   Ser Asn Gly Ile Glu Ala Ser Leu Leu Thr Asp Pro Lys Asp Val Ser
   1               5                   10                  15
   Gly Arg Thr Val Asp Tyr Ile Ile Ala Gly Gly Gly Leu Val Gly Leu
               20                  25                  30
   Thr Thr Ala Ala Arg Leu Thr Glu Asn Pro Asn Ile Ser Val Leu Val
           35                  40                  45
   Ile Glu Ser Gly Ser Tyr Glu Ser Asp Arg Gly Pro Ile Ile Glu Asp
       50                  55                  60
   Leu Asn Ala Tyr Gly Asp Ile Phe Gly Ser Ser Val Asp His Ala Tyr
   65                  70                  75                  80
   Glu Thr Val Glu Leu Ala Thr Asn Asn Gln Thr Ala Leu Val Arg Ser
                   85                  90                  95
   Gly Asn Gly Leu Gly Gly Ser Thr Leu Val Asn Gly Gly Thr Trp Thr
               100                 105                 110
   Arg Pro His Lys Ala Gln Val Asp Ser Trp Glu Thr Val Phe Gly Asn
           115                 120                 125
   Glu Gly Trp Asn Trp Asp Asn Val Ala Ala Tyr Ser Leu Gln Ala Glu
       130                 135                 140
   Arg Ala Arg Ala Pro Asn Ala Lys Gln Ile Ala Ala Gly His Tyr Phe
   145                 150                 155                 160
   Asn Ala Ser Cys His Gly Val Asn Gly Thr Val His Val Gly Pro Arg
                   165                 170                 175
   Asp Thr Gly Asp Asp Tyr Ser Pro Ile Val Lys Ala Leu Met Ser Ala
               180                 185                 190
   Val Glu Asp Arg Gly Val Pro Thr Lys Lys Asp Phe Gly Cys Gly Asp
           195                 200                 205
   Pro His Gly Val Ser Met Phe Pro Asn Thr Leu His Glu Asp Gln Val
       210                 215                 220
   Arg Ser Asp Ala Ala Arg Glu Trp Leu Leu Pro Asn Tyr Gln Arg Pro
   225                 230                 235                 240
   Asn Leu Gln Val Leu Thr Gly Gln Tyr Val Gly Lys Val Leu Leu Ser
                   245                 250                 255
   Gln Asn Gly Thr Thr Pro Arg Ala Val Gly Val Glu Phe Gly Thr His
               260                 265                 270
   Lys Gly Asn Thr His Asn Val Tyr Ala Lys His Glu Val Leu Leu Ala
           275                 280                 285
   Ala Gly Ser Ala Val Ser Pro Thr Ile Leu Glu Tyr Ser Gly Ile Gly
       290                 295                 300
   Met Lys Ser Ile Leu Glu Pro Leu Gly Ile Asp Thr Val Val Asp Leu
   305                 310                 315                 320
   Pro Val Gly Leu Asn Leu Gln Asp Gln Thr Thr Asn Thr Val Arg Ser
                   325                 330                 335
   Arg Ile Thr Ser Ala Gly Ala Gly Gln Gly Gln Ala Ala Trp Phe Ala
               340                 345                 350
   Thr Phe Asn Glu Thr Phe Gly Asp Tyr Ser Glu Lys Ala His Glu Leu
           355                 360                 365
   Leu Asn Thr Lys Leu Glu Gln Trp Ala Glu Glu Ala Val Ala Arg Gly
       370                 375                 380
   Gly Phe His Asn Thr Thr Ala Leu Leu Ile Gln Tyr Glu Asn Tyr Arg
   385                 390                 395                 400
   Asp Trp Ile Val Asn His Asn Val Ala Tyr Ser Glu Leu Val Leu Asp
                   405                 410                 415
   Thr Ala Gly Val Ala Ser Phe Asp Val Trp Asp Leu Leu Pro Phe Thr
               420                 425                 430
   Arg Gly Tyr Val His Ile Leu Asp Lys Asp Pro Tyr Leu His His Phe
           435                 440                 445
   Ala Tyr Asp Pro Gln Tyr Phe Leu Asn Glu Leu Asp Leu Leu Gly Gln
       450                 455                 460
   Ala Ala Ala Thr Gln Leu Ala Arg Asn Ile Ser Asn Ser Gly Ala Met
   465                 470                 475                 480
   Gln Thr Tyr Phe Ala Gly Glu Thr Ile Pro Gly Asp Asn Leu Ala Tyr
                   485                 490                 495
   Asp Ala Asp Leu Ser Ala Trp Thr Glu Tyr Ile Pro Tyr His Phe Arg
               500                 505                 510
   Pro Asn Tyr His Gly Val Gly Thr Cys Ser Met Met Pro Lys Glu Met
           515                 520                 525
   Gly Gly Val Val Asp Asn Ala Ala Arg Val Tyr Gly Val Gln Gly Leu
       530                 535                 540
   Arg Val Ile Asp Gly Ser Ile Pro Pro Thr Gln Met Ser Ser His Leu
   545                 550                 555                 560
   Met Thr Val Phe Tyr Ala Met Ala Leu Lys Ile Ser Asp Ala Ile Leu
                   565                 570                 575
   Glu Asp Tyr Ala Ser Met Gln
               580
<210> 7
   <211> 583
   <212> PRT
   <213> Aspergillus niger
<400> 7
   Ser Asn Gly Ile Glu Ala Ser Leu Leu Thr Asp Pro Lys Asp Val Ser
   1               5                   10                  15
   Gly Arg Thr Val Asp Tyr Ile Ile Ala Gly Gly Gly Leu Val Gly Leu
               20                  25                  30
   Thr Thr Ala Ala Arg Leu Thr Glu Asn Pro Asn Ile Ser Val Leu Val
           35                  40                  45
   Ile Glu Ser Gly Ser Tyr Glu Ser Asp Arg Gly Pro Ile Ile Glu Asp
       50                  55                  60
   Leu Asn Ala Tyr Gly Asp Ile Phe Gly Ser Ser Val Asp His Ala Tyr
   65                  70                  75                  80
   Glu Thr Val Glu Leu Ala Thr Asn Asn Gln Thr Ala Leu Val Arg Ser
                   85                  90                  95
   Gly Asn Gly Leu Gly Gly Ser Thr Leu Val Asn Gly Gly Thr Trp Thr
               100                 105                 110
   Arg Pro His Lys Ala Gln Val Asp Ser Trp Glu Thr Val Phe Gly Asn
           115                 120                 125
   Glu Gly Trp Asn Trp Asp Asn Val Ala Ala Tyr Ser Leu Gln Ala Glu
       130                 135                 140
   Arg Ala Arg Ala Pro Asn Ala Lys Gln Ile Ala Ala Gly His Tyr Phe
   145                 150                 155                 160
   Asn Ala Ser Cys His Gly Val Asn Gly Thr Val His Ala Gly Pro Arg
                   165                 170                 175
   Asp Thr Gly Asp Asp Tyr Ser Pro Ile Val Lys Ala Leu Met Ser Ala
               180                 185                 190
   Val Glu Asp Arg Gly Val Pro Thr Lys Lys Asp Phe Gly Cys Gly Asp
           195                 200                 205
   Pro His Gly Val Ser Met Phe Pro Asn Thr Leu His Glu Asp Gln Val
       210                 215                 220
   Arg Ser Asp Ala Ala Arg Glu Trp Leu Leu Pro Asn Tyr Gln Arg Pro
   225                 230                 235                 240
   Asn Leu Gln Val Leu Thr Gly Gln Tyr Val Gly Lys Val Leu Leu Ser
                   245                 250                 255
   Gln Asn Gly Thr Thr Pro Arg Ala Val Gly Val Glu Phe Gly Thr His
               260                 265                 270
   Lys Gly Asn Thr His Asn Val Tyr Ala Lys His Glu Val Leu Leu Ala
           275                 280                 285
   Ala Gly Ser Ala Val Ser Pro Thr Ile Leu Glu Tyr Ser Gly Ile Gly
       290                 295                 300
   Met Lys Ser Ile Leu Glu Pro Leu Gly Ile Asp Thr Val Val Asp Leu
   305                 310                 315                 320
   Pro Val Gly Leu Asn Leu Gln Asp Gln Thr Thr Ser Thr Val Arg Ser
                   325                 330                 335
   Arg Ile Thr Ser Ala Gly Ala Gly Gln Gly Gln Ala Ala Trp Phe Ala
               340                 345                 350
   Thr Phe Asn Glu Thr Phe Gly Asp Tyr Ser Glu Lys Ala His Glu Leu
           355                 360                 365
   Leu Asn Thr Lys Leu Glu Gln Trp Ala Glu Glu Ala Val Ala Arg Gly
       370                 375                 380
   Gly Phe His Asn Thr Thr Ala Leu Leu Ile Gln Tyr Glu Asn Tyr Arg
   385                 390                 395                 400
   Asp Trp Ile Val Asn His Asn Val Ala Tyr Ser Glu Leu Phe Leu Asp
                   405                 410                 415
   Thr Ala Gly Val Ala Ser Phe Asp Val Trp Asp Leu Leu Pro Phe Thr
               420                 425                 430
   Arg Gly Tyr Val His Ile Leu Asp Lys Asp Pro Tyr Leu His His Phe
           435                 440                 445
   Ala Tyr Asp Pro Gln Tyr Phe Leu Asn Glu Leu Asp Leu Leu Gly Gln
       450                 455                 460
   Ala Ala Ala Thr Gln Leu Ala Arg Asn Ile Ser Asn Ser Gly Ala Met
   465                 470                 475                 480
   Gln Thr Tyr Phe Ala Gly Glu Thr Ile Pro Gly Asp Asn Leu Ala Tyr
   485 490 495
   Asp Ala Asp Leu Ser Ala Trp Thr Glu Tyr Ile Pro Tyr His Phe Arg
   500 505 510
   Pro Asn Tyr His Gly Val Gly Thr Cys Ser Met Met Pro Lys Glu Met
   515 520 525
   Gly Gly Val Val Asp Asn Ala Ala Arg Val Tyr Gly Val Gln Gly Leu
   530 535 540
   Arg Val Ile Asp Gly Ser Ile Pro Pro Thr Gln Met Ser Ser His Pro
   545 550 555 560
   Met Thr Val Phe Tyr Ala Met Ala Leu Lys Ile Ser Asp Ala Ile Leu
   565 570 575
   Glu Asp Tyr Ala Ser Met Gln
   580
<210> 8
   <211> 583
   <212> PRT
   <213> Aspergillus niger
<400> 8
   Ser Asn Gly Ile Glu Ala Ser Leu Leu Thr Asp Pro Lys Asp Val Ser
   1 5 10 15
   Gly Arg Thr Val Asp Tyr Ile Ile Ala Gly Gly Gly Leu Val Gly Leu
   20 25 30
   Thr Thr Ala Ala Arg Leu Thr Glu Asn Pro Asn Ile Ser Val Leu Val
   35 40 45
   Ile Glu Ser Gly Phe Tyr Glu Ser Asp Arg Gly Pro Ile Ile Glu Asp
   50 55 60
   Leu Asn Ala Tyr Gly Asp Ile Phe Gly Ser Ser Val Asp His Ala Tyr
   65 70 75 80
   Glu Thr Val Glu Leu Ala Thr Asn Asn Gln Thr Ala Leu Val Arg Ser
   85 90 95
   Gly Asn Gly Leu Gly Gly Ser Thr Leu Val Asn Gly Gly Thr Trp Thr
   100 105 110
   Arg Pro His Lys Ala Gln Val Asp Ser Trp Glu Thr Val Phe Gly Asn
   115 120 125
   Glu Gly Trp Asn Trp Asp Asn Val Ala Ala Tyr Ser Leu Gln Ala Glu
   130 135 140
   Arg Ala Arg Ala Pro Asn Ala Lys Gln Ile Ala Ala Gly His Tyr Phe
   145 150 155 160
   Asn Ala Ser Cys His Gly Val Asn Gly Thr Val His Ala Gly Pro Arg
   165 170 175
   Asp Thr Gly Asp Asp Tyr Ser Pro Ile Val Lys Ala Leu Met Ser Ala
   180 185 190
   Val Glu Asp Arg Gly Val Pro Thr Lys Lys Asp Phe Gly Cys Gly Asp
   195 200 205
   Pro His Gly Val Ser Met Phe Pro Asn Thr Leu His Glu Asp Gln Val
   210 215 220
   Arg Ser Asp Ala Ala Arg Glu Trp Leu Leu Pro Asn Tyr Gln Arg Pro
   225 230 235 240
   Asn Leu Gln Val Leu Thr Gly Gln Tyr Val Gly Lys Val Leu Leu Ser
   245 250 255
   Gln Asn Gly Thr Thr Pro Arg Ala Val Gly Val Glu Phe Gly Thr His
   260 265 270
   Lys Gly Asn Thr His Asn Val Tyr Ala Lys His Glu Val Leu Leu Ala
   275 280 285
   Ala Gly Ser Ala Val Ser Pro Thr Ile Leu Glu Tyr Ser Gly Ile Gly
   290 295 300
   Met Lys Ser Ile Leu Glu Pro Leu Gly Ile Asp Thr Val Val Asp Leu
   305 310 315 320
   Pro Val Gly Leu Asn Leu Gln Asp Gln Thr Thr Ser Thr Val Arg Ser
   325 330 335
   Arg Ile Thr Ser Ala Gly Ala Gly Gln Gly Gln Ala Ala Trp Phe Ala
   340 345 350
   Thr Phe Asn Glu Thr Phe Gly Asp Tyr Ser Glu Lys Ala His Glu Leu
   355 360 365
   Leu Asn Thr Lys Leu Glu Gln Trp Ala Glu Glu Ala Val Ala Arg Gly
   370 375 380
   Gly Phe His Asn Thr Thr Ala Leu Leu Ile Gln Tyr Glu Asn Tyr Arg
   385 390 395 400
   Asp Trp Ile Val Asn His Asn Val Ala Tyr Ser Glu Leu Phe Leu Asp
   405 410 415
   Thr Ala Gly Val Ala Ser Phe Asp Val Trp Asp Leu Leu Pro Phe Thr
   420 425 430
   Arg Gly Tyr Val His Ile Leu Asp Lys Asp Pro Tyr Leu His His Phe
   435 440 445
   Ala Tyr Asp Pro Gln Tyr Phe Leu Asn Glu Leu Asp Leu Leu Gly Gln
   450 455 460
   Ala Ala Ala Thr Gln Leu Ala Arg Asn Ile Ser Asn Ser Gly Ala Met
   465 470 475 480
   Gln Thr Tyr Phe Ala Gly Glu Thr Ile Pro Gly Asp Asn Leu Ala Tyr
   485 490 495
   Asp Ala Asp Leu Ser Ala Trp Thr Glu Tyr Ile Pro Tyr His Phe Arg
   500 505 510
   Pro Asn Tyr His Gly Val Gly Thr Cys Ser Met Met Pro Lys Glu Met
   515 520 525
   Gly Gly Val Val Asp Asn Ala Ala Arg Val Tyr Gly Val Gln Gly Leu
   530 535 540
   Arg Val Ile Asp Gly Ser Ile Pro Pro Thr Gln Met Ser Ser His Val
   545 550 555 560
   Met Thr Val Phe Tyr Ala Met Ala Leu Lys Ile Ser Asp Ala Ile Leu
   565 570 575
   Glu Asp Tyr Ala Ser Met Gln
   580
<210> 9
   <211> 583
   <212> PRT
   <213> Aspergillus niger
<400> 9
   Ser Asn Gly Ile Glu Ala Ser Leu Leu Thr Asp Pro Lys Asp Val Ser
   1               5                   10                  15
   Gly Arg Thr Val Asp Tyr Ile Ile Ala Gly Gly Gly Leu Val Gly Leu
               20                  25                  30
   Thr Thr Ala Ala Arg Leu Thr Glu Asn Pro Asn Ile Ser Val Leu Val
           35                  40                  45
   Ile Glu Ser Gly Ser Tyr Glu Ser Asp Arg Gly Pro Ile Ile Glu Asp
       50                  55                  60
   Leu Asn Ala Tyr Gly Asp Ile Phe Gly Ser Ser Val Asp His Ala Tyr
   65                  70                  75                  80
   Glu Thr Val Glu Leu Ala Thr Asn Asn Gln Thr Ala Leu Val Arg Ser
                   85                  90                  95
   Gly Asn Gly Leu Gly Gly Ser Thr Leu Val Asn Gly Gly Thr Trp Thr
               100                 105                 110
   Arg Pro His Lys Ala Gln Val Asp Ser Trp Glu Thr Val Phe Gly Asn
           115                 120                 125
   Glu Gly Trp Asn Trp Asp Asn Val Leu Ala Tyr Ser Leu Gln Ala Glu
       130                 135                 140
   Arg Ala Arg Ala Pro Asn Ala Lys Gln Ile Ala Ala Gly His Tyr Phe
   145                 150                 155                 160
   Asn Ala Ser Cys His Gly Val Asn Gly Thr Val His Ala Gly Pro Arg
                   165                 170                 175
   Asp Thr Gly Asp Asp Tyr Ser Pro Ile Val Lys Ala Leu Met Ser Ala
               180                 185                 190
   Val Glu Asp Arg Gly Val Pro Thr Lys Lys Asp Phe Gly Cys Gly Asp
           195                 200                 205
   Pro His Gly Val Ser Met Phe Pro Asn Thr Leu His Glu Asp Gln Val
       210                 215                 220
   Arg Ser Asp Ala Ala Arg Glu Trp Leu Leu Pro Asn Tyr Gln Arg Pro
   225                 230                 235                 240
   Asn Leu Gln Val Leu Thr Gly Gln Tyr Val Gly Lys Val Leu Leu Ser
                   245                 250                 255
   Gln Asn Gly Thr Thr Pro Arg Ala Val Gly Val Glu Phe Gly Thr His
               260                 265                 270
   Lys Gly Asn Thr His Asn Val Tyr Ala Lys His Glu Val Leu Leu Ala
           275                 280                 285
   Ala Gly Ser Ala Val Ser Pro Thr Ile Leu Glu Tyr Ser Gly Ile Gly
       290                 295                 300
   Met Lys Ser Ile Leu Glu Pro Leu Gly Ile Asp Thr Val Val Asp Leu
   305                 310                 315                 320
   Pro Val Gly Leu Asn Leu Gln Asp Gln Thr Thr Ala Thr Val Arg Ser
                   325                 330                 335
   Arg Ile Thr Ser Ala Gly Ala Gly Gln Gly Gln Ala Ala Trp Phe Ala
               340                 345                 350
   Thr Phe Asn Glu Thr Phe Gly Asp Tyr Ser Glu Lys Ala His Glu Leu
           355                 360                 365
   Leu Asn Thr Lys Leu Glu Gln Trp Ala Glu Glu Ala Val Ala Arg Gly
       370                 375                 380
   Gly Phe His Asn Thr Thr Ala Leu Leu Ile Gln Tyr Glu Asn Tyr Arg
   385                 390                 395                 400
   Asp Trp Ile Val Asn His Asn Val Ala Tyr Ser Glu Leu Phe Leu Asp
                   405                 410                 415
   Thr Ala Gly Val Ala Ser Phe Asp Val Trp Asp Leu Leu Pro Phe Thr
               420                 425                 430
   Arg Gly Tyr Val His Ile Leu Asp Lys Asp Pro Tyr Leu His His Phe
           435                 440                 445
   Ala Tyr Asp Pro Gln Tyr Phe Leu Asn Glu Leu Asp Leu Leu Gly Gln
       450                 455                 460
   Ala Ala Ala Thr Gln Leu Ala Arg Asn Ile Ser Asn Ser Gly Ala Met
   465                 470                 475                 480
   Gln Thr Tyr Phe Ala Gly Glu Thr Ile Pro Gly Asp Asn Leu Ala Tyr
                   485                 490                 495
   Asp Ala Asp Leu Ser Ala Trp Thr Glu Tyr Ile Pro Tyr His Phe Arg
               500                 505                 510
   Pro Asn Tyr His Gly Val Gly Thr Cys Ser Met Met Pro Lys Glu Met
           515                 520                 525
   Gly Gly Val Val Asp Asn Ala Ala Arg Val Tyr Gly Val Gln Gly Leu
       530                 535                 540
   Arg Val Ile Asp Gly Ser Ile Pro Pro Thr Gln Met Ser Ser His Val
   545                 550                 555                 560
   Met Thr Val Phe Tyr Ala Met Ala Leu Lys Ile Ser Asp Ala Ile Leu
                   565                 570                 575
   Glu Asp Tyr Ala Ser Met Gln
               580
<210> 10
   <211> 1749
   <212> DNA
   <213> Aspergillus niger
<400> 10
   agcaatggca ttgaagccag cctcctgact gatcccaagg atgtctccgg ccgcacggtc      60
   gactacatca tcgctggtgg aggtctggtt ggactcacca ccgctgctcg tctgacggag     120
   aaccccaaca tcagtgtgct cgtcatcgaa agtggctcct acgagtcgga cagaggtcct     180
   atcattgagg acctgaacgc ctacggcgac atctttggca gcagtgtaga ccacgcctac     240
   gagaccgtgg agctcgctac caacaatcaa accgcgctgg tccgctccgg aaatggtctc     300
   ggtggctcta ctctagtgaa tggtggcacc tggactcgcc cccacaaggc acaggttgac     360
   tcttgggaga ctgtctttgg aaatgagggc tggaactggg acaatgtggc cgcctactcc     420
   ctccaggctg agcgtgctcg cgcaccaaat gccaaacaga tcgctgctgg ccactacttc     480
   aacgcatcct gccatggtgt taatggtact gtccatgccg gaccccgcga caccggcgat     540
   gactattctc ccatcgtcaa ggctctcatg agcgctgtcg aagaccgggg cgttcccacc     600
   aagaaagact tcggatgcgg tgacccccat ggtgtgtcca tgttccccaa caccttgcac     660
   gaagaccaag tgcgctccga tgccgctcgc gaatggctac ttcccaacta ccaacgtccc     720
   aacctgcaag tcctgaccgg acagtatgtt ggtaaggtgc tccttagcca gaacggcacc     780
   acccctcgtg ccgttggcgt ggaattcggc acccacaagg gcaacaccca caacgtttac     840
   gctaagcacg aggtcctcct ggccgcgggc tccgctgtct ctcccacaat cctcgaatat     900
   tccggtatcg gaatgaagtc catcctggag ccccttggta tcgacaccgt cgttgacctg     960
   cccgtcggct tgaacctgca ggaccagacc accgctaccg tccgctcccg catcacctct    1020
   gctggtgcag gacagggaca ggccgcttgg ttcgccacct tcaacgagac ctttggtgac    1080
   tattccgaaa aggcacacga gctgctcaac accaagctgg agcagtgggc cgaagaggcc    1140
   gtcgcccgtg gcggattcca caacaccacc gccttgctca tccagtacga gaactaccgc    1200
   gactggattg tcaaccacaa cgtcgcgtac tcggaactct tcctcgacac tgccggagta    1260
   gccagcttcg atgtgtggga ccttctgccc ttcacccgag gatacgttca catcctcgac    1320
   aaggacccct accttcacca cttcgcctac gaccctcagt acttcctcaa cgagctggac    1380
   ctgctcggtc aggctgccgc tactcaactg gcccgcaaca tctccaactc cggtgccatg    1440
   cagacctact tcgctgggga gactatcccc ggtgataacc tcgcgtatga tgccgatttg    1500
   agcgcctgga ctgagtacat cccgtaccac ttccgtccta actaccatgg cgtgggtact    1560
   tgctccatga tgccgaagga gatgggcggt gttgttgata atgctgcccg tgtgtatggt    1620
   gtgcagggac tgcgtgtcat tgatggttct attcctccta cgcaaatgtc gtcccatgtc    1680
   atgacggtgt tctatgccat ggcgctaaaa atttcggatg ctatcttgga agattatgct    1740
   tccatgcag                                                            1749
<210> 11
   <211> 1749
   <212> DNA
   <213> Aspergillus niger
<400> 11
   agcaatggca ttgaagccag cctcctgact gatcccaagg atgtctccgg ccgcacggtc    60
   gactacatca tcgctggtgg aggtctggtt ggactcacca ccgctgctcg tctgacggag   120
   aaccccaaca tcagtgtgct cgtcatcgaa agtggctcct acgagtcgga cagaggtcct   180
   atcattgagg acctgaacgc ctacggcgac atctttggca gcagtgtaga ccacgcctac   240
   gagaccgtgg agctcgctac caacaatcaa accgcgctgg tccgctccgg aaatggtctc   300
   ggtggctcta ctctagtgaa tggtggcacc tggactcgcc cccacaaggc acaggttgac   360
   tcttgggaga ctgtctttgg aaatgagggc tggaactggg acaatgtggc cgcctactcc   420
   ctccaggctg agcgtgctcg cgcaccaaat gccaaacaga tcgctgctgg ccactacttc   480
   aacgcatcct gccatggtgt taatggtact gtccatgttg gaccccgcga caccggcgat   540
   gactattctc ccatcgtcaa ggctctcatg agcgctgtcg aagaccgggg cgttcccacc   600
   aagaaagact tcggatgcgg tgacccccat ggtgtgtcca tgttccccaa caccttgcac   660
   gaagaccaag tgcgctccga tgccgctcgc gaatggctac ttcccaacta ccaacgtccc   720
   aacctgcaag tcctgaccgg acagtatgtt ggtaaggtgc tccttagcca gaacggcacc   780
   acccctcgtg ccgttggcgt ggaattcggc acccacaagg gcaacaccca caacgtttac   840
   gctaagcacg aggtcctcct ggccgcgggc tccgctgtct ctcccacaat cctcgaatat    900
   tccggtatcg gaatgaagtc catcctggag ccccttggta tcgacaccgt cgttgacctg    960
   cccgtcggct tgaacctgca ggaccagacc accagtaccg tccgctcccg catcacctct   1020
   gctggtgcag gacagggaca ggccgcttgg ttcgccacct tcaacgagac ctttggtgac   1080
   tattccgaaa aggcacacga gctgctcaac accaagctgg agcagtgggc cgaagaggcc   1140
   gtcgcccgtg gcggattcca caacaccacc gccttgctca tccagtacga gaactaccgc   1200
   gactggattg tcaaccacaa cgtcgcgtac tcggaactca ttctcgacac tgccggagta   1260
   gccagcttcg atgtgtggga ccttctgccc ttcacccgag gatacgttca catcctcgac   1320
   aaggacccct accttcacca cttcgcctac gaccctcagt acttcctcaa cgagctggac   1380
   ctgctcggtc aggctgccgc tactcaactg gcccgcaaca tctccaactc cggtgccatg   1440
   cagacctact tcgctgggga gactatcccc ggtgataacc tcgcgtatga tgccgatttg   1500
   agcgcctgga ctgagtacat cccgtaccac ttccgtccta actaccatgg cgtgggtact   1560
   tgctccatga tgccgaagga gatgggcggt gttgttgata atgctgcccg tgtgtatggt   1620
   gtgcagggac tgcgtgtcat tgatggttct attcctccta cgcaaatgtc gtcccatacg   1680
   atgacggtgt tctatgccat ggcgctaaaa atttcggatg ctatcttgga agattatgct   1740
   tccatgcag                                                           1749
<210> 12
   <211> 1749
   <212> DNA
   <213> Aspergillus niger
<400> 12
   agcaatggca ttgaagccag cctcctgact gatcccaagg atgtctccgg ccgcacggtc      60
   gactacatca tcgctggtgg aggtctggtt ggactcacca ccgctgctcg tctgacggag     120
   aaccccaaca tcagtgtgct cgtcatcgaa agtggctcct acgagtcgga cagaggtcct     180
   atcattgagg acctgaacgc ctacggcgac atctttggca gcagtgtaga ccacgcctac     240
   gagaccgtgg agctcgctac caacaatcaa accgcgctgg tccgctccgg aaatggtctc     300
   ggtggctcta ctctagtgaa tggtggcacc tggactcgcc cccacaaggc acaggttgac     360
   tcttgggaga ctgtctttgg aaatgagggc tggaactggg acaatgtggc cgcctactcc     420
   ctccaggctg agcgtgctcg cgcaccaaat gccaaacaga tcgctgctgg ccactacttc     480
   aacgcatcct gccatggtgt taatggtact gtccatattg gaccccgcga caccggcgat     540
   gactattctc ccatcgtcaa ggctctcatg agcgctgtcg aagaccgggg cgttcccacc     600
   aagaaagact tcggatgcgg tgacccccat ggtgtgtcca tgttccccaa caccttgcac     660
   gaagaccaag tgcgctccga tgccgctcgc gaatggctac ttcccaacta ccaacgtccc     720
   aacctgcaag tcctgaccgg acagtatgtt ggtaaggtgc tccttagcca gaacggcacc     780
   acccctcgtg ccgttggcgt ggaattcggc acccacaagg gcaacaccca caacgtttac     840
   gctaagcacg aggtcctcct ggccgcgggc tccgctgtct ctcccacaat cctcgaatat     900
   tccggtatcg gaatgaagtc catcctggag ccccttggta tcgacaccgt cgttgacctg     960
   cccgtcggct tgaacctgca ggaccagacc accagtaccg tccgctcccg catcacctct    1020
   gctggtgcag gacagggaca ggccgcttgg ttcgccacct tcaacgagac ctttggtgac    1080
   tattccgaaa aggcacacga gctgctcaac accaagctgg agcagtgggc cgaagaggcc    1140
   gtcgcccgtg gcggattcca caacaccacc gccttgctca tccagtacga gaactaccgc    1200
   gactggattg tcaaccacaa cgtcgcgtac tcggaactcc ttctcgacac tgccggagta    1260
   gccagcttcg atgtgtggga ccttctgccc ttcacccgag gatacgttca catcctcgac    1320
   aaggacccct accttcacca cttcgcctac gaccctcagt acttcctcaa cgagctggac    1380
   ctgctcggtc aggctgccgc tactcaactg gcccgcaaca tctccaactc cggtgccatg    1440
   cagacctact tcgctgggga gactatcccc ggtgataacc tcgcgtatga tgccgatttg    1500
   agcgcctgga ctgagtacat cccgtaccac ttccgtccta actaccatgg cgtgggtact    1560
   tgctccatga tgccgaagga gatgggcggt gttgttgata atgctgcccg tgtgtatggt    1620
   gtgcagggac tgcgtgtcat tgatggttct attcctccta cgcaaatgtc gtcccatgtc    1680
   atgacggtgt tctatgccat ggcgctaaaa atttcggatg ctatcttgga agattatgct    1740
   tccatgcag                                                            1749
<210> 13
   <211> 1749
   <212> DNA
   <213> Aspergillus niger
<400> 13
   agcaatggca ttgaagccag cctcctgact gatcccaagg atgtctccgg ccgcacggtc      60
   gactacatca tcgctggtgg aggtctggtt ggactcacca ccgctgctcg tctgacggag     120
   aaccccaaca tcagtgtgct cgtcatcgaa agtggctcct acgagtcgga cagaggtcct     180
   atcattgagg acctgaacgc ctacggcgac atctttggca gcagtgtaga ccacgcctac     240
   gagaccgtgg agctcgctac caacaatcaa accgcgctgg tccgctccgg aaatggtctc     300
   ggtggctcta ctctagtgaa tggtggcacc tggactcgcc cccacaaggc acaggttgac     360
   tcttgggaga ctgtctttgg aaatgagggc tggaactggg acaatgtggc cgcctactcc     420
   ctccaggctg agcgtgctcg cgcaccaaat gccaaacaga tcgctgctgg ccactacttc     480
   aacgcatcct gccatggtgt taatggtact gtccatgttg gaccccgcga caccggcgat     540
   gactattctc ccatcgtcaa ggctctcatg agcgctgtcg aagaccgggg cgttcccacc     600
   aagaaagact tcggatgcgg tgacccccat ggtgtgtcca tgttccccaa caccttgcac     660
   gaagaccaag tgcgctccga tgccgctcgc gaatggctac ttcccaacta ccaacgtccc     720
   aacctgcaag tcctgaccgg acagtatgtt ggtaaggtgc tccttagcca gaacggcacc     780
   acccctcgtg ccgttggcgt ggaattcggc acccacaagg gcaacaccca caacgtttac     840
   gctaagcacg aggtcctcct ggccgcgggc tccgctgtct ctcccacaat cctcgaatat     900
   tccggtatcg gaatgaagtc catcctggag ccccttggta tcgacaccgt cgttgacctg     960
   cccgtcggct tgaacctgca ggaccagacc accaataccg tccgctcccg catcacctct    1020
   gctggtgcag gacagggaca ggccgcttgg ttcgccacct tcaacgagac ctttggtgac    1080
   tattccgaaa aggcacacga gctgctcaac accaagctgg agcagtgggc cgaagaggcc    1140
   gtcgcccgtg gcggattcca caacaccacc gccttgctca tccagtacga gaactaccgc    1200
   gactggattg tcaaccacaa cgtcgcgtac tcggaactct ttctcgacac tgccggagta    1260
   gccagcttcg atgtgtggga ccttctgccc ttcacccgag gatacgttca catcctcgac    1320
   aaggacccct accttcacca cttcgcctac gaccctcagt acttcctcaa cgagctggac    1380
   ctgctcggtc aggctgccgc tactcaactg gcccgcaaca tctccaactc cggtgccatg    1440
   cagacctact tcgctgggga gactatcccc ggtgataacc tcgcgtatga tgccgatttg    1500
   agcgcctgga ctgagtacat cccgtaccac ttccgtccta actaccatgg cgtgggtact    1560
   tgctccatga tgccgaagga gatgggcggt gttgttgata atgctgcccg tgtgtatggt    1620
   gtgcagggac tgcgtgtcat tgatggttct attcctccta cgcaaatgtc gtcccatccg    1680
   atgacggtgt tctatgccat ggcgctaaaa atttcggatg ctatcttgga agattatgct    1740
   tccatgcag                                                            1749
<210> 14
   <211> 1704
   <212> DNA
   <213> Aspergillus niger
<400> 14
   agcaatggca ttgaagccag cctcctgact gatcccaagg atgtctccgg ccgcacggtc      60
   gactacatca tcgctggtgg aggtctggtt ggactcacca ccgctgctcg tctgacggag     120
   aaccccaaca tcagtgtgct cgtcatcgaa agtggctcct acgagtcgga cagaggtcct     180
   atcattgagg acctgaacgc ctacggcgac atctttggca gcagtgtaga ccacgcctac     240
   gagaccgtgg agctcgctac caacaatcaa accgcgctgg tccgctccgg aaatggtctc     300
   ggtggctcta ctctagtgaa tggtggcacc tggactcgcc cccacaaggc acaggttgac     360
   tcttgggaga ctgtctttgg aaatgagggc tggaactggg acaatgtggc cgcctactcc     420
   ctccaggctg agcgtgctcg cgcaccaaat gccaaacaga tcgctgctgg ccactacttc     480
   aacgcatcct gccatggtgt taatggtact gtccatgttg gaccccgcga caccggcgat     540
   gactattctc ccatcgtcaa ggctctcatg agcgctgtcg aagaccgggg cgttcccacc     600
   aagaaagact tcggatgcgg tgacccccat ggtgtgtcca tgttccccaa caccttgcac     660
   gaagaccaag tgcgctccga tgccgctcgc gaatggctac ttcccaacta ccaacgtccc     720
   aacctgcaag tcctgaccgg acagtatgtt ggtaaggtgc tccttagcca gaacggcacc     780
   acccctcgtg ccgttggcgt ggaattcggc acccacaagg gcaacaccca caacgtttac     840
   gctaagcacg aggtcctcct ggccgcgggc tccgctgtct ctcccacaat cctcgaatat     900
   tccggtatcg gaatgaagtc catcctggag ccccttggta tcgacaccgt cgttgacctg     960
   cccgtcggct tgaacctgca ggaccagacc accaataccg tccgctcccg catcacctct    1020
   gctggtgcag gacagggaca ggccgcttgg ttcgccacct tcaacgagac ctttggtgac    1080
   tattccgaaa aggcacacga gctgctcaac accaagctgg agcagtgggc cgaagaggcc    1140
   gtcgcccgtg gcggattcca caacaccacc gccttgctca tccagtacga gaactaccgc    1200
   gactggattg tcaaccacaa cgtcgcgtac tcggaactcg ttctcgacac tgccggagta    1260
   gccagcttcg atgtgtggga ccttctgccc ttcacccgag gatacgttca catcctcgac    1320
   aaggacccct accttcacca cttcgcctac gaccctcagt acttcctcaa cgagctggac    1380
   ctgctcggtc aggctgccgc tactcaactg gcccgcaaca tctccaactc cggtgccatg    1440
   cagacctact tcgctgggga gactatcccc ggtgataacc tcgcgtatga tgccgatttg    1500
   agcgcctgga ctgagtacat cccgtaccac ttccgtccta actaccatgg cgtgggtact    1560
   tgctccatga tgccgaagga gatgggcggt gttgttgata atgctgcccg tgtgtatggt    1620
   gtgcagggac tgcgtgtcat tgatggttct attcctccta cgcaaatgtc gtcccatctg    1680
   atgacggtgt tctatgccat ggcg                                           1704
<210> 15
   <211> 1749
   <212> DNA
   <213> Aspergillus niger
<400> 15
   agcaatggca ttgaagccag cctcctgact gatcccaagg atgtctccgg ccgcacggtc     60
   gactacatca tcgctggtgg aggtctggtt ggactcacca ccgctgctcg tctgacggag    120
   aaccccaaca tcagtgtgct cgtcatcgaa agtggctcct acgagtcgga cagaggtcct    180
   atcattgagg acctgaacgc ctacggcgac atctttggca gcagtgtaga ccacgcctac    240
   gagaccgtgg agctcgctac caacaatcaa accgcgctgg tccgctccgg aaatggtctc    300
   ggtggctcta ctctagtgaa tggtggcacc tggactcgcc cccacaaggc acaggttgac    360
   tcttgggaga ctgtctttgg aaatgagggc tggaactggg acaatgtggc cgcctactcc    420
   ctccaggctg agcgtgctcg cgcaccaaat gccaaacaga tcgctgctgg ccactacttc 480
   aacgcatcct gccatggtgt taatggtact gtccatgccg gaccccgcga caccggcgat 540
   gactattctc ccatcgtcaa ggctctcatg agcgctgtcg aagaccgggg cgttcccacc 600
   aagaaagact tcggatgcgg tgacccccat ggtgtgtcca tgttccccaa caccttgcac 660
   gaagaccaag tgcgctccga tgccgctcgc gaatggctac ttcccaacta ccaacgtccc 720
   aacctgcaag tcctgaccgg acagtatgtt ggtaaggtgc tccttagcca gaacggcacc 780
   acccctcgtg ccgttggcgt ggaattcggc acccacaagg gcaacaccca caacgtttac 840
   gctaagcacg aggtcctcct ggccgcgggc tccgctgtct ctcccacaat cctcgaatat 900
   tccggtatcg gaatgaagtc catcctggag ccccttggta tcgacaccgt cgttgacctg 960
   cccgtcggct tgaacctgca ggaccagacc accagtaccg tccgctcccg catcacctct 1020
   gctggtgcag gacagggaca ggccgcttgg ttcgccacct tcaacgagac ctttggtgac 1080
   tattccgaaa aggcacacga gctgctcaac accaagctgg agcagtgggc cgaagaggcc 1140
   gtcgcccgtg gcggattcca caacaccacc gccttgctca tccagtacga gaactaccgc 1200
   gactggattg tcaaccacaa cgtcgcgtac tcggaactct tcctcgacac tgccggagta 1260
   gccagcttcg atgtgtggga ccttctgccc ttcacccgag gatacgttca catcctcgac 1320
   aaggacccct accttcacca cttcgcctac gaccctcagt acttcctcaa cgagctggac 1380
   ctgctcggtc aggctgccgc tactcaactg gcccgcaaca tctccaactc cggtgccatg 1440
   cagacctact tcgctgggga gactatcccc ggtgataacc tcgcgtatga tgccgatttg 1500
   agcgcctgga ctgagtacat cccgtaccac ttccgtccta actaccatgg cgtgggtact 1560
   tgctccatga tgccgaagga gatgggcggt gttgttgata atgctgcccg tgtgtatggt 1620
   gtgcagggac tgcgtgtcat tgatggttct attcctccta cgcaaatgtc gtcccatccg 1680
   atgacggtgt tctatgccat ggcgctaaaa atttcggatg ctatcttgga agattatgct 1740
   tccatgcag 1749
<210> 16
   <211> 1749
   <212> DNA
   <213> Aspergillus niger
<400> 16
   agcaatggca ttgaagccag cctcctgact gatcccaagg atgtctccgg ccgcacggtc      60
   gactacatca tcgctggtgg aggtctggtt ggactcacca ccgctgctcg tctgacggag     120
   aaccccaaca tcagtgtgct cgtcatcgaa agtggcttct acgagtcgga cagaggtcct     180
   atcattgagg acctgaacgc ctacggcgac atctttggca gcagtgtaga ccacgcctac     240
   gagaccgtgg agctcgctac caacaatcaa accgcgctgg tccgctccgg aaatggtctc     300
   ggtggctcta ctctagtgaa tggtggcacc tggactcgcc cccacaaggc acaggttgac     360
   tcttgggaga ctgtctttgg aaatgagggc tggaactggg acaatgtggc cgcctactcc     420
   ctccaggctg agcgtgctcg cgcaccaaat gccaaacaga tcgctgctgg ccactacttc     480
   aacgcatcct gccatggtgt taatggtact gtccatgccg gaccccgcga caccggcgat     540
   gactattctc ccatcgtcaa ggctctcatg agcgctgtcg aagaccgggg cgttcccacc     600
   aagaaagact tcggatgcgg tgacccccat ggtgtgtcca tgttccccaa caccttgcac     660
   gaagaccaag tgcgctccga tgccgctcgc gaatggctac ttcccaacta ccaacgtccc     720
   aacctgcaag tcctgaccgg acagtatgtt ggtaaggtgc tccttagcca gaacggcacc     780
   acccctcgtg ccgttggcgt ggaattcggc acccacaagg gcaacaccca caacgtttac     840
   gctaagcacg aggtcctcct ggccgcgggc tccgctgtct ctcccacaat cctcgaatat     900
   tccggtatcg gaatgaagtc catcctggag ccccttggta tcgacaccgt cgttgacctg     960
   cccgtcggct tgaacctgca ggaccagacc accagtaccg tccgctcccg catcacctct    1020
   gctggtgcag gacagggaca ggccgcttgg ttcgccacct tcaacgagac ctttggtgac    1080
   tattccgaaa aggcacacga gctgctcaac accaagctgg agcagtgggc cgaagaggcc    1140
   gtcgcccgtg gcggattcca caacaccacc gccttgctca tccagtacga gaactaccgc    1200
   gactggattg tcaaccacaa cgtcgcgtac tcggaactct tcctcgacac tgccggagta    1260
   gccagcttcg atgtgtggga ccttctgccc ttcacccgag gatacgttca catcctcgac    1320
   aaggacccct accttcacca cttcgcctac gaccctcagt acttcctcaa cgagctggac    1380
   ctgctcggtc aggctgccgc tactcaactg gcccgcaaca tctccaactc cggtgccatg    1440
   cagacctact tcgctgggga gactatcccc ggtgataacc tcgcgtatga tgccgatttg    1500
   agcgcctgga ctgagtacat cccgtaccac ttccgtccta actaccatgg cgtgggtact    1560
   tgctccatga tgccgaagga gatgggcggt gttgttgata atgctgcccg tgtgtatggt    1620
   gtgcagggac tgcgtgtcat tgatggttct attcctccta cgcaaatgtc gtcccatgtc    1680
   atgacggtgt tctatgccat ggcgctaaaa atttcggatg ctatcttgga agattatgct    1740
   tccatgcag                                                            1749
<210> 17
   <211> 1749
   <212> DNA
   <213> Aspergillus niger
<400> 17
   agcaatggca ttgaagccag cctcctgact gatcccaagg atgtctccgg ccgcacggtc      60
   gactacatca tcgctggtgg aggtctggtt ggactcacca ccgctgctcg tctgacggag     120
   aaccccaaca tcagtgtgct cgtcatcgaa agtggctcct acgagtcgga cagaggtcct     180
   atcattgagg acctgaacgc ctacggcgac atctttggca gcagtgtaga ccacgcctac    240
   gagaccgtgg agctcgctac caacaatcaa accgcgctgg tccgctccgg aaatggtctc    300
   ggtggctcta ctctagtgaa tggtggcacc tggactcgcc cccacaaggc acaggttgac    360
   tcttgggaga ctgtctttgg aaatgagggc tggaactggg acaatgtgtt ggcctactcc    420
   ctccaggctg agcgtgctcg cgcaccaaat gccaaacaga tcgctgctgg ccactacttc    480
   aacgcatcct gccatggtgt taatggtact gtccatgccg gaccccgcga caccggcgat    540
   gactattctc ccatcgtcaa ggctctcatg agcgctgtcg aagaccgggg cgttcccacc    600
   aagaaagact tcggatgcgg tgacccccat ggtgtgtcca tgttccccaa caccttgcac    660
   gaagaccaag tgcgctccga tgccgctcgc gaatggctac ttcccaacta ccaacgtccc    720
   aacctgcaag tcctgaccgg acagtatgtt ggtaaggtgc tccttagcca gaacggcacc    780
   acccctcgtg ccgttggcgt ggaattcggc acccacaagg gcaacaccca caacgtttac    840
   gctaagcacg aggtcctcct ggccgcgggc tccgctgtct ctcccacaat cctcgaatat    900
   tccggtatcg gaatgaagtc catcctggag ccccttggta tcgacaccgt cgttgacctg    960
   cccgtcggct tgaacctgca ggaccagacc accgctaccg tccgctcccg catcacctct   1020
   gctggtgcag gacagggaca ggccgcttgg ttcgccacct tcaacgagac ctttggtgac   1080
   tattccgaaa aggcacacga gctgctcaac accaagctgg agcagtgggc cgaagaggcc   1140
   gtcgcccgtg gcggattcca caacaccacc gccttgctca tccagtacga gaactaccgc   1200
   gactggattg tcaaccacaa cgtcgcgtac tcggaactct tcctcgacac tgccggagta   1260
   gccagcttcg atgtgtggga ccttctgccc ttcacccgag gatacgttca catcctcgac   1320
   aaggacccct accttcacca cttcgcctac gaccctcagt acttcctcaa cgagctggac   1380
   ctgctcggtc aggctgccgc tactcaactg gcccgcaaca tctccaactc cggtgccatg   1440
   cagacctact tcgctgggga gactatcccc ggtgataacc tcgcgtatga tgccgatttg   1500
   agcgcctgga ctgagtacat cccgtaccac ttccgtccta actaccatgg cgtgggtact   1560
   tgctccatga tgccgaagga gatgggcggt gttgttgata atgctgcccg tgtgtatggt   1620
   gtgcagggac tgcgtgtcat tgatggttct attcctccta cgcaaatgtc gtcccatgtc   1680
   atgacggtgt tctatgccat ggcgctaaaa atttcggatg ctatcttgga agattatgct   1740
   tccatgcag                                                           1749